(19) Europäisches Patentamt
European Patent Office
Office européen des brevets

(11) Veröffentlichungsnummer: **0 132 566**
**B1**

(12) **EUROPÄISCHE PATENTSCHRIFT**

(45) Veröffentlichungstag der Patentschrift: **01.07.87**

(21) Anmeldenummer: **84106671.5**

(22) Anmeldetag: **12.06.84**

(51) Int. Cl.[4]: **C 07 C 62/38,** C 07 C 69/757, C 07 C 149/40, C 07 C 121/60, C 07 C 121/48, C 07 C 103/19, C 07 C 49/84, A 61 K 31/19

(54) **Substituierte Benzophenone.**

(30) Priorität: **15.06.83 CH 3270/83**

(43) Veröffentlichungstag der Anmeldung: **13.02.85 Patentblatt 85/7**

(45) Bekanntmachung des Hinweises auf die Patenterteilung: **01.07.87 Patentblatt 87/27**

(84) Benannte Vertragsstaaten: **AT BE CH DE FR GB IT LI LU NL SE**

(56) Entgegenhaltungen: **GB-A-1 195 294**

(73) Patentinhaber: **CIBA- GEIGY AG, Klybeckstrasse 141, CH- 4002 Basel (CH)**

(72) Erfinder: **Haas, Georges, Dr.,, Im Rehwechsel 24, CH- 4102 Binningen (CH)**
Erfinder: **von Sprecher, Andreas, Dr.,, Nelkenweg 5, CH- 4104 Oberwil (CH)**
Erfinder: **Ferrini, Pier Giorgio, Dr.,, Im Rehwechsel 22, CH- 4102 Binningen (CH)**

(74) Vertreter: **Zumstein, Fritz jun., Dr., Dr. F. Zumstein sen. Dr. E. Assmann Dr. R. Koenigsberger Dipl.- Ing. F. Klingseisen Dr. F. Zumstein jun. Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 132 566 B1

LIBER, STOCKHOLM 1987

**Beschreibung**

Die Erfindung betrifft neue substituierte Benzophenone der Formel

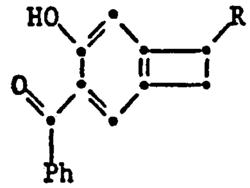

(I),

worin Ph unsubstituiertes oder substituiertes Phenyl bedeutet und R freies, verestertes oder amidiertes Carboxy bedeutet, und ihre Salze, Verfahren zu ihrer Herstellung, die Verwendung von Verbindungen der Formel I und ihre Salze als Wirkstoff in bzw. zur Herstellung von pharmazeutischen Präparaten sowie derartige pharmazeutische Präparate.

Substituiertes Phenyl ist beispielsweise durch Niederalkyl, Niederalkoxy, Niederalkylthio, Niederalkanoyloxy, Halogen, Trifluormethyl, Carboxy, Niederalkoxycarbonyl, Cyano und/oder Nitro substituiertes, insbesondere mono- oder disubstituiertes Phenyl.

Verestertes Carboxy ist beispielsweise mit einem niederen aliphatischen Alkohol verestertes Carboxy, wie Niederalkoxycarbonyl.

Amidiertes Carboxy weist als Aminogruppe beispielsweise unsubstituiertes oder durch niedere aliphatische Reste mono- oder disubstituiertes Amino auf und bedeutet beispielsweise Carbamyl, N-Mono oder N,N-Diniederalkylcarbamyl oder N,N-Niederalkylen- bzw. N,N-(Aza)-, N,N-(Oxa)- oder N,N-(Thia)niederalkylencarbamyl.

Vor- und nachstehend sind unter niederen organischen Resten und Verbindungen vorzugsweise solche zu verstehen, die bis und mit 7, insbesondere bis und mit 4 Kohlenstoffatome (C-Atome) aufweisen.

Niederalkyl ist beispielsweise Methyl, Aethyl, Propyl, Isopropyl, Butyl, Isobutyl, Sekundärbutyl oder Tertiärbutyl, kann aber auch ein Pentyl-, Hexyl- oder Heptylrest sein.

Niederalkoxy ist beispielsweise Methoxy, Aethoxy, Propyloxy, Isopropyloxy, Butyloxy, Isobutyloxy, Sekundärbutyloxy oder Tertiärbutyloxy, kann aber auch ein Pentyl-, Hexyl- oder Heptyloxyrest sein.

Niederalkylthio ist beispielsweise Methylthio, Aethylthio, Propylthio, Butylthio, Isobutylthio, Sekundarbutylthio oder Tertiärbutylthio, kann aber auch eine Pentylthio, Hexylthio- oder Heptylthiogruppe sein.

Niederalkanoyloxy ist z. B. Acetoxy, Propionyloxy, Butyryloxy oder Isobutyryloxy, ferner Valeroyloxy, Pivaloyloxy oder Caproyloxy, Halogen ist beispielsweise Halogen der Atomnummer bis und mit 35, wie Fluor oder Chlor ferner Brom.

Niederalkoxycarbonyl ist beispielsweise Methoxy-, Aethoxy-, Propyloxy-, Isopropyloxy-, Butyloxy-, Isobutyloxy-, Sekundärbutyloxy- oder Tertiärbutyloxycarbonyl, kann aber auch ein Pentyloxy-, Hexyloxy- oder Heptyloxycarbonylrest sein.

N-Mono- oder N,N-Diniederalkylcarbamyl ist beispielsweise N-Methyl-, N,N-Dimethyl-, N-Aethyl-, N,N-Diäthyl-, N-Propyl-, N-Isopropyl-, N-Butyl- oder N-Isobutylcarbamyl.

N,N-Niederalkylen- bzw. N,N-(Aza)-, N,N-(Oxa)- oder N,N-(Thia)niederalkylencarbamyl weist beispielsweise 3 bis und mit 8, insbesondere 5 oder 6, Ringglieder auf und bedeutet beispielsweise Pyrrolidino-, Piperidino-, Piperazino- bzw. N'-Niederalkyl-, wie N'-Methylpiperazino-, Morpholino- oder Thiomorpholinocarbonyl.

Salze von Verbindungen der Formel I sind insbesondere deren Salze mit pharmazeutisch verwendbaren Basen, wie nicht-toxische, von Metallen oder Gruppen Ia, Ib, IIa und IIb abgeleitete Metallsalze z. B. Alkalimetall-, insbesondere Natrium- oder Kaliumzalze, Erdalkalimetall-, insbesondere Calcium- oder Magnesiumsalze, Kupfer-, Aluminium- oder Zinksalze, ebenso Ammoniumsalze mit Ammoniak oder organischen Aminen, wie gegebenenfalls C-hydroxylierten aliphatischen Aminen, insbesondere Mono-, Di- oder Triniederalkylaminen, z. B. Methyl-, Aethyl-, Dimethyl- oder Diäthylamin, Mono-, Di- oder Tri(hydroxyniederalkyl)-aminen, wie Aethanol-, Diäthanol- oder Triäthanolamin, Tris(hydroxymethyl)-aminomethan oder 2-Hydroxytertiär-butylamin, oder N-(Hydroxyniederalkyl)-N,N-diniederalkylaminen bzw. N-(Polyhydroxyniederalkyl)-N-niederalkylaminen, wie 2-(Dimethylamino)-äthanol oder D-Glucamin.

Die neuen Verbindungen der Formel I weisen wertvolle pharmakologische Eigenschaften auf. Insbesondere zeigen sie eine ausgeprägte antinociceptive Wirksamkeit sowie eine vorzügliche Hemmwirkung auf die Prostaglandinsynthese, ebenso eine ausgeprägte antiinflammatorische Wirkung. So erweisen sie sich in vivo im durch Phenyl-p-benzochinon induzierten Writhingsyndrom der Maus nach J. Pharmacol. exp. Therap. 125, 237 (1959), ebenso im Essigsäure-indizierten Writhingsyndrom der Maus bei peroralen Dosen ab etwa 1,0 mg/kg, gleichermassen im experimentellen Carrageenan-Pfotenödem sowie in der Adjuvans-Arthritis der Ratte bei peroralen Dosen ab 1 bzw. ab 0,1 mg/kg. In vitro zeigen sie z. B. im der Versuchsanordnung nach Prostaglandins 7, 123 (1974) Konzentrationsbereich ab etwa 0,1 μM/Liter eine deutliche Hemmwirkung auf die Prostaglandinsynthese aus Arachidonsäure.

Die Verbindungen der Formel I und ihre pharmazeutisch verwendbaren Salze sind dementsprechend

ausgezeichnet geeignet als Arzneimittelwirkstoffe zur Behandlung schmerzhaft-entzündlicher Erkrankung, vor allem des rheumatischen Formenkreises, wie der chronischen Arthritis.

Die Erfindung betrifft in erster Linie Verbindungen der Formel I, worin Ph unsubstituiertes oder durch Niederalkyl, Niederalkoxy, Niederalkylthio, Niederalkanoyloxy, Halogen, Trifluormethyl, Carboxy, Niederalkoxycarbonyl, Cyano und/oder Nitro substituiertes Phenyl bedeutet und R Carboxy, mit einem niederen aliphatischen Alkohol verestertes Carboxy oder als Aminogruppe unsubstituiertes oder durch niedere aliphatische Reste mono- oder disubstituiertes Amino aufweisendes Carbamyl darstellt, und ihre Salze, insbesondere pharmazeutisch verwendbaren Salze, mit Basen.

Die Erfindung betrifft vor allem Verbindungen der Formel I, worin Ph unsubstituiertes oder durch Niederalkyl, Niederalkoxy, Niederalkanoyloxy, Halogen, Trifluormethyl, Cyano und/oder Nitro mono- oder disubstituiertes Phenyl bedeutet und R Carboxy, mit einem niederen aliphatischen Alkohol verestertes Carboxy, wie Niederalkoxycarbonyl, oder als Aminogruppe unsubstituiertes oder durch niedere aliphatische Reste mono- oder disubstituiertes Amino aufweisendes Carbamyl, wie Carbamyl, N-Mono- oder N,N-Diniederalkylcarbamyl oder N,N-Niederalkelen- bzw. N,N-(Aza)niederalkylen-, N,N-(Oxa)niederalkylen- oder N,N-(Thia)niederalkylencarbamyl, darstellt, und ihre Salze, insbesondere pharmazeutisch verwendbaren Salze, mit Basen.

Die Erfindung betrifft insbesondere Verbindungen der Formel I, worin Ph unsubstituiertes oder durch Niederalkyl mit bis und mit 4 C-Atomen, wie Methyl, Niederalkoxy mit bis und mit 4 C-Atomen, wie Methoxy, Niederalkanoyloxy mit bis und mit 4 C-Atomen, wie Acetoxy, Halogen der Atomnummer bis und mit 35, wie Fluor oder Brom, und/oder Trifluormethyl mono- oder disubstituiertes Phenyl bedeutet und R Carboxy, Niederalkoxycarbonyl mit bis und mit 5 C-Atomen, wie Methoxy-, bzw. Aethoxycarbonyl, oder Carbamyl bzw. N-Niederalkylcarbamyl mit bis und mit 5 C-Atomen, wie Carbamyl bzw. N-Methylcarbamyl, bedeutet, und ihre Salze, insbesondere pharmazeutisch verwendbaren Salze, mit Basen.

Die Erfindung betrifft insbesondere ebenso Verbindungen der Formel 1, worin Ph unsubstituiertes oder durch Niederalkyl mit bis und mit 4 C-Atomen, wie Methyl, Niederalkylthio mit bis und mit 4 C-Atomen, wie Methylthio, Niederalkoxycarbonyl mit bis und mit 4 C-Atomen im Niederalkoxyteil, wie Methoxycarbonyl, Halogen der Atomnummer bis und mit 35, wie Fluor, Chlor oder Brom, und/oder Cyano, insbesondere in 2- und/oder 4-Stellung, mono- oder disubstituiertes Phenyl bedeutet und R Carboxy oder Carbamyl, ferner Niederalkoxycarbonyl mit bis und mit 4 C-Atomen im Niederalkoxyteil, wie Methoxycarbonyl, bedeutet, und ihre Salze, insbesondere pharmazeutisch verwendbaren Salze, mit Basen.

Die Erfindung betrifft in allererster Linie Verbindungen der Formel I, worin Ph durch Niederalkyl mit bis und mit 4 C-Atomen, wie Methyl, oder vorzugsweise Halogen der Atomnummer bis und mit 35, wie Fluor, Chlor oder Brom, insbesondere in 2-Stellung, monosubstituiertes oder, insbesondere in 2- und 4-Stellung, disubstituiertes Phenyl bedeutet und R Carboxy oder Carbamyl, ferner Niederalkoxycarbonyl mit bis und mit 4 C-Atomen im Niederalkoxyteil, wie Methoxycarbonyl, bedeutet, und ihre Salze, insbesondere pharmazeutisch verwendbaren Salze, mit Basen.

Die Erfindung betrifft namentlich die in den Beispielen genannte Verbindungen der Formel I und ihre pharmazeutisch verwendbaren Salze.

Die Verbindungen der Formel I können nach an sich bekannten Methoden hergestellt werden, beispielsweise indem man eine Verbindung bzw. ein Verbindungsgemisch der Formel

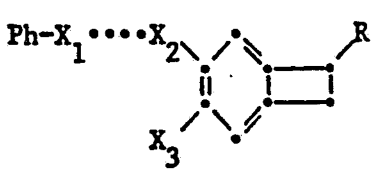

(II) ,

worin $X_1$ und $X_2$ gemeinsam eine über die Carbonylgruppe mit dem Rest Ph verbundene Gruppe der Formel -C(=O)-O- bedeutet und $X_3$ Wasserstoff ist oder einer der Reste $X_1$ und $X_3$ eine Gruppe der Formel -C(=O)-Z, der andere Wasserstoff und $X_2$ eine Gruppe R'O- bedeutet, wobei R' Niederalkyl, z. B. Methyl, oder eine Hydroxyschutzgruppe R'', wie α-Arylniederalkyl, z. B. Benzyl, Triniederalkylsilyl, z. B. Trimethylsilyl, oder eine Gruppe der Formel Ph-C(=O)- darstellt, oder sofern $X_3$ Wasserstoff ist, auch Wasserstoff bedeuten kann und Z reaktionsfähiges verestertes Hydroxy, z. B. Halogen, vor allem Chlor, oder sofern $X_1$ eine Gruppe C(=0)-Z ist, eine Gruppe Ph-C(=O)-O- darstellt, der Säurebehandlung unterwirft, das Primarprodukt ohne Isolierung solvolytisch zersetzt und gewünschtenfalls die verfahrensgemäß erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

Für die Säurebehandlung geeignete Säuren sind beispielsweise komplexe Protonensäuren, wie Schwefel-, Pyroschwefel-, Phosphor-, Pyrophosphor- oder Polyphosphorsäure oder Lewis-Säuren, wie komplexe Metallhalogenide der Formel $M^nY_n$ (III), worin M ein n-wertiges, koordinativ ungesättigtes Metallkation der Gruppe IIb, IIIa, IIIb, IVb, Va oder VIIIb des periodischen Systems der Elemente, z. B. das $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$- bzw. $Eisen^{VI}$-ion und Y ein Halogenatom der

Atomnummer bis und mit 35, wie Fluor oder Chlor, darstellt.

Die Säurebehandlung erfolgt in üblicher Weise beispielsweise in einem gegenüber den Reaktionsteilnehmern inerten Lösungsmittel, wie einem Alkan, z. B. in Hexan, Aromaten, z. B. in Benzol, Toluol oder dergleichen, Halogenalkan, z. B. Tetrachlormethan, Dichlormethan oder Trichloräthan, oder Schwefelkohlenstoff, erforderlichenfalls unter Kühlen oder Erwärmen, z. B. im Temperaturbereich von etwa -5° bis +30° C, und/oder unter Inertgas, wie Stickstoff.

Die solvolytische Zersetzung ders Primärproduktes erfolgt in üblicher Weise, beispielsweise durch Hydrolyse, d.h. Behandlung mit Wasser, aber auch durch Behandlung mit hydroxygruppenhaltigen organischen Verbindungen, wie Alkoholen, z. B. Niederalkanolen, oder Carbonsäure, z. B. Niederalkansäuren, erforderlichenfalls in Gegenwart saurer Solvolysemittel, wie von Mineralsäuren, z. B. von Salzsäure oder Schwefelsäure, oder von Ammoniumsalzen solcher Säuren, z. B. von Ammoniumchlorid, wenn angezeigt unter Kühlen, z. B. im Temperaturbereich von etwa -15° bis +20° C, und/oder unter Inertgas wie Stickstoff.

Eine bevorzugte Verfahrensweise ist z. B. dadurch gekennzeichnet, dass man auf ein Gemisch von Verbindungen der Formeln IIa und IIb bzw. auf eine Verbindung der Formel IIc

$Ph-X_1$ (IIa) und 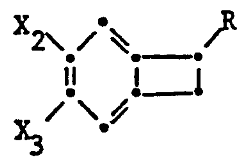 (IIb),

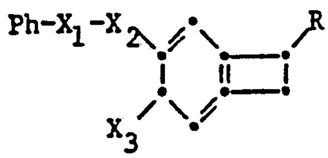 (IIc),

eine der genannten komplexen Protonensäuren und/oder deren Anhydride oder ein Metallhalogenid der Formel $M^nY_n$, vorzugsweise Zinkchlorid, Aluminiumtrichlorid, Zinntetrachlorid oder Antimonpentachlorid, einwirken lässt, wobei Y und n die vorstehend angegebenen Bedeutungen haben.

In einer besonders bevorzugten Ausführungsform dieses Verfahrens geht man beispielsweise von Verbindungen der Formeln

Ph-C(=O)-Hal (IId) und 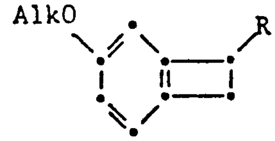 (IIe)

oder von einer Verbindung der Formel

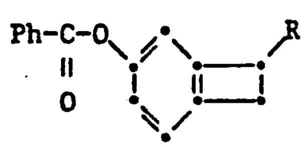 (IIf)

aus, worin Hal ein Halogen, z.B. Chlor, und Alk Niederalkyl bedeutet, und verwendet als Metallhalogenid vorzugsweise Aluminiumtrichlorid.

Die Zwischenprodukte der Formeln II können z. B. hergestellt werden, indem man eine Verbindung der Formel

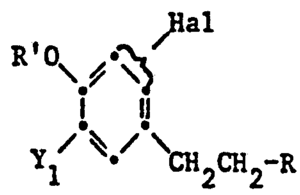

(IV),

worin $Y_1$ Wasserstoff oder gegebenenfalls verestertes Carboxy, wie Carboxy oder Niederalkoxycarbonyl, bedeutet, oder ein Phenolat- und gegebenenfalls Carboxylatsalz davon in üblicher Weise, z. B. mittels eines Alkalimetallamides, wie Kaliumamid in Ammoniak oder Lithium-N,N-diäthylamid in Tetrahydrofuran, cyclisiert und gewünschtenfalls ein Reaktionsprodukt der Formel IIb ($X_2$=OH, $X_3$ =H) mittels einer Verbindung der Formel IId O-acyliert bzw. in einem Reaktionsprodukt IIb ($X_2$=OR'', $X_3$ = gegebenenfalls verestertes Carboxy) verestertes Carboxy zu Carboxy hydrolysiert und Carboxy z. B. mittels Thionylchlorid, in Halogencarbonyl überführt.

Die Verbindungen der Formel I können ferner hergestellt werden, indem man in einer Verbindung der Formel

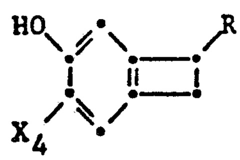

(V),

worin $X_4$ einen oxidativ oder solvolytisch in eine Gruppe der Formel -C(=O)-Ph überführbaren Rest bedeutet, $X_4$ zu der gewünschten Gruppe der Formel -C(=O)-Ph oxidiert oder solvolysiert und gewünschtenfalls die verfahrensgemäss erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

Oxidativ in eine Gruppe der Formel -C(=O)-Ph überführbare Reste sind beispielsweise solche der Formel -C($X_5X_6$)-Ph, worin $X_5$ Wasserstoff und $X_6$ Wasserstoff oder gegebenenfalls veräthertes oder verestertes Hydroxy, wie Hydroxy, Niederalkoxy, Halogen, Niederalkanoyloxy oder gegebenenfalls substituiertes Benzoyloxy, bedeuten.

Die Oxidation erfolgt in üblicher Weise durch Umsetzung mit einem geeigneten Oxidationsmittel, vorteilhaft in einem Lösungs- oder Verdünnungsmittel, erforderlichenfalls unter Kühlen oder Erwärmen, z. B. bei etwa 0 bis 100°C, in einem geschlossenen Gefäss und/oder unter Inertgas, wie Stickstoff. Geeignete Oxidationsmittel sind beispielsweise Sauerstoff, vorzugsweise in Gegenwart eines Katalysators, wie einer Silber-, Mangan-, Eisen- oder Kobaltverbindung, Perverbindugen, wie Wasserstoffperoxid, Metallperoxide, z. B. Nickelperoxid, Perkohlensäure und ihre Salze oder organische Persäuren, z. B. m-Chlorperbenzoesäure, Phthalmonopersäure oder Peressigsäure, oder ihre Salze, oxidierende Sauerstoffsäuren oder deren Salze oder Anhydride, wie unterhalogenige Säuren und ihre Salze, z. B. Natriumhypochlorit, Halogensäuren und ihre Salze, z. B. Jodsäuren, Perjodsäuren, Kaliumjodat, Natriumperiodat oder Kaliumchlorat, Salpetersäure bzw. salpetrige Säure und deren Salze und Anhydride, z. B. Kaliumnitrat, Natriumnitrit, Stickoxid, Distickstofftrioxid oder Stickstoffdioxid, oder oxidierende Schwermetallverbindungen, wie Chrom-VI-, Chrom-IV-, Mangan-IV-, Mangan-VII-, Silber-II-, Kupfer-II-, Quecksilber-II-, Vanadium-V- oder Wismut-II-verbindungen, z. B. Pyridiniumdichromat, Kaliumdichromat, Chromtrioxid, Mangandioxid, Kaliumpermanganat, Silber-II-oxid, Kupfer-II-oxid, Quecksilberoxid oder Wismutoxid. Inerte Lösungsmittel sind beispielsweise gegenüber dem jeweils zur Anwendung gelangenden Oxidationsmittel inerte Lösungsmittel, wie Wasser, Ketone, z. B. Aceton, Carbonsäuren und ihre Anhydride, z. B. Essigsäure oder Acetanhydrid, Halogenkohlenwasserstoffe, z. B. Di- oder Tetrachlormethan, oder Aromaten oder Heteroaromaten, z. B. Benzol, oder Pyridin, oder deren Gemische.

Weitere geeignete Oxidationsmittel sind beispielsweise N-Halogen-, vorzugsweise N-Chlor- oder N-Bromdicarbonsäureimide, z. B. N-Chlor- oder N-Brom-niederalkandicarbonsäureimide, wie N-Chlor- oder N-Bromsuccinimid, in Gegenwart von Thioäthern, wie aliphatischer und/oder araliphatischer Sulfide, z. B. von Niederalkylthioalkanen, wie Dimethylsulfid, oder Niederalkylthioniederalkylbenzolen, wie α-Methylthiotoluol. Weitere selektive Oxidationsmittel für Hydroxymethylen sind, z. B. Ketone, wie Diniederalkylketone, z. B. Aceton oder Methyläthylketon, gegebenenfalls ungesättigte Cycloalkanone, vorzugsweise solche mit 5 bis 7 Ringgliedern, z. B. Cyclopentanon, Cyclohexanon, Cyclohex-2-enon oder Chinone. Die Umsetzung mit Ketonen wird vorteilhaft in Gegenwart eines katalytischen Mittels und unter Verwendung eines Überschusses des oxidierenden Ketons vorgenommen. Dafür geeignete katalytische Mittel sind beispielsweise Alkoholate, vorzugsweise Niederalkanolate oder Phenolate von koordinativ ungesättigten Metallen, wie Magnesium, Bor oder vor allem Aluminium, z. B. Aluminiumphenolat oder -isopropanolat.

Solvolytisch in eine Gruppe der Formel -C(=O)-Ph überführbare Reste sind beispielsweise an der Carbonylgruppe funktionell abgewandelte Reste -C(=O)-Ph, z. B. der Formel -C($X_5X_6$)-Ph, worin $X_5$ und $X_6$ unabhängig voneinander mit einem einwertigen Alkohol veräthertes oder verestertes Hydroxy, wie Niederalkoxy, gegebenenfalls substituiertes Phenoxy, Halogen, Niederalkanoyloxy oder gegebenenfalls substituiertes Benzyloxy, gegebenenfalls mit einem einwertigen Mercaptan veräthertes Mercapto, wie Mercapto, Niederalkylthio oder gegebenenfalls substituiertes Phenylthio, oder gegebenenfalls substituiertes Amino, wie Amino, N-Mono- oder N,N-Diniederalkylamino oder gegebenenfalls substituiertes Anilino, bedeuten oder gemeinsam mit einem zweiwertigen Alkohol oder Mercaptan veräthertes Hydroxy oder Mercapto, wie Niederalkylendioxy, z. B. Aethylendioxy oder 1,3-Propylendioxy, Niederalkylendithio, z. B. Aethylendithio oder 1,3-Propylendithio, oder gegebenenfalls substituiertes 1,2-Phenylendioxy bzw. 1,2-Phenylendithio, oder Thioxo oder gegebenenfalls substituiertes Imino, wie Niederalkylimino oder gegebenenfalls substituiertes Anilo bzw. Benzylimino, darstellen.

Die solvolytische Überführung von Gruppen $X_4$ in solche der Formel -C(=O)-Ph erfolgt beispielsweise durch Hydrolyse. Die Hydrolyse wird in üblicher Weise durchgeführt, erforderlichenfalls in Gegenwart eines Lösungs- oder Verdünnungsmittels und/oder Hilfsmittels, unter Kühlen oder Erwärmen, z. B. im Temperaturbereich von etwa 0 bis 100°C, in einem geschlossenen Gefäss und/oder unter Inertgas. Geeignete Lösungs- oder Verdünnungsmittel sind beispielsweise mit Wasser mischbare Lösungsmittel, wie Alkohole oder Ketone, z. B. Niederalkanole oder Diniederalkylketone, ferner N,N-Diniederalkylamide oder N-Niederalkyllactame, z. B. Dimethylformamid oder N-Methylpyrrolidon, ferner Diniederalkylsulfoxide, z. B. Dimethylsulfoxid, ferner Dioxan oder Essigsäure. Hilfsmittel sind beispielsweise saure oder basische Solvolysemittel, wie Protonensäuren, z. B. Mineralsäuren, u.a. Chlor-, Brom- oder Jodwasserstoffsäure, Schwefelsäure oder Phosphorsäure, oder organische Carbonsäure, z. B. Niederalkansäuren, u.a. Essigsäure, oder Basen, wie Alkalimetall- oder Erdalkalimetallhydroxide, z. B. Natrium-, Kalium- oder Calciumhydroxid, Alkalimetallcarbonate, z. B. Natrium- oder Kaliumcarbonat, oder Stickstoffbasen, wie Ammoniak, für die Umesterung ferner Alkoholate, wie Alkalimetallniederalkanolate. z. B. Natriummethanolat. Ausgehend von gegebenenfalls thioketalisierten Thioxoverbindungen, z. B. der Formel V, worin $X_5$ und $X_6$ veräthertes Mercapto der gemeinsam Thioxo bedeuten kommen als weitere Hilfsmittel Schwermetallverbindungen, z. B. Quecksilberverbindungen, oder übliche Oxidationsmittel, z. B. N-Chlorsuccinimid, in Betracht.

Die Ausgangsstoffe können nach an sich bekannten Methoden hergestellt werden.

So erhält man Zwischenprodukte der Formel V, worin $X_5$ Wasserstoff und $X_6$ Hydroxy bedeutet, beispielsweise indem man in üblicher Weise Verbindungen der Formeln

Ph - $Y_2$ (VI) und 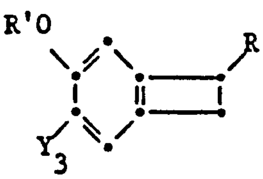 (VII),

worin R'O freies oder vorzugsweise in Salzform, z. B. in Alkali- oder Erdalkalimetallsalzform, vorliegendes oder durch eine Hydroxyschutzgruppe R'', wie Triniederalkylsilyl, z. B. Trimethylsilyl, oder α-Aralkyl, z. B. Benzyl, geschütztes Hydroxy bedeutet, miteinander umsetzt, worin einer der Reste $Y_2$ und $Y_3$, vorzugsweise $Y_2$, einen metallischen Rest, wie ein Alkalimetallatom oder eine Halogenmagnesiumgruppe, und der andere, vorzugsweise $Y_3$ Formyl darstellt, beispielsweise in einem ätherartigen Lösungsmittel, wie Tetrahydrofuran, und gegebenenfalls die Schutzgruppe R'' in üblicher Weise abspaltet. Aus so erhältlichen Verbindungen der Formel V, worin $X_4$ eine Gruppe Ph($X_5$)($X_6$)-, $X_5$ Wasserstoff und $X_6$ Hydroxy ist, sind durch übliche Veresterung mittels eines entsprechenden Carbonsäureanhydrides auch deren Ester mit organischen Carbonsäuren zugänglich. In analoger Weise erhält man auch Verbindungen der Formel V, worin $X_5$ und $X_6$ Halogen, Wasserstoff oder gemeinsam gegebenenfalls substituiertes Imino darstellen, indem man von Verbindungen der Formeln VI und VII ausgeht, worin $Y_2$ bzw. $Y_3$ einen metallischen Rest und $Y_3$ bzw. $Y_2$ eine Trihalogenmethyl-, Halogenmethyl bzw. gegebenenfalls N-substituierte α-Halogeniminomethyl- oder Cyanogruppe bedeuten.

Zwischenprodukte der Formel V, worin $X_4$ eine Gruppe -C($X_5$)($X_6$)-Ph und $X_5$ sowie $X_6$ Halogen oder gemeinsam Imino bedeuten, können aber ebenso erhalten werden, indem man eine Verbindung bzw. ein Verbindungsgemisch der Formel II, worin einer der Reste $X_1$ und $X_3$ Trihalogenmethyl oder Cyano und der andere Wasserstoff bedeutet und $X_2$ veräthertes Hydroxy ist oder $X_1$ und $X_2$ eine über die Iminomethylgruppe mit Ph verbundene Gruppe der Formel -C(=NH)-O- ist, mit einer komplexen Protonensäure, wie Schwefel- oder Polyphosphorsäure, oder vor allem einem koordinativ ungesättigtem Metallhalogenid, wie Aluminiumtrichlorid, behandelt.

Zwischenprodukte der Formel V, worin $X_5$ und $X_6$ jeweils mit einem einwertigen oder gemeinsam mit einem zweiwertigen Alkohol oder Mercaptan veräthertes Hydroxy bedeutet, können ferner hergestellt werden, indem man eine Verbindung der Formel

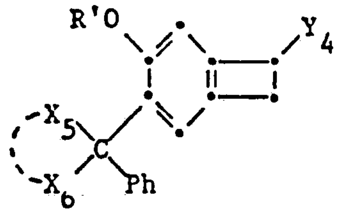

(VIII)

worin R'O freies oder vorzugsweise in einer Salzform, z. B. einer Alkalioder Erdalkalimetallsalzform, vorliegendes oder durch eine Hydroxyschutzgruppe R'', wie Triniederalkylsilyl, z. B. Trimethylsilyl, oder α-Aralkyl, z. B. Benzyl, geschütztes Hydroxy und $Y_4$ Halogen bedeutet, in üblicher Weise, z. B. durch Umsetzung mit Magnesium und anschliessend mit Kohlendioxid oder einem Halogenameisensäurederivat z. B. der Formel Hal-R (IX), oder mit einem Metallcarbonyl, wie Eisen- oder Nickeltetracarbonyl umsetzt und gegebenenfalls die Schutzgruppe R'' abspaltet. Nach dieser Methode sind auch Verbindungen der Formel V zugänglich, worin $X_5$ veräthertes Hydroxy oder Mercapto und $X_6$ Wasserstoff darstellt

Die Ausgangsstoffe können z. B. durch übliche Ketalisierung z. B. mittels eines Orthoameisensäuretriniederalkylesters, einer Verbindung der Formel

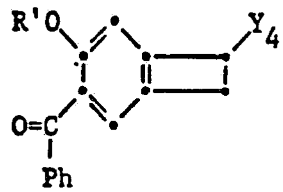

(X)

erhalten werden.

Die Verbindungen der Formel I können ferner hergestellt werden, indem man eine Verbindung der Formel

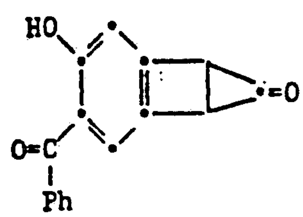

(XI)

oder ein Phenolatsalz davon, mit Wasser, einem Alkohol oder mit Ammoniak oder einem mindestens ein Wasserstoffatom aufweisenden Amin behandelt, aus einem verfahrensgemäss erhältlichen Isomerengemisch erforderlichenfalls das Isomere der Formel I isoliert und gewünschtenfalls die verfahrensgemäss erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

Dabei arbeitet man in üblicher Weise, erforderlichenfalls in Gegenwart einer Base, wie eines Alkalimetallhydroxides, -alkoholates oder -amides oder indem man einen Alkohol, Ammoniak bzw. das Amin in Form eines Metallsalzes, z. B. als Alkalimetallsalz einsetzt, vorteilhaft in einem Lösungs- oder Verdünnungsmittel, das gegenüber den Reaktionsteilnehmern inert sein oder aus einem Überschuss von Wasser des Alkohols oder des Amins bestehen kann, erforderlichenfalls unter Erwärmen, z. B. bei etwa 20 bis 100° C, und/oder unter Inertgas, wie Stickstoff.

Die Ausgangsstoffe der Formel XI werden vorteilhaft in situ hergestellt und ohne Isolierung weiterumgesetzt. Dabei geht man beispielsweise von einer Verbindung der Formel

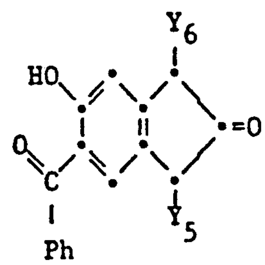

(XII),

worin einer der Reste $Y_5$ und $Y_6$ reaktionsfähiges verestertes Hydroxy wie Halogen z. B. Chlor, Brom oder Jod, oder Sulfonyloxy, wie Niederalkan- gegebenenfalls substituiertes Benzol- oder Fluorsulfonyloxy, z. B. Methan-, Aethan-, Benzol-, p-Toluol- oder Fluorsulfonyloxy, und der andere Wasserstoff bedeutet, oder einem Phenolat- und Enolatsalz davon aus und setzt dieses in Gegenwart einer geeigneten Base mit Wassar, einem Alkohol, Ammoniak bzw. einem Amin um. Geeignete Basen sind für die Umsetzung mit Wasser beispielsweise Alkalimetallhydroxide, wie Natrium- oder Kaliumhydroxid, für die Umsetzung mit einem Alkohol beispielsweise Metallalkoholate, vorzugsweise Alkalimetallalkoholate, desselben, wie dessen Natrium- oder Kaliumalkoholat, und bei der Umsetzung mit Ammoniak bzw. einem Amin entsprechende Alkalimetallamide, z.B. Natrium- oder Kaliumamide.

Die Reaktion kann aber auch so geführt werden, dass man die Verbindung der Formel XII zunächst durch Behandlung mit einer Base, als welche ausser den genannten auch Metallbasen, d.h. von Kohlenwasserstoffen abgeleitete metallorganische Verbindungen, wie Niederalkyl- oder Phenyllithiumverbindungen, Niederalkyl- oder Phenylmagnesiumhalogenide und dergleichen, in Betracht kommen, in das entsprechende Salz überführt, welches spontan zu einer Verbindung der Formel XI cyclisiert, und diese dann ohne Isolierung in der angegebenen Weise weiterumsetzt.

Die Verbindungen der Formel I können weiterhin hergestellt werden, indem man eine Verbindung der Formel

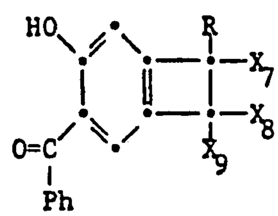

(XIII)

worin mindestens einer der Reste $X_7$ und $X_8$ gegebenenfalls veräthertes oder verestertes Hydroxy und ein davon verschiedener Rest $X_7$ oder $X_8$ sowie $X_9$ für Wasserstoff steht oder $X_7$ Wasserstoff bedeutet und $X_8$ und $X_9$ gegebenenfalls funktionell abgewandeltes Oxo darstellen, oder ein Phenolat- und/oder Carboxylatsalz davon reduziert und gewünschtenfalls die verfahrensgemäss erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

Veräthertes oder verestertes Hydroxy ist beispielsweise Niederalkoxy, gegebenenfalls substituiertes Phenoxy, Halogen, wie Chlor, Brom oder Jod, Niederalkanoyloxy oder gegebenenfalls substituiertes Benzoyloxy. Funktionell abgewandeltes Oxo ist beispielsweise Thioxo, gegebenenfalls substituiertes Imino, wie Imino oder Anilo, ketalisiertes bzw. thioketalisiertes Oxo, z. B. Bis-Niederalkoxy, wie Bismethoxy bzw. Bisäthoxy, Niederalkylendioxy, wie Aethylendioxy oder 1,3-Propylendioxy, Bis-Niederalkylthio, wie Bismethylthio, oder Niederalkylendithio, wie Aethylendithio.

Die Reduktion erfolgt in üblicher Weise, beispielsweise durch Behandlung mit katalytisch aktiviertem oder nascierendem Wasserstoff, wie mit Wasserstoff in Gegenwart eines Hydrierungskatalysators, z. B. von Platin, Nickel oder Palladium oder einer Verbindung dieser Metalle, u.a. von Platin, Platinoxid, Raney-Nickel oder Palladium, oder durch Säurebehandlung unedler Metalle oder Metallegierungen, z. B. von Zink, Natriumamalgam, amalgemiertem Aluminium und dergleichen, erzeugtem nascierendem Wasserstoff. Verbindungen der Formel XIII ($X_8$ + $X_9$ = Oxo) können vorteilhaft ferner durch Behandlung mit Hydrazin in Gegenwart einer Base, wie eines Alkalimetallhydroxides oder -alkoholates, vorteilhaft unter Erhitzen, z. B. auf

etwa 130 bis 250°C, reduziert werden. Dabei kann man auch von Verbindungen der Formel XIII ausgehen, worin $X_8$ und $X_9$ gemeinsam funktionell abgewandeltes Oxo(ausser ketalisiertem bzw. thioketalisiertem Oxo) darstellt. Aus Ketalen bzw. Thioketalen muss die Oxoverbindung zunächst, z. B. durch saure Hydrolyse, freigesetzt werden.

Falls $X_7$ eine freie Hydroxygruppe und $X_8$ und $X_9$ Wasserstoff bedeutet, kann man auch eine wässrige Suspension von Phosphor und Jod, Jodwasserstoffsäure, Zinn-II-chlorid oder ein Alkalimetall-, z. B. Natriumsulfit oder -dithionit, als Reduktionsmittel verwenden.

Die Ausgangsstoffe der Formel XIII können nach an sich bekannten Methoden hergestellt werden, beispielsweise indem man in eine Verbindung der Formel

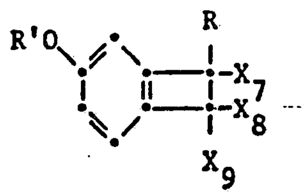

(XIV)

wobei R' Wasserstoff, Niederalkyl, wie Methyl, oder eine Hydroxyschutzgruppe R", wie α-Aralkyl, z. B. Benzyl, oder Tri-niederalkylsilyl, z. B. Trimethylsilyl, oder eine Gruppe der Formel Ph-C(=O)- bedeutet, in üblicher Weise, z. B. wie für die Verbindungen der Formeln II angegeben, die Gruppe der Formel Ph-C(=O)-einführt.

Verbindungen der Formel XIV, worin $X_7$ Wasserstoff und $X_8$ und $X_9$ Oxo ist, sind ihrerseits beispielsweise durch Ringschluss einer Verbindung der Formel

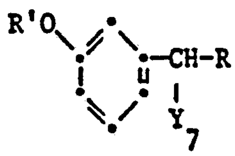

(XV)

worin $Y_7$ gegebenenfalls verestertes oder mit einer Mineralsäure anhydridisiertes Carboxy, wie Carboxy, Niederalkoxycarbonyl oder Chlorcarbonyl bedeutet, z. B. unter den Bedingungen der Kondensation bzw. Umlagerungen von Verbindungen der Formel II, oder ausgehend von einer Verbindung der Formel

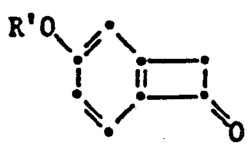

(XVI)

durch Überführung in das Enolatsatz, z. B. mittels einer Metallbase, wie Natriumhydrid, und anschliessende Umsetzung mit einem reaktiven Kohlensäurederivat, wie Kohlendioxid, einem Kohlensäurediester, Halogenameisensäureester oder Carbamylchlorid, oder durch α-Halogenierung, z. B. mit Brom, N-Bromsuccinimid oder N-Chlorsuccinimid, Umsetzung mit einem Alkalimetallcyanid und nachfolgende Solvolyse der Cyanogruppe zu einer Gruppe R zugänglich. In analoger Weise erhält man auch Verbindungen der Formel XIV ($X_9$ = Hydroxy; $X_7$, $X_8$ = H) durch Überführung eines Aldehydes der Formel

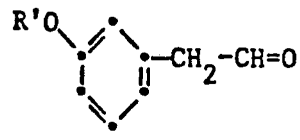

(XVII)

in ein Salz, Umsetzung mit einem reaktiven Kohlensäurederivat zu einer Verbindung der Formel

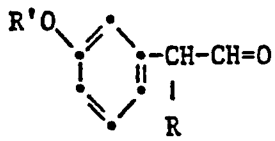

(XVIII)

und nachfolgenden Ringschluss. Anschliessend wird dann die Gruppe Ph-C(=O)- in der für Verbindungen der Formel II angegebenen Weise eingeführt.

Verbindungen der Formel XIII, worin $X_7$ Hydroxy bedeutet und $X_8$ und $X_9$ Wasserstoff darstellen, können ferner hergestellt werden, indem man eine Verbindung der Formel

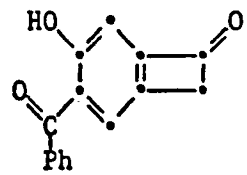

(XIX)

in üblicher Weise mit wässrigem Alkalimetallcyanid umsetzt und in dem erhaltenen α-Hydroxynitril der Formel

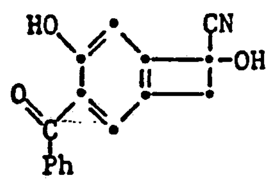

(XX)

die Cyanogruppe in üblicher Weise in gegebenenfalls verestertes oder amidiertes Carboxy R überführt.

Die Verbindungen der Formel I kann man ferner herstellen, indem man in einem von einem Ester oder Amid der Formel I verschiedenen funktionellen Carboxyderivat derselben oder einem Salz davon die funktionell abgewandelte Carboxygruppe solvolytisch in eine gegebenenfalls veresterte oder amidierte Carboxygruppe überführt und gewünschtenfalls eine verfahrungsgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung oder in ein anderes Salz überführt, und/oder ein verfahrensgemäss erhältliches Isomerengemisch in die Komponenten auftrennt.

Derartige funktionelle Carboxyderivate sind beispielsweise Säureanhydride, wia gemischte Anhydride mit Carbonsäuren, wie Niederalkansäuren, z. B. mit Ameisen- oder Essigsäure, oder anorganischen Säuren, wie Halogenwasserstoffsäuren, z. B. mit Chlorwasserstoffsäure, oder Cyanwasserstoffsäure, oder innere Anhydride, d.h. Ketone, Ortho-Anhydride, wie entsprechende Trihalogen- z. B. Trichlor- oder Tribrommethylverbindungen, Ortho-Anhydridester, wie Estern der Formel I entsprechende Dihalogenmethanolester, z. B. Niederalkoxydihalogenmethylverbindungen, Orthoester, wie Estern der Formel I

entsprechende Orthoester, z. B. Triniederalkoxymethylverbindungen, Iminoäther und Amidine, wie Estern oder Amiden der Formel I entsprechende Iminoäther, Iminoester und Amidine oder Niederalkyleniminoäther, wie als funktionell abgewandelte Carboxygruppe 4,4-oder 5,5-Dimethyl-oxazinyl-(2) oder 4,6,6-Trimethyl-dihydrooxazinyl-(2) aufweisende Iminoäther, Mono- und Dithiocarbonsäuren und deren Ester und Amide, vorzugsweise jedoch Nitrile.

Funktionelle Carboxyderivate von Verbindungen der Formel I sind beispielsweise Verbindungen der Formel

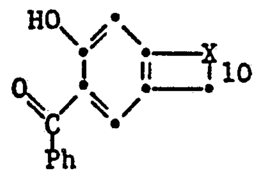

(XXI) ;

worin $X_{10}$ eine Gruppe der Formeln 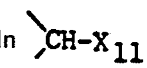

oder 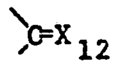

bedeutet, $X_{11}$ von veresterten oder amidierten Carboxy R verschiedenes funktionell abgewandeltes Carboxy, wie anhydridisiertes Carboxy, Trihalogenmethyl, veräthertes Hydroxydihalogenmethyl, z. B. Niederalkoxydichlormethyl, veräthertes Trihydroxymethyl, z. B. Triniederalkoxymethyl, veräthertes oder verestertes C-Hydroxyiminomethyl, z. B. C-Niederalkoxyiminomethyl, 4,4- oder 5,5-Dimethyloxa-zinyl-(2), 4,6,6-Trimethyldihydro-oxazinyl-(2) oder C-Halogeniminomethyl, gegebenenfalls veräthertes oder amidiertes Mono- oder Dithiocarboxy oder Cyano, und $X_{12}$ gegebenenfalls funktionell abgewandeltes, wie acetalisiertes oder thioacetalisiertes Carbonyl, z. B. 1,3-Dithian-2-yliden, darstellt.

Die Solvolyse erfolgt in üblicher Weise, beispielsweise durch Hydrolyse, Alkoholyse, d.h. Umsetzung mit einem Alkohol oder Ammonolyse bzw. Aminolyse, d.h. Umsetzung mit Ammoniak oder einem mindestens ein Wasserstoffatom aufweisenden Amin.

Durch Hydrolyse kann man beispielsweise eines der genannten funktionellen Carboxyderivate, ferner Ester und Amide, in die Carbonsäuran der Formel I (R = Carboxy), ferner Ortho-Anhydridester, Orthoester und Iminoäther in Ester der Formel I (R = verestertes Carboxy) und Iminoester, Amidine bzw. Nitrile in Amide (R = amidiertes Carboxy) überführen.

Die Hydrolyse erfolgt in üblicher Weise durch Behandlung mit Wasser, erforderlichenfalls in Gegenwart eines Hydrolysemittels, in einem Lösungs- oder Verdünnungsmittel, unter Kühlen oder Erwärmen, z. B. im Temperaturbereich von etwa 0 bis 100°C, und/oder unter Inertgas, wie Stickstoff, Hydrolysemittel sind beispielsweise saure oder basische Hydrolysemittel. Saure Hydrolysemittel sind beispielsweise Protonensäuren, wie Mineralsäuren, z. B. Schwefelsäure, wie Halogen-, z. B. Chlor-, Brom- oder Jodwasserstoffsäure, oder Phosphorsäure, Sulfonsäuren, wie p-Toluolsulfonsäure, oder Carbonsäuren, z. B. Niederalkansäuren, z. B. Ameisen- oder Essigsäure. Basische Hydrolysemittel sind beispielsweise Hydroxide oder Carbonate von Alkali- oder Erdalkalimetallen, z. B. Natrium- oder Kaliumhydroxid, Natrium- oder Kaliumcarbonat oder Calciumhydroxid, oder Stickstoffbasen, wie Ammoniak oder organische Amine, wie Triniederalkylamine, Pyridin oder Benzyl-triäthylamoniumhydroxid. Lösungs- oder Verdünnungsmittel sind beispielsweise mit Wasser mischbare organische Lösungsmittel, wie Alkohole, z. B. Niederalkanole, Ketone, z. B. Diniederalkylketone, N,N-Diniederalkylniederalkansäureamide bzw. N-Niederalkylniederalkansäurelactame, z. B. Dimethylformamid oder N-Methylpyrrolidon, oder Diniederalkylsulfoxide, z. B. Dimethylsulfoxid.

Durch Alkoholyse kann man beispielsweise Säureanhydride, wie gemischte Anhydride mit Carbonsäuren oder anorganischen Säuren oder Ketene, in Ester der Formel I (R = verestertes Carboxy) überführen.

Die Alkoholyse erfolgt in üblicher Weise, erforderlichenfalls in Gegenwart eines Kondensationsmittels, in einem Lösungsmittel, unter Kühlen oder Erwärmen, z. B. im Temperaturbereich von etwa 0° bis 150°C, in einem geschlossenen Gefäss und/oder unter Inertgas, wie Stickstoff. Kondensationsmittel sind beispielsweise saure Kondensationsmittel, wie Mineralsäuren, z. B. Schwefelsäure oder Halogen-, wie Chlor-, Brom- oder Jodwasserstoffsäure, oder vor allem basische Kondensationsmittel, wie Alkalimetallalkoholate entsprechender Alkohole, wie Alkalimetallniederalkanolate, z. B. Natriummethanolat oder Natriumäthanolat, Alkalimetallhydroxide oder -carbonate, z. B. Natrium- oder Kaliumhydroxid oder Kaliumcarbonat, oder tertiäre Stickstoffbasen, wie heteroaromatische Stickstoffbasen, z. B. Pyridin, oder Triniederalkylamine, z. B. Triäthylamin. Als Lösungsmittel kommen neben einem Überschuss des betreffenden Alkohols bzw. der verwendeten tertiären Stickstoffbase beispielsweise Aether, wie Diniederalkyl- oder Niederalkylenäther, z. B. Diäthyläther, Tetrahydrofuran oder Dioxan, Kohlenwasserstoffe, wie aromatische oder araliphatische Kohlenwasserstoffe, z. B. Benzol, Toluol oder Xylole, N,N-Diniederalkylniederalkansäureamide bzw. N-Niederalkylniederalkansäurelactame, z. B. Dimethylformamid oder N-Methylpyrrolidon, oder Diniederalkylsulfoxide, z. B. Dimethylsulfoxid, in Betracht.

Durch Ammono- bzw. Aminolyse kann man beispielsweise Säureanhydride, wie gemischte Anhydride mit Carbonsäuren oder Mineralsäuren und Ketene, in Amide der Formel I (R = amidiertes Carboxy) überführen.

Die Ammono- bzw. Aminolyse erfolgt in üblicher Weise, erforderlichenfalls in Gegenwart eines Kondensationsmittels, in einem Lösungsmittel, unter Kühlen oder Erwärmen, z. B. im Temperaturbereich von etwa 0 bis 150°C, in einem geschlossenen Gefäss und/oder unter Inertgas, wie Stickstoff. Kondensationsmittel sind beispielsweise basische Kondensationsmittel, wie Alkalimetallhydroxide- oder carbonate, z. B. Natrium- oder Kaliumhydroxid oder Kaliumcarbonat, oder tertiäre organische Stickstoffbasen, wie heteroaromatische Stickstoffbasen, z. B. Pyridin, oder Triniederalkylamine, z. B. Triäthylamin. Als Lösungsmittel kommen neben einem Überschuss eines gegebenenfalls verwendeten organischen Amins beispielsweise Aether, wie Diniederalkyl- oder Niederalkylenäther, z. B. Diäthyläther, Tetrahydrofuran oder Dioxan, Kohlenwasserstoffe, wie aromatische oder araliphatische Kohlenwasserstoffe, z. B. Benzol, Toluol oder Xylole, N,N-Diniederalkylniederalkansäureamide bzw. N-Niederalkylniederalkansäurelactame, z. B. Dimethylformamid oder N-Methylpyrrolidon, oder Diniederalkylsulfoxide, z. B. Dimethylsulfoxid, in Betracht.

Die Verbindungen der Formel I können ferner hergestellt werden, indem man in einer Verbindung der Formel

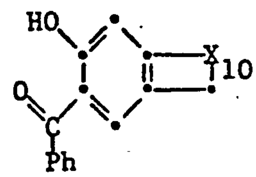

(XXI)

worin $X_{10}$ eine Gruppe der Formel 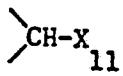

und $X_{11}$ einen oxidativ in eine Gruppe R überführbaren Rest bedeutet, den Rest $X_{11}$ zu einer Gruppe R oxidiert und gewünschtenfalls eine verfahrensgemäss erhältliche Verbindung in eine andere Verbindung der Formel I überführt und/oder eine verfahrensgemäss erhältliche freie Verbindung in ein Salz oder ein Verfahren gemäss erhältliches Salz in die freie Verbindung oder in ein anderes Salz überführt.

Oxidativ in Gruppen R überführbare Reste sind beispielsweise zu Carboxy R oxidierbare, von organischen Carbonsäuren abgeleitete Acylgruppen, gegebenenfalls substituierte, wie einen Phenylrest aufweisende, Niederalkenyl- bzw. Niederalkinylreste, Methyl, Aminomethyl oder Hydroxymethyl, ferner gegebenenfalls in 5-Stellung substituiertes, wie Diniederalkoxymethyl aufweisendes, 2-Furyl. Zu Carboxy oxidierbare, von organischen Carbonsäuren abgeleitetes Acyl $X_{11}$ ist beispielsweise gegebenenfalls substituiertes, z.B. in 2-Stellung durch freies oder funktionell abgewandeltes Carboxy, H8alogen oder Oxo und/oder in 2- und/oder 3-Stellung Hydroxy aufweisendes, in höherer als der 2-Stellung gegebenenfalls zusätzlich durch gegebenenfalls verestertes Carboxy oder eine Phenylgruppe substituiertes Niederalkanoyl, bzw. Niederalkenoyl, wie Niederalkanoyl, insbesondere gegebenenfalls in Hydratform, Enolätherform, z. B. als Enoläther mit einem Alkohol der Formel $R_0$-OH, worin $R_0$ einen Kohlenwasserstoffrest, wie Niederalkyl, bedeutet als Acetal, z. B. Diniederalkyl- oder Niederalkylenacetal, oder Acylat, z. B. als Diniederalkanoyloxy- oder Dihalogenmethylgruppe vorliegendes Formyl, ferner Acetyl, Propionyl, Pivaloyl und dergleichen, gegebenenfalls funktionell abgewandeltes, wie verestertes oder amidiertes Oxalo, z. B. Oxalo oder Niederalkoxyoxalyl, 2-Mono-, 2,2-Di- oder 2,2,2-Tri-halogenniederalkanoyl, z. B. Mono-, Di- oder Trichloracetyl, Mono-, Di- oder Tribromacetyl oder Mono-, Di- oder Trijodacetyl, 2-Oxoniederalkanoyl oder 2-Oxophenylniederalkanoyl, wie Pyruvoyl oder Benzoylcarbonyl, 2-Hydroxyniederalkanoyl, z. B. Glykoloyl, oder gegebenenfalls funktionell abgewandeltes, z. B. verestertes oder amidiertes 3-Carboxy-2,3-dihydroxypropionyl, wie Tartrono oder Niederalkoxytartronoyl, oder gegebenenfalls durch eine Phenylgruppe substituiertes Niederalkenoyl, z. B. Acryloyl, Methacryloyl, Crotonyl, Cinnamoyl und dergleichen. Weitere Gruppen $X_{11}$ sind verätherte Hydroxymethylgruppen, wie Niederalkoxymethyl, die zu verestertem Carboxy, wie Niederalkoxycarbonyl, R oxidiert werden können.

Die Oxidation erfolgt in üblicher Weise durch Umsetzung mit einem geeigneten Oxidationsmittel, vorteilhaft in einem Lösungs- oder Verdünnungsmittel, erforderlichenfalls unter Kühlen oder Erwärmen, z. B. bei etwa 0 bis 100°C, in einem geschlossenen Gefäss und/oder unter Inertgas, wie Stickstoff. Geeignete Oxidationsmittel sind beispielsweise Sauerstoff, vorzugsweise in Gegenwart eines Katalysators, wie einer Silber-, Mangan-, Eisen- oder Kobaltverbindung, Perverbindungen, wie Wasserstoffperoxid, Metallperoxide, z. B. Nickelperoxid, Perkohlensäure und ihre Salze oder organische Persäuren, z. B. m-Chlorperbenzoesäure, Phthalmonopersäure oder Peressigsäure, oder ihre Salze, oxidierende Sauerstoffsäuren oder deren Salze oder Anhydride, wie unterhalogenige Säuren und ihre Salze, z. B. Natriumhypochlorit Halogensäuren und ihre Salze, z. B. Natriumhypochlorit, Halogensäuren und ihre Salze, z. B. Jodsäuren, Perjodsäuren, Kaliumjodat, Natriumperjodat oder Kaliumchlorat, Salpetersäure bzw. salpetrige Säure und deren Salze und Anhydride, z. B. Kaliumnitrat, Natriumnitrit, Stickoxid, Distickstofftrioxid oder Stickstoffdioxid, oder oxidierende Schwermetallverbindungen, wie Chrom-VI-, Chrom-IV-, Mangan-IV-, Mangan-VII-, Silber-II-, Kupfer-II-, Quecksilber-II-, Vanadium-V- oder Wismut-II-verbindungen, z. B. Natriumdichromat, Kaliumdichromat,

Chromtrioxid, Mangandioxid, Kaliumpermanganat, Silber-II-oxid, Kupfer-II-oxid, Quecksilberoxid oder Wismutoxid. Inerte Lösungsmittel sind beispielsweise gegenüber dem jeweils zur Anwendung gelangenden Oxidationsmittel inerte Lösungsmittel, wie Wasser, Ketone, z. B. Aceton, Carbonsäuren und ihre Anhydride, z. B. Essigsäure oder Acetanhydrid, Halogenkohlenwasserstoffe, z. B. Tetrachlormethan, oder Aromaten oder Heteroaromaten, z. B. Benzol, oder Pyridin, oder deren Gemische.

Gegebenenfalls verestertes Oxalo $X_{11}$ der Formel -C(=O)-R kann ferner unter Decarbonylierung, beispielsweise in Gegenwart eines geeigneten Oxidationsmittels, erforderlichenfalls unter Kühlen oder Erwärmen, z. B. bei etwa 0 bis 100°C, in einem inerten Lösungsmittel, in einem geschlossenen Gefäss und/oder unter Inertgas, wie Stickstoff, oxidiert werden. Geeignete Oxidationsmittel sind beispielsweise Perverbindungen, wie Wasserstoffperoxid oder Percarbonsäuren, z. B. Perkohlensäure oder organische Percarbonsäuren, u.a. Peressigsäure, Perameisensäure, Perbenzoesäure und dergleichen, ferner Halogensauerstoffsäuren und ihre Salze, wie Chlorsäure bzw. Alkalimetallchlorite, oder oxidierende Metallverbindungen, wie Silberoxid, Kaliumpermanganat oder Chromtrioxid. Geeignete Lösungsmittel sind beispielsweise gegebenenfalls wasserhaltige Ketene, wie Aceton, oder Carbonsäuren, wie Essigsäuren.

Die als Ausgangsstoffe genannten Verbindungen der Formel XXI, worin $X_{10}$ die angegebene Bedeutung hat und insbesondere eine Gruppe 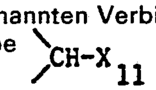

darstellt, können nach an sich bekannten Methoden hergestellt werden, beispielsweise in Abwandlung einiger der vorstehend beschriebenen Verfahrensweisen zur Herstellung der Formel I, wobei man jeweils von entsprechenden Ausgangsstoffen der Formeln IIa und IIb bzw. IId und IIe, IIc bzw. IIf, V, VII oder XXXVII ausgeht, die anstelle der Gruppe 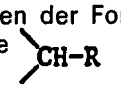

eine Gruppe $X_{10}$ vorzugsweise der Formel 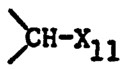

aufweisen.

Eine bevorzugte Verfahrensweise besteht z. B. darin, dass man eine Verbindung der Formel

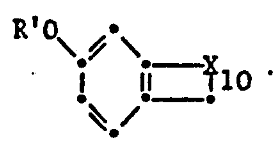

(XXII)

wobei R' Wasserstoff, Niederalkyl, wie Methyl, oder eine Hydroxyschutzgruppe R'', wie α-Aralkyl, z. B. Butyl, oder Triniederalkylsilyl, z. B. Trimethylsilyl, oder eine Cruppe der Formel Ph-C(=O)- bedeutet, in Gegenwart von Aluminiumchlorid oder eines anderen der genannten komplexen Metallhalogenide mit einer Verbindung Ph-C(=O)Hal (IId) umsetzt, wobei R' vorzugsweise einen Niederalkylrest und Hal Halogen, z. B. Chlor bedeutet.

Die als Ausgangsstoffe zu verwendenden Ketene, d.h. Verbindungen der Formel XXI, worin $X_{10}$ eine Gruppe der Formel 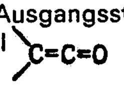

darstellt, können ferner hergestellt werden, indem man ein entsprechendes α-Diazoketon, z. B. eine Verbindung der Formel

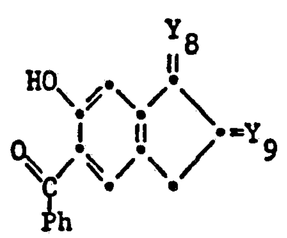

(XXIII)

worin einer der Reste $Y_8$ und $Y_9$ Oxo und der andere eine Gruppe der Formel $=N_2$ bedeutet, in üblicher Weise unter Abspaltung von Stickstoff umlagert. Führt man die Umlagerung in Gegenwart von Wasser, einem

Alkohol oder von Anmoniak bzw. einem mindestens ein Wasserstoffatom aufweisenden Amin durch, reagiert das primär gebildete Keten ohne Isolierung erfindungsgemäss weiter. Dabei arbeitet man vorteilhaft bei erhöhtar Temperatur, z. B. im Temperaturbereich von etwa 40 bis 100°C, unter Belichtung, z. B. mit einer Ultraviolettlichtquelle, und/oder in Gegenwart von Silber oder Silberwerbindungen, z. B. von Silber, Silbernitrat oder Silberoxid. Diazoketone der Formel XXIII können z. B. erhalten werden, indem man eine Verbindung der Formeln

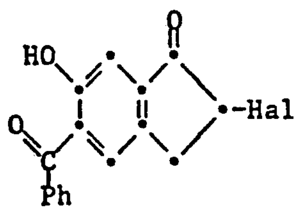 (XXIV) bzw. 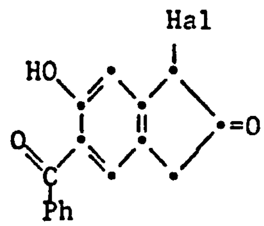 (XXV)

worin Hal Halogen bedeutet, zugänglich aus dem entsprechenden Indanon durch Halogenierung, z. B. mit N-Bromsuccinimid, mit Hydrazin umsetzt, z. B. bei etwa 60°C in Aethanol, und das Primärprodukt oxidiert, z. B. durch Behandlung mit Mangandioxid in Chloroform.

Ketene der Formel XXI, worin $X_{10}$ eine Gruppe der Formel $>C=C=O$

ist kann man ferner erhalten, indem man ein Diketon der Formel

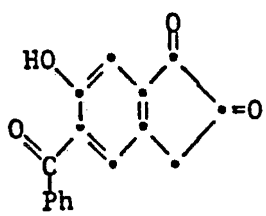 (XXVI)

mit einem Sulfonsäurehydrazid, z. B. mit p-Toluolsulfonsäurehydrazid, umsetzt und das gebildete Hydrazon XXIII ($Y_8$ bzw. $Y_9$ = N-$NH_2$ statt =$N_2$) mit Alkali, z. B. mit Natronlauge, behandelt, oder durch Umsetzung mit Hydroxylamin oximiert und das Oxim (in Formel XXIII ist $Y_8$ bzw. $Y_9$=N-OH statt =$N_2$) mit Chloramin bzw. Ammoniak und Natriumhypochlorit, behandelt. Man kann zu den genannten Oximen auch gelangen, indem man ein entsprechendes Indanon mit salpetriger Säure kondensiert. Ebenso kann man die genannten Hydrazon erhalten, indem man eine Verbindung der Formeln XXIV oder XXV aminiert und das Primärprodukt in üblicher Weise, z. B. mit Natriumnitrit und Essigsäure, diazotiert.

Von Säuren der Formel I abgeleitete Nitrile, z. B. Verbindungen der Formel XXI, worin $X_{10}$ eine Gruppe

$>CH-X_{11}$

und $X_{11}$ Cyano bedeutet, können ferner hergestellt werden, indem man eine Verbindung der Formel

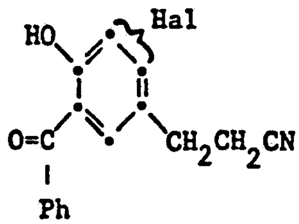

(IV')

oder ein Salz davon in üblicher Weise, z. B. mittels eines Alkalimetallamides, wie Kaliumamid in Ammoniak oder Lithium-N,N-diäthylamid in Tetrahydrofuran, cyclisiert oder indem man eine Verbindung der Formel

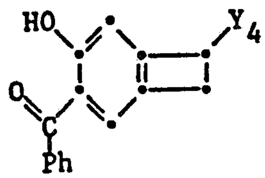

(XXVII),

worin $Y_4$ reaktionsfähiges verestertes Hydroxy, wie Halogen oder Sulfonyloxy, z. B. Jod, Brom, Chlor oder p-Toluolsulfonyloxy, bedeutet, oder ein Phenolatsalz davon in üblicher Weise mit Ammonium- oder einem Metallcyanid, z. B. mit Natrium- oder Kaliumcyanid, umsetzt. Verbindungen der Formel XXVII ihrerseits können z. B. erhalten werden, indem man ein entsprechendes Benzocyclobutenverbindung halogeniert, z. B. durch Behandlung mit einem Halogen oder mittels N-Brom- oder N-Chlorsuccinimid, oder in einer entsprechenden Benzocyclobutenonverbindung die Oxogruppe zu Hydroxy reduziert und dieses durch Umsetzung mit einem Halogenierungsmittel, wie Phosphortribromid, oder einem Sulfonsäurehalogenid umsetzt. Man kann aber auch an eine Verbindung der Formel

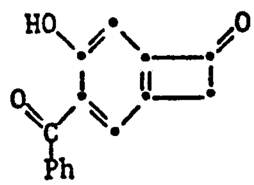

(XXVIII)

Cyanwasserstoffsäure addieren und in dem gebildeten α-Cyanhydrin die Hydroxygruppe, gegebenenfalls unter vorhergehender Wasserabspaltung, reduktiv durch Wasserstoff ersetzen, z. B. wie für entsprechende Verbindungen der Formel XIII angegeben.

Die Verbindungen der Formel XXVIII können z. B. hergestellt werden, indem man eine Verbindung der Formel

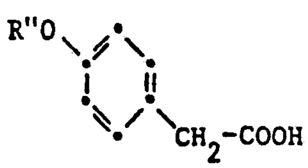

(XXIX),

worin R" z. B. Niederalkyl ist, in üblicher Weise, z. B. durch Säurebehandlung, wie durch Erhitzen mit Schwefelsäure oder Polyphosphorsäure cyclisiert und die erhaltene Verbindung der Formel

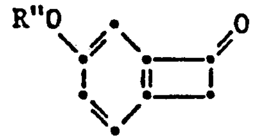

(XXX)

in Gegenwart von Aluminiumtrichlorid mit einer Verbindung der Formel
Ph-C(=O)Hal (IId)
umsetzt.

Als Ausgangsstoffe zu verwendende Iminoäther und Amidine werden vorteilhaft in situ hergestellt, indem man das entsprechende Nitril mit einem Alkohol oder Amin behandelt, üblicherweise in Gegenwart einer Mineralsäure, oder indem man das Nitril zunächst durch Mineralsäurebehandlung in einem Iminoester überführt und anschliessend der Alkoholyse bzw. Ammono- oder Aminolyse unterwirft. Als Mineralsäure ist dabei vor allem Chlor- oder Bromwasserstoffsäure geeignet. In analoger Weise kann man auch Orthoanhydridester durch partielle Alkoholyse von Orthoanhydriden, z. B. Trihalogenmethylverbindungen, herstellen.

Verbindungen der Formel XXI, worin $X_{10}$ eine Gruppe der Formel $>CH-X_{11}$ und $X_{11}$ eine 4,4- oder 5,5-Dimethyl-oxazinyl-(2)- oder 4,6,6-Trimethyl-dihydro-oxazinylgruppe, acetalisiertes Formyl oder eine Orthoestergruppe bedeutet, können ferner hergestellt werden, indem man in üblicher Weise Verbindungen der Formeln

$Ph-Y_2$ (VI) und 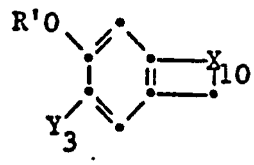 (XXXI)

miteinander umsetzt, worin einer der Reste $Y_2$ und $Y_3$, vorzugsweise $Y_2$ einen metallischen Rest, wie ein Alkalimetallatom oder eine Halogenmagnesiumgruppe, und der andere, vorzugsweise $Y_3$, gegebenenfalls in Salzform, z. B. als Alkalimetallsalz, vorliegende oder funktionell abgewandeltes, wie verestertes oder in einer Anhydridform, z. B. als Halogenid vorliegendes Carboxy bedeutet, und R'O- freies oder vorzugsweise in einer Salzform, z. B. einer Alkali- oder Erdalkalimetallsalzform, vorliegendes oder durch eine Hydroxyschutzgruppe R'', wie Triniederalkylsilyl, z. B. Trimethylsilyl, oder α-Aralkyl, z. B. Benzyl, geschütztes Hydroxy bedeutet und gegebenenfalls die Hydroxyschutzgruppe R'' abspaltet.

In einer Abwandlung dieses Verfahrens kann man auch von Verbindungen der Formeln VI und XXI ausgehen, die als funktionell abgewandelte Carboxygruppe Cyano enthalten, man erhält so das Iminoderivat der Verbindung der Formel XXI, dessen Iminogruppe im Verlaufe der Hydrolysereaktion zu Oxo hydrolisiert wird.

In Enolätherform vorliegende Aldehyde der Formel XXI, worin $X_{10}$ eine Gruppe $>C=CH-OR_o$ bedeutet, können ferner hergestellt werden, indem man eine Verbindung der Formel

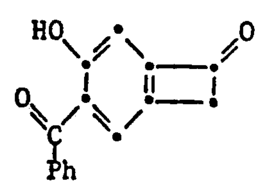

(XXXII)

oder ein Phenolatsalz davon, in üblicher Weise mit einer Verbindung der Formel
$(R_1)_3P = CH_2 - OR_o$ (XXXIII),
worin die Reste $R_0$ und $R_1$ gleiche oder verschiedene Kohlenwasserstoffreste bedeuten, z. B. $(R_1)_3P$ Triphenylphosphino und $R_0$ Niederalkyl ist, umsetzt.

Aus diesen können die Aldehyde der Formel XXI, worin $X_{10}$ eine Gruppe der Formel

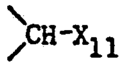

und $X_{11}$ gegebenenfalls in Hydratform vorliegendes Formyl bedeutet, gewünschtenfalls durch milde Hydrolyse freigesetzt werden, vorteilhaft unter Säurekatalyse. Ebenso kann man Acetale und Acylale, worin $X_{10}$ eine Gruppe der Formel 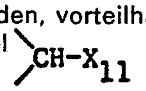

und $X_{11}$ acetalisiertes oder acylalisiertes Formyl ist, zu den entsprechenden Aldehyden hydrolysieren.

Verbindungen der Formel XXI, worin $X_{10}$ eine Gruppe der Formel 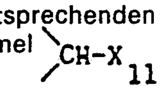

und $X_{11}$ eine gegebenenfalls veresterte Oxalgruppe können ferner erhalten werden, indem man eine Verbindung der Formel

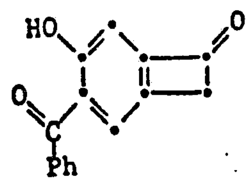

(XXVIII)

in üblicher Weise, z. B. in Gegenwart von Acetanhydrid, bei etwa 50 bis 100°C, mit Hippursäure kondensiert und das Kondensationsprodukt der Formel

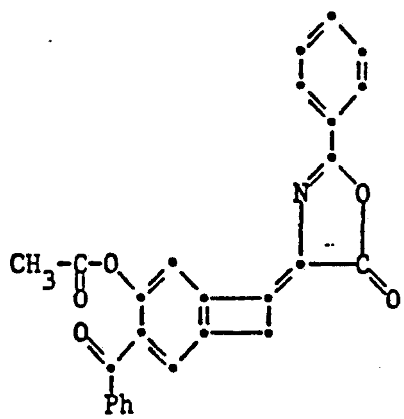

(XXXIV)

in üblicher Weise z. B. durch Behandeln mit Natronlauge hydrolysiert.

Verbindungen der Formel XXI, worin $X_{10}$ eine Gruppe 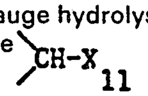

und $X_{11}$ gegebenenfalls in Hydratform vorliegendes Formyl ist, können ferner unter den Bedingungen ihrer Weiteroxidation in situ hergestellt werden, indem man die Formylgruppe durch Oxidation z. B. von Methyl, Hydroxymethyl oder gegebenenfalls substituierten Niederalkenyl oder gegebenenfalls wie angegebenen substituierten Niederalkanoyl bzw. Niederalkenoyl bildet oder aus einem ihrer Derivate, welches anstelle von Formyl $X_{11}$ funktionelles abgewandeltes Formyl aufweist, wie einem Acetal, z. B. aus Diniederalkoxy- oder Niederalkylendioxymethyl, Acylal, z. B. aus Dihalogenmethyl, Enoläther, z. B. einem Enolniederalkyläther, Enolester, z. B. Enolniederalkansäureester oder Enolester mit Halogenwasserstoffsäuren, Oxim oder Enamin, z. B. Enamin mit Pyrrolidin, Piperidin, Morpholin, Thiomorpholin oder einem Diniederalkylamin, in Freiheit setzt, vorzugsweise hydrolytisch, z. B. unter sauren Bedingungen. Die erwähnten Acetale kann man beispielsweise erhalten, indem man eine Verbindung der Formel

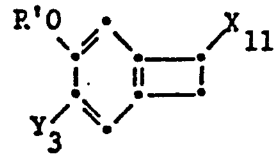

(XXXV),

worin R'O freies oder vorzugsweise in einer Salzform, z. B. einer Alkali- oder Erdalkalimetallsalzform, vorliegendes oder durch eine Hydroxyschutzgruppe R'', wie Triniederalkylsilyl, z. B. Trimethylsilyl, oder α-Aralkyl, z. B. Benzyl, geschütztes Hydroxy bedeutet mit einer Verbindung der Formel

Ph - $Y_2$ (V)

umsetzt, worin $X_{11}$ acetalisiertes Formyl ist une einer der Reste $Y_2$ und $Y_3$ eine Metallgruppe z. B. die Formel -Li, -Na, -Mg/2, -Mg-Hal, -Cd/2, -CdHal oder ZnHal (Hal = Halogen, wie Chlor, Brom oder Jod) ist und der andere eine in Salzform vorliegende oder veresterte Hydroxygruppe bedeutet, und erforderlichenfalls die Schutzgruppe abspaltet, z. B. mittels Magnesiumjodid. Analog kann man Verbindungen der Formel XXI, worin $X_{10}$ eine Gruppe der Formel 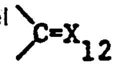

und $X_{12}$ 1,3-Dithian-2-yliden bedeutet, ferner erhalten, indem man eine Verbindung der Formel

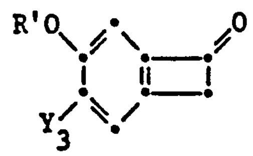

(XXXVI),

worin R' eine Hydroxyschutzgruppe bedeutet, mit 2-Trimethylsilyl-1,3-dithian-2-yl-lithium umsetzt, das Reaktionsprodukt mit einer Verbindung der Formel V umsetzt und erforderlichenfalls die Schutzgruppe abspaltet.

Die Verbindungen der Formel I können ferner hergestellt werden, indem man in einer Verbindung der Formel

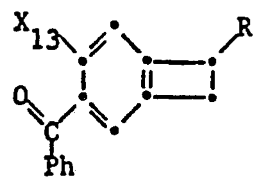

(XXXVII),

worin $X_{13}$ eine in Hydroxy überführbare Gruppe darstellt, oder in einem Salz davon $X_{13}$ in Hydroxy überführt und gewünschtenfalls die verfahrensgemäss erhältliche Verbindung in eine andere Verbindung dar Formel I umwandelt und/oder eine verfahrensgemäss erhältliche Verbindung in ein Salz oder ein verfahrensgemäss erhältliches Salz in die freie Verbindung oder in ein anderes Salz überführt.

In Hydroxy überführbare Gruppen sind beispielsweise in üblicher Weise geschützte Hydroxygruppen, die als Schutzgruppe durch neutrale, saure oder basische Solvolyse, vor allem Hydrolyse, oder durch Reduktion abspaltbare Gruppen anstelle des Wasserstoffatoms aufweisen. Schutzgruppen dieser Art, ebenso selektive Verfahren zu ihrer Abspaltung, d.h. zur Überführung der geschützten Hydroxygruppe in Hydroxy, sind in der Literatur zu Genüge bekannt.

Neben anderen üblichen Hydroxy-Schutzgruppen kommen als in Hydroxy überführbare Gruppen $X_{13}$ beispielsweise verätherte Hydroxygruppen, wie Niederalkoxy, Triniederalkylsilyloxy oder gegebenenfalls substituierte Phenylniederalkoxygruppen, z. B. Methoxy, Trimethylsilyloxy oder Benzyloxy, insbesondere geschützte Hydroxygruppen der Formel

R''O-, worin R'' Niederalkyl, z. B. Methyl, α-Aralkyl, z. B. Benzyl, oder Triniederalkylsilyl, z. B. Trimethylsilyl, darstellt, veresterte Hydroxygruppen, wie mit anorganischen Säuren oder mit organischan Carbonsäuren, veresterte Hydroxygruppen, z. B. Niederalkanoyloxygruppen oder Gruppen der Formel Ph-C(O=)-O-, ferner Diazoniumgruppen in Batracht.

Die Überführung dieser und ähnlicher Gruppen $X_{13}$ erfolgt in üblicher Weise, beispielsweise durch Hydrolyse, erforderlichenfalls in Gegenwart eines, vorzugsweise sauren, Hydrolysemittels, eine Lösungs- oder

Verdünnungsmittels oder eines Lösungsvermittlers, unter Kühlen oder Erwärmen, z. B. im Temperaturbereich von etwa 0 bis 120°C, und/oder unter Inertgas. Hydrolysemittel sind neben üblichen basischen Hydrolysemitteln, wie Alkalimetallhydroxiden, als saure Hydrolysemittel beispielsweise Lewissäuren, wie komplexe Metallhalogenide der Formel $M^nYn$ (III), worin M ein n-wertiges, koordinativ ungesättigtes Metallkation der Gruppe IIa, IIb, IIIa, IIIb, IVb, Va oder VIIIb des periodischen Systems der Elemente, z. B. das Magnesium-, $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$- bzw. Eisen VIion, Y ein Halogenatom der Atomnummer bis und mit 35, wie Fluor oder Chlor bedeutet, z. B. Aluminiumtrichlorid, oder Protonensäuren, wie Mineralsäuren, z. B. Chlor-, Brom- oder Jodwasserstoffsäure, Schwefelsäure, Phosphorsäure oder Polyphosphorsäure, ebenso komplexe Metallsäuren, z. B. Hexachloroantimonsäure, Tetrafluorborsäure und dergleichen, im Falle von mit organischen Carbonsäuren veresterten Hydroxygruppen $X_{13}$ ferner Niederalkansäuren, wie Essigsäure. Lösungsmittel sind bei der Hydrolyse beispielsweise mit Wasser mischbare organische Lösungsmittel.

So kann man verätherte Hydroxygruppen beispielsweise durch Behandeln mit wässriger Jodwasserstoffsäure, mit Bromwasserstoffsäure in hochkonzentrierter, z. B. 98%-ger Essigsäure oder durch Behandeln mit Aluminiumtrichlorid hydrolysieren. In einer Abwandlung dieses Verfahrens kann man z. B. Verbindungen der Formeln

$Ph - X_1$ (IIa) und 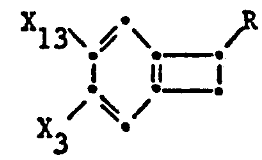 (XXXVIII),

worin $X_1$ eine Gruppe der Formel -C(=O)-Z und Z anhydridisiertes Hydroxy, wie Halogen oder eine Gruppe der Formel Ph-C(=O)-O, $X_{13}$ z. B. veräthertes Hydroxy, wie Niederalkoxy, und $X_3$ Wasserstoff bedeutet, mit einer Lewissäure, wie mit einem komplexen Metallhalogenid der Formel $M^nYn$ (III), z. B. mit Aluminiumtrichlorid, behandeln und aus der dabei möglicherweise primär gebildete Verbindung der Formel XXXVII worin $X_{13}$ z. B. veräthertes Hydroxy bedeutet, die Hydroxygruppe durch Zugabe von Wasser freisetzen.

Ebenso kann man eine Verbindung der Formel IIb, worin $X_2$ gegebenenfalls veräthertes Hydroxy und $X_3$ Wasserstoff bedeutet, in Gegenwart einer Protonensäure oder eines der genannten komplexen Metallhalogenide der Formel $M^nY_n$ (III) mit einem Überschuss einer Verbindung der Formel IIa, worin $X_1$ eine Gruppe der Formel -C(=O)-Z ist, behandeln und anschliessend aus einer möglicherweise gebildeten Verbindung der Formel XXXVII, worin $X_{13}$ mit der Säure der Formel Ph-COOH verestertes Hydroxy bedeutet, die Hydroxygruppe durch Hydrolyse freisetzen.

In als Gruppe $X_{13}$ eine gegebenenfalls substituierte α-Phenylniederalkoxygruppe oder eine andere übliche, durch Reduktion spaltbare geschützte Hydroxygruppe aufweisende Verbindungen der Formel XXXVII kann die Hydroxygruppe vorteilhaft reduktiv freigesetzt werden. So kann man beispielsweise hydrieren, d.h. mit Wasserstoff in Gegenwart eines Hydrierungskatalysators, z. B. eines Palladium-, Platin-, Nickel- oder Rhodiumkatalysators, z.B. von Palladium auf Kohle oder von Raney-Nickel, reduzieren.

Ferner kann man ausgehend von Verbindungen der Formel XXXVII, worin $X_{13}$ mit einer organischen Carbonsäure verestertes Hydroxy ist, die Hydroxygruppe durch Umesterung, d.h. Behandlung mit einem Alkohol, z. B. Niederalkanol, in Gegenwart eines sauren oder basischen Mittels, wie einer Mineralsäure, z. B. von Schwefelsäure oder eines Alkalimetallhydroxides oder -alkoholates, z. B. von Natriumhydroxid oder eines Natriumniederalkanolates, freisetzen.

Die Überführung von Diazonium $X_{13}$ in Hydroxy erfolgt beispielsweise unter in situ-Bildung der Diazoniumgruppe, beispielsweise indem man eine entsprechende Aminoverbindung, worin $X_{13}$ Amino ist, in üblicher Weise diazotiert und das gebildete Diazoniumsalz ohne Isolierung durch Behandlung mit Wasser, vorzugsweise bei erhöhter Temperatur, z. B. bei etwa 40 bis 120°C, durch Hydroxy ersetzt.

Die Ausgangsstoffe der Formel XXXVII können nach an sich bekannten Verfahren hergestellt werden, beispielsweise ausgehend von Verbindungen der Formeln IIe, IIf oder XXXIV, von Verbindungen der Formeln V, XI, XII, XIII, XIX, XX, XXI, XXIII, XXIV, XXV, XXVI, XXVII, XXVIII oder XXXII, die anstelle der phenolischen Hydroxygruppe eine Gruppe $X_{13}$ (ausser Diazonium unter Einschluss von Amino) aufweisen, von Verbindungen der Formeln II, IIb, IV, VII, VIII, X, XIV, XV, XVI, XVII, XVIII, XXII, XXXI, XXXV, XXXVI oder XXXIX, die anstelle der Gruppe R'0 eine Gruppe $X_{13}$ aufweisen, bzw. von Verbindungen der Formeln XXIX oder XXX, die anstelle der Gruppe R"0 eine andere Gruppe $X_{13}$ aufweisen, wobei man sich z. B. der angegebenen Verfahrensweise bedient.

Bei der praktischen Durchführung der vorstehend beschriebenen Bildungsweisen kann es vorteilhaft sein, mehrere gleichartige Reaktionen nach Art einer "Eintopfreaktion" zusammenzufassen, beispielsweise die oxidative Überführung eines Restes $X_4$ in eine Gruppe Ph-C(=O)- in Verbindung der Formel V mit der oxidativen Überführung eines Restes $X_{10}$ in eine Gruppe

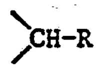

in Verbindungen der Formel XXI zu kombinieren indem man von einer entsprechenden Verbindung der Formel

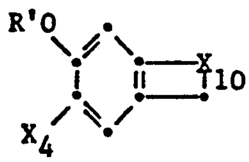

(XXXIX)

ausgeht. Ebenso kann man die solvolytische Überführung eines Restes $X_4$ in eine Gruppe Ph-C(=O)- in Verbindungen der Formel V mit der solvolytischen Überführung eines Restes $X_{10}$ in eine Gruppe 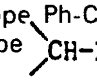

in Verbindungen der Formel XXI und/oder die solvolytische Überführung von $X_{13}$ in Hydroxy in Verbindungen der Formel XXXVII zusammenfassen, indem man von einer entsprechenden Verbindung der Formel XXXIX oder einer Verbindung der Formel

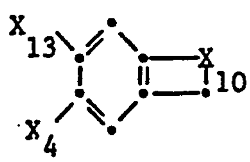

(XL) .

ausgaht. Ferner kann man z.B. die phenolische Hydroxygruppe in Verbindungen der Formeln V, XI, XII, XIII, XIX, XXI, XXIII, XXIV, XXV, XXVI, XXVII, XXVIII und XXXII durch Silylierung bzw. in Verbindungen der Formeln XI, XII, XIII, XIX, XXI, XXIII, XXIV, XXV, XXVI, XXVII, XXVIII, und XXXII durch Benzylieren, Acylieren, Alkylieren oder Silylieren intermediär schützen und die Schutzgruppe nach Durchführung der Umsetzung wieder abspalten.

Verfahrensgemäss erhältliche Verbindungen können ferner in andere Verbindungen der Formel I überführt werden.

So kann man beispielsweise eine freie Carboxylgruppe R in üblicher Weise, z. B. durch Behandeln mit einem Diazoniederalkan oder Triniederalkyloxonium-/Triniederalkylcarboxonium- oder Diniederalkylcarboniumsalz, wie Hexachloroantimonat oder Hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester, z. B. einem Niederalkancarbonsäureester, Triniederalkylphosphit, Diniederalkylsulfit oder dem Pyrocarbonat, oder einem Mineralsäure- oder Sulfonsäureester, z. B. dem Chlor- oder Bromwasserstoffsäure- oder Schwefelsäure-, Benzolsulfonsäure-, Toluolsulfonsäure- oder Methansulfonsäureester, das entsprechenden Alkohols oder einem davon abgeleiteten Olefin, zu einer veresterten Carboxylgruppe R verestern.

Die Umsetzung mit dem entsprechenden Alkohol selbst kann vorteilhaft in Gegenwart eines sauren Katalysators erfolgen, wie einer Protonensäure, z. B. von Chlor- oder Bromwasserstoff-, Schwefel-, Phosphor-, Bor-, Benzolsulfon- und/oder Toluolsulfonsäure, oder eine Lewissäure, z. B. von Bortrifluorid-Aetherat, in einem inerten Lösungsmittel, insbesondere einem Überschuss des eingesetzten Alkohols und erforderlichenfalls in Gegenwart eines wasserbindenden Mittels und/oder unter destillativer, z. B. azeotroper, Entfernung des Reaktionswassers und/oder bei erhöhter Temperatur.

Die Umsetzung mit einam reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z. B. in Benzol oder Toluol, oder einem Überschuss des eingesetzten Alkoholdarivates oder des entsprechenden Alkohols. Ausgehend von einem Mineralsäure- oder Sulfonsäureester setzt man die zu veresternde Säure vorteilhaft in Form einas Salzes, z. B. des Natrium- oder Kaliumsalzes, und arbeitet erforderlichenfalls in Gegenwart eines basichen Kondensationsmittels, wie einer anorganischen Base, z. B. von Natrium- oder Kalium- oder Calciumhydroxid oder -carbonat, oder einer tertiären organischen Stickstoffbase, z. B. von Triäthylamin oder Pyridin, und/oder in einem inerten Lösungsmittel, wie einer der vorstehenden tertiären Stickstoffbasen oder eines polaren Lösungsmittels, z. B. in Dimethylformamid und/oder bei erhöhter Temperatur.

Die Umsetzung mit einem Olefin kann beispielsweise in Gegenwart eines sauren Katalysators, z. B. einer Lewissäure, z. B. von Bortrifluorid, einer Sulfonsäure, z. B. von p-Toluolsulfonsäure, oder vor allem eines basischen Katalysators, z. B. von Natrium oder Kaliumhydroxid, vorteilhaft in einem inerten Lösungsmittel, wie

einem Aether, z. B. in Diäthyläther oder Tetrahydrofuran, erfolgen.

Eine freie Carboxylgruppe R kann ferner durch Umsetzung mit Ammoniak oder einem mindestens ein Wasserstoffatom aufweisenden Amin in üblicher Weise, unter Dehydratisierung des intermediär gebildeten Ammoniumsalzes, z. B. durch azeotrope Destillation mit Benzol oder Toluol oder trockenes Erhitzen, in eine amidierte Carboxylgruppe R überführt werden.

Die vorstehend beschriebenen Umwandlungen freier in veresterte oder amidierte Carboxylgruppen R können aber auch so durchgeführt werden, dass man eine Verbindung der Formel I, worin R Carboxyl ist, zunächst in üblicher Weise in ein reaktionsfähiges Derivat, beispielsweise mittels eines Halogenides des Phosphors oder Schwefels, z.B. mittels Phosphorichlorid oder -bromid, Phosphorpentachlorid oder Thionylchorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives Amid, z. B. das von Imidazol oder 3,5-Dimethylpyrazol abgeleitete Amid, überführt und das erhaltene reaktionsfähige Derivat dann in üblicher Weise, z. B. wie nachstehend für die Umesterung bzw. gegenseitige Umwandlung veresterter und amidierter Carboxylgruppen R beschrieben, mit einem entsprechenden Alkohol, Ammoniak oder dem entsprechenden mindestens ein Wasserstoffatom aufweisenden Amin zu der gewünschten Gruppe R umsetzt.

Eine veresterte Carboxylgruppe R kann in üblicher Weise, z. B. durch Hydrolyse in Gegenwart eines Katalysators, beispeilsweise eines basichen oder sauren Mittels, wie einer starken Base, z. B. von Natrium- oder Kaliumhydroxid, oder einer Mineralsäure, z. B. von Salzsäure, Schwefelsäure oder Phosphorsäure, zur freien Carboxylgruppe R oder z. B. xdurch Umsetzung mit Ammoniak oder dem entsprechenden, mindestens ein Wasserstoffatom aufweisenden Amin in eine amididerte Carboxylgruppe R überführt werden.

Eine veresterte Carboxylgruppe R kann ferner in üblicher Weise, z. B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z. B. von Natrium- oder Kaliumhydroxid, oder einer starken Säure, wie einer Mineralsäure, z.B. von Salzsäure, Schewfelsäure oder Phosphorsäure, oder einer organischen Sulfonsäure, z. B. von p-Toluolpulfonsäure, oder einer Lewissäure, z. B. von Brotrifluorid-Aetherat, zu einer anderen veresterten Carboxylgruppa R umgeestert werden.

Eine amidierte Carboxylgruppe R kann in üblicher Weise, z.B. durch Hydrolyse in Gegenwart eines Katalysators, beispielsweise einer starken Base, wie eines Alkalimetall- oder Erdalkalimetallhydroxides oder -carbonates, z. B. von Natrium- oder Kaliumhydroxid oder -carbonat, oder einer strakten Säure, wie einer Mineralsäure, z. B. von Salzsäure, Schwefelsäure oder Phosphorsäure in die freie Carboxylgruppe R umgewandelt werden.

Die neuen Verbindungen können, je nach der Wahl der Ausgangsstoffe und Arbeitsweisen, in Form eines der möglichen Isomeren oder als Gemisch derselben, z. B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastareomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung eines sauren Endstoffes mit einer mit der racemischen Säure Salze bildenden optisch aktiven Base und Trennung der auf diese Weise erhaltenen Salze, z. B. auf Grund ihrer verschiedenen Löslichkeiten, in die Diasteromeren, aus denen die Antipoden durch Einwirkung geeigneter Mittel freigesetzt werden können, zerlegen. Vorteilhaft isoliert man den wirksameren der bieden Antipoden.

Erhaltene freie Verbindungen der Formel I, können in an sich bekannter Weise in Salze überführt werden. Saure Verbindungen, z. B. solche, in denen $R_4$ Carboxy und/oder $R_2$ und/oder $R_3$ Hydroxy ist, werden z. B. mit der entsprechenden Base, andere z. B. durch Behandeln mit einer Säure, z. B. einer eingangs erwähnte Salze bildenden Säure üblicherweise in Gegenwart eines Lösungs- oder Verdünnungsmittels in Salze überführt werden.

Erhaltene Salze können in an sich bekannter Weise in die freien Verbindungen umgewandelt werden, z. B. durch Behandeln mit einem sauren Reagens, wie einer Mineralsäure, bzw. einer Base, z. B. Alkalilauge.

Die Verbindungen einschliesslich ihrer Salze können auch in Form ihrer Hydrate erhalten werden oder da zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenige Ausführungsformen des Verfahrens, nach denen man von einer auf irgendeiner Stufe des Verfahrans als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu derer Herstellung bilden ebenfalls einen Gegenstand der Erfindung.

Bei den erfindungsgemässen pharmazeutischen Präparaten, welche Verbindungen der Formel I oder

pharmazeutisch verwendbare Salze davon enthalten, handet es sich um solche zur enteralen wie oralen oder rektalen, und parentralen Verabreichung sowie zur topischen Anwendung am Wamblütter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem alter und dem individuellen Zustand, sowie von der Applikationsweise ab. Im Normalfall ist für einen etwa 75 kg schweren Warmblüter bei oraler Applikation eine ungefähre Tagesdosis von etwa 30-300 mg, vorteilhaft in mehreren gleichen Teildosen verteilt, zu veranschlagen.

Die neuen pharmazeutischen Präparete enthalten z. B. von etwa 10 % bis etwa 80 %, vorzugsweise von etwa 20 % bis etwa 60 % des Wirkstoffs. Erfindungsgemäss pharmazeutische Präparate zur enteralen bzw. parenteralen Verabreichung sind z. B. solche in Dosiseinheitsformen wie Dragées, Tabletten, Kapseln oder Suppositorien, ferner Ampullen. Diese werden in an sich bekannter Weise, z. B. mittels konventioneller Misch-, Granulier-, Dragier-, Lösungs- oder Lyophilisierungsverfahren hergestellt. So kann mah pharmazeitische Präparate zu oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwändig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, verner Bindemittel, wie Stärkekleister unter Verwendung z. B. von Mais-, Weizen-, Reis- oder Kartoffelstärkte, Gelatine, Tragakanth, Methylcellulose und/oder Polyvinylpyrrolidon, und/oder wenn erwünscht, Sprengmittel, wie die obgenannten Stärken, ferner Carboxymethylstärke, quervernetztes Polyvinylpyrroldon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzüge, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifiezierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher wie Glycerin oder Sorbitol. Die Steckkapslen können den Wirkstoff in Form einas Granulats, z. B. im Gemisch mit Füllstoffen, wie Lactose, Bindemitteln, wie Stärken, und/oder Gleitmitteln, wie Talk oder Magneziumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglycole oder höhere Alkanole. Ferner können auch Galatine-Rektalkapseln verwendat werden, die eine Kombination des Wirkstoffs mit einer Grundmasse enthalten; als Grundmassenstoffe kommen z. B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z. B. Sesamöl, oder synthetische Fettsäureester, z. B. Aathyloleat oder Triglyceride, verwendet, oder wässrige Injektionssuspensionen, welche viskositätserhöhende Stoffe, z. B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20 % des Wirkstoffs enthalten.

Creme sind Oel-in-Wasser-Emulsionen, die mehr als 50% Wasser aufweisen. Als ölige Grundlage verwendet man in erster Linie Fettalkohole, z. B. Lauryl-, Cetyl- oder Stearyalkohol, Fettsäuren, z. B. Palmitin- oder Stearinsäure, flüssige bis feste Wachse, z. B. Isopropylmyristat, Wollwachs oder Bienenwachs, und/oder Kohlenwasserstoffe, z. B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z. B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylenfettalkoholäther oder -fettsäureester, oder entsprechende ionische Emulgatoen, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z. B. Cetylalkohol oder Stearylalkohol, verwendet. Zusätze zur Wasserphase sind u.a. Mittel welche die Austrocknung der Creme vermindern, z. B. Polyalkohole wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 %

Wasser oder wässrige Phasen enthalten. Als Fettphase kommen in erster Linie Kohlenwasserstoffe, z. B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z. B. Cetylalkohol oder Wollwachsalkohole, bzw. Wollwachs enthalten. Emulgatoren sind entsprechende liphophile Substanzen, wie Sorbitanfettsäureester (Spans), z. B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/odter Poläthylenglykol, sowie Konservierungsmittel, Riechstoffe, etc.

Fettsalben sind wasserfrei und enthalten als Grundlage insbesondere Kohlenwasserstoff, z. B. Paraffin, Vaseline und/oder flüssige Paraffine, ferner natürliche oder partialsynthetische Fett, z. B. Kokosfettsäuretriglycerid, oder vorzugsweise gehörtete Oele, z. B. hydriertes Erdnuss- oder Rizinusöl, ferner Fettsäurepartialester des Glycerins, z. B. Glycerinmono- und -distearat, sowie z. B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z. B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z. B. Dichlordifluormethan und Dichortetrafluoräthan, als Treibmittel verwendet werden. Als Oelphase verwendet man u.a. Kohlenwasserstoffe, z. B. Paraffinöl, Fettalkohole, z. B. Cetylalkoho, Fettsäureester, z. B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel, etc.

Tinkturen und Lösungen weisen meistens eine wässerigäthanolische Grundlage auf, der u.a. Polyalkohole, z. B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, liphophile Substanzen als Eesatz für die der Haut mit dem Aethanol entzogenen Fettsubstanzen, und, falls notwendig, ander Hilfs- und Zusatzmittal beigegeben sind.

Die Herstellung der topisch verwendbaren pharmazeutischen Präparate erfolgt in an sich bekannter Weise, z. B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und der Salze von solchen Verbindungen mxit salzbildenden Eigenschaften, vorzugsweise zur Behandlung von Entzündungen, in erster Linie von entzündlichen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

**Beispiel 1**:

95.7 g fein gepulvertes Aluminiumchlorid werden in 180 ml absolutem Methylenchlorid suspendiert. Unter Rühren und Kühlen werden 35 g 5-Methoxybenzocyclobuten-1-carbonsäuremethylester, gelöst in wenig Methylenchlorid so zugetropft, dass die Temperatur unter 30° bleibt. Bei der gleichen Temperatur tropft man 50.5 g Benzoylchlorid zu und erwärmt anschliessend während 4 Stunden zum Rückfluss. Nach dem Abkühlen wird das Reaktionsgemisch auf 600 g Eis gegossen. Die organische Phase wird abgetrennt und die wässrige Phase 3 mal mit je 150 ml Methylenchlorid extrahiert. Die vereinigen organischen Phasen werden einmal mit 250 ml Wasser gewaschen, über Magnesiumsulfat getrocknet und eingedampft. Das überschüssige Benzoylchlorid wird am Hochvakuum abdestilliert und der Rückstand aus Aether/Petroläther kristallisiert. Man erhält den 4-Benzoyl-5-hydroxy-benzocyclobuten-1-carbonsäuremethylester in Form gelber Kristalle vom Smp. 75-76°.

**Beispiel 2**:

14 g 4-Benzoyl-5-hydroxy-benzocyclobuten-1-carbonsäure-methylester werden in 150 ml Ether gelöst und zu 62 ml 2N wässriger Natriumhydroxyd-Lösung und 25 ml Wasser gegeben. Diese Mischung wird bei Raumtemperatur während 1 Stunde kräftig gerührt. Die Etherphase wird abgetrennt und einmal mit 75 ml Wasser extrahiert. Die wässrige Phase wird einmal mit 75 ml Ether gawaschen. Die vereinigten wässrigen Phasen werden mit 2N wässriger Salzsäure sauer gestellt und 3 mal mit je 100 ml Methylenchlorid extrahiert.

Die vareinigten Methylenhlorid-Extrakte werden über Magnesiumsulfat getrocknet und eingedampft. Kristallisation des Rückstandes aus Ether/Petrolether liefert die 4-Benzoyl-5-hydroxy-benzocyclobuten-1-carbonsäure in gelber Kristalle vom Smp. 167-169°.

**Beispiel 3:**

26,6 g fein gepulvertes Aluminiumchlorid werden in 60 ml Methylenchlorid suspendiert. Unter Rühren und schwachem Kühlen werden bei Raumtemperatur 9,61 g 5-Methoxybenzocyclobuten-1-carbonsäuremethylester gelöst in wenig Methylenchlorid zugetropft. Nach 30 Minuten Rühren bei Raumtemperatur tropft man 15,85 g o-Fluorbenzoylchlorid zu und erwärmt das entstandene Reaktionsgemisch während 4 Stunden zum Rückfxluss. Nach dem Abkühlen wird das Reaktionsgemisch auf Eis gegossen und gut mit Methylenchlorid extrahiert. Die vereinigten organischen Phasen werden mit Wasser gewaschen, über Magnesiumsulfat getrocknet und eingedampft. Der gelbe ölige Rückstand (20 g) wird an 400 g Kieselgel mit Methylenchlorid chromatographiert. Die Fraktionen 7-16 (Fraktionen à 50 ml) werden vereinigt und eingeengt. Der Rückstand wird aus Ether/Petrolether kristallisiert. Man erhält den 4-(o-Fluorbenzoyl)-5-hydroxy-benzocyclobuten-1-carbonsäuremethylester in Form gelber Kristalle vom Smp. 59-61°.

**Beispiel 4:**

12,1 g 4-(m-Chlorbenzoyl)-5-hydroxy-benzocyclobuten-1-carbonsäuremethylester werden in 120 ml Aether gelöst, mit 100 ml 2 N wässriger Natriumhydroxyd-Lösung versetzt und während 15 Minuten bei Raumtemperatur gut gerührt. Die Aetherphase wird abgetrennt und mit Wasser gewaschen. Die stark gelb gefärbte wässrige Phase wird mit Aether gewaschen. Die vereinigtenwässrigen Phasen werden mit konzentriertar Salzsäure sauer gestellt und gut mit Methylenchlorid extrahiert. Die vereinigten Methylenchlorid-Extrakte werden über Magnesiumsulfat getrocknet und eingedampft. Kristallisation des Rückstandes aus Aether/Petroläther ergibt die 4-(m-Chlorbenzoyl)-5-hydroxy-benzocyclobuten-1-carbonsäure als gelbe Kristalle vom Smp. 138-139°.

**Beispiel 5:**

28,26 g Aluminiumtrichlorid werden in 70 ml Dichlormethan suspendiert und bei 20 bis 30° innerhalb von 15 Minuten tropfenweise mit 10,19 g 5-Methoxybenzocyclobuten-1-carbonsäuremethylester versetzt. Man lässt 30 Minuten rühren, tropft innerhalb von 20 Minuten 20,8 g m-Methylthiobenzoylchlorid hinzu, lässt 4 Stunden bei Raumtemperatur rühren, giesst auf ein Gemisch von 200 g Eis, 20 ml konzentrierter Salzsäure und 200 ml Wasser, lässt 30 Minuten rühren und schüttelt zweimal mit je 50 ml Dichlormethan und einmal mit 50 ml eines Gemisches von Trichlormethan und Methanol (3:1) aus. Die organischen Phasen werden vereinigt, zweimal mit Wasser gewaschen, über Magnesiumsulfat getrocknet mit Kieselgur behandelt und eingedampft. Chromatographie an Kieselgel mit Hexan/Essigester (4:1) als Laufmittel ergibt den 4-(m-Methylthiobenzoyl)-5-hydroxy-benzocyclobuten-1-carbonsäuremethylester als gelbes Oel.

**Beispiel 6:**

In analoger Weise wie in Beispiel 5 beschrieben erhält man ausgehend von 37,12 g Aluminiumtrichlorid, 13,42 g 5-Methoxybenzocyclobuten-1-carbonsäuremethylester und 26,05 g o-Methylthiobenzoylchlorid den 4-(o-Methylthiobenzoyl)-5-hydroxy-benzocyclobuten-1-carbon-säuremethylester als viskoses, gelbes Oel.

**Beispiel 7:**

In analoger Weise wie in Beispiel 5 beschrieben erhält man ausgehend von 26,6 g Aluminiumtrichlorid, 9,61 g 5-Methoxybenzo-cyclobuten-1-carbonsäuremethylester und 17,56 g p-Cyanobenzoylchlorid den 4-(p-Cyanobenzoyl)-5-hydroxy-benzocyclobuten-1-carbonsäuremethylester vom Smp. 113-114°.

**Beispiel 8:**

In analoger Weise wie in Beispiel 5 beschrieben erhält man ausgehend von 26,6 g Aluminiumtrichlorid, 9,61 g 5-Methoxybenzocyclobuten-1-carbonsäuremethylester und 20,95 g 2,4-Dichlorbenzoylchlorid den 4-(2,4-Dichlorbenzoyl)-5-hydroxy-benzocyclobuten-1-carbonsäuremethylester von Smp. 99-100°.

**Beispiel 9:**

In analoger Weise wie in Beispiel 5 beschrieben erhält man ausgehend von 26,6 g Aluminiumtrichlorid, 9,61 g 5-Methoxybenzocyclobuten-1-carbonsäuremethylester und 20,95 g 3,4-Dichlorbenzoylchlorid den 4-(3,4-Dichlorbenzoyl)-5-hydroxy-benzocyclobuten-1-carbonsäuremethylester vom Smp. 95-96°.

**Beispiel 10:**

5,71 g 4-(o-Methylthiobenzoyl)-5-hydroxy-benzocyclobuten-1-carbonsäuremethylester werden in 70 ml Aether gelöst, mit 45 ml 2n-Natronlauge versetzt und 1 Stunde bei Raumtemperatur gerührt. Die Wasserphase wird abgetrennt und die Aetherphase zweimal mit je 25 ml Wasser ausgeschüttelt. Die wässrigen Phasen werden vereinigt, mit 2n-Salzsäure angesäuert und dreimal mit je 50 ml Dichlormethan extrahiert. Die Auszüge werden vereinigt, mit Wasser gewaschen, über Magnesiumsulfat getrocknet und eingedampft. Nach Umkristallisieren des Rückstandes aus Dichlormethan/Hexan erhält man die 4-(o-Methylthiobenzoyl)-5-hydroxy-benzocyclobuten-1-carbonsäure vom Smp. 177-178°.

**Beispiel 11:**

In analoger Weise wie in Beispiel 10 beschrieben erhält man ausgehend von 4,9 g 4-(m-Methylthiobenzoyl)-5-hydroxy-benzocyclobuten-1-carbonsäuremethylester die 4-(m-Methylthiobenzoyl)-5-hydroxy-benzocyclobuten-1-carbonsäure vom Smp. 122-124°.

**Beispiel 12:**

3,0 g 4-(α-Hydroxybenzyl)-5-hydroxy-benzocyclobuten-1-carbonsäure werden in 30 ml Dichlormethan gelöst und innerhalb von 10 Minuten unter Rühren tropfenweise mit 30 g Pyridiniumdichromat in 100 ml Dichlormethan versetzt. Man lässt 7 Stunden bei Raumtemperatur nachrühren, dekantiert vom schwarzen Rückstand ab, filtriert über Kieselgel, dampft zur Trockne ein und kristallisiert aus Aether/Hexan um. Man erhält die 4-Benzoyl-5-hydroxy-benzocyclobuten-1-carbonsäure vom Smp. 167-169°.

Das Ausgangsmaterial kann z. B. folgendenmassen hergestellt werden:

3,84 g 5-Methoxybenzocyclobuten-1-carbonsäuremethylester werden in 20 ml Dichlormethan gelöst, auf 0° abgekühlt, bei 0 bis 5° tropfenweise zunächst innerhalb von 3 Minuten mit 3,16 g Titantetrachlorid und dann innerhalb von 20 Minuten mit 3,83 g Dichlordimethyläther ($Cl_2CHOCH_3$) versetzt. Man lässt 5 Minuten bei 0 bis 5° nachrühren, allmählich auf Raumtemperatur erwärmen und 15 Minuten bei 35° nachrühren. Dann lässt man auf Raumtemperatur abkühlen, giesst auf 25 g Eis, trennt die organische Phase ab, schüttelt die Wasserphase dreimal mit je 10 ml Dichlormethan nach, vereinigt die organischen Phasen, wäscht dreimal mit je 15 ml Eiswasser, trocknet über Magnesiumsulfat dampft zur Trockne ein und chromatographiert an Kieselgel mit Dichlormethan als Laufmittel. Man erhält den 4-Formyl-5-methoxybenzocyclobuten-1-carbonsäuremethylester als farbloses Oel.

1,6 g derselben werden in 25 ml Aether gelöst, mit 19 ml 2n-Natronlauge versetzt und 20 Minuten bei Raumtemperatur heftig gerührt. Man trennt die Aetherphase ab, säuert die wässrige Phase mit 2n-Salzsäure schwach an, schüttelt zweimal mit je 50 ml eines Gemisches Dichlormethan und Essigester (4:1) aus, wäscht mit wenig Eiswasser nach, trocknet über Magnesiumsulfat und dampft zur Trockne ein. Man erhält die 4-Formyl-5-methoxy-benzocyclobuten-1-carbonsäure als gelbliches Oel.

0,91 g derselben werden in 5 ml Tetrahydrofuran gelöst und zu einer siedenden, frischbereiteten Lösung von Phenylmagnesiumbromid (aus 0,39 g Magnesium und 2,51 g Brombenzol in 10 ml Tetrahydrofuran) zugetropft. Man erwärmt 90 Minuten unter Rühren zum Rückfluss, lässt auf Raumtemperatur abkühlen und versetzt mit 20 g Eis und dann bis zum Verschwinden der Trübung mit gesättigter Ammoniumchloridlösung. Die organische Phase wird abgetrennt und die wässrige Phase zweimal mit Essigester ausgeschüttelt. Die organischen Phasen werden vereinigt, nacheinander mit 40 %iger Natriumbisulfitlösung, gesättigter Natriumbicarbonatlösung und Wasser gewaschen, über Magnesiumsulfat getrocknet und eingedampft. Man erhält die 4-(α-Hydroxybenzyl)-

5-methoxy-benzocyclobuten-1-carbonsäure vom Smp. 143-146°, die in analoger Weise wie in Beispiel 20 in die 4-(α-Hydroxybenzyl)-5-hydroxy-benzocyclobuten-1-carbonsäure überführt werden kann.

**Beispiel 13:**

10,65 g 4-(2,6-Dichlorbenzoyl)-5-hydroxy-benzocyclobuten-1-carbonitril werden in einem Gemisch von 100 ml konzentrierter Salzsäure und 100 ml Essigsäure suspendiert und 4 Stunden bei 100° gerührt. Man lässt auf Raumtemperatur abkühlen, saugt ab, wäscht mit Wasser gründlich nach, lässt an der Luft trocknen und kristallisiert aus Trichlormethan/Hexan unter Zusatz von wenig Methanol um. Man erhält die 4-(2,6-Dichlorbenzoyl)-5-hydroxy-benzocyclobuten-1-carbonsäure vom Smp. 202-203°.

Das Ausgangsmaterial kann z. B. folgendermassen hergestellt werden:

Zu einer Suspension von 26,6 g Aluminiumtrichlorid in 60 ml Dichlormethan werden unter Rühren bei 20 bis 30° 7,96 g 5-Methoxybenzo-cyclobuten-1-carbonitril in 40 ml Dichlormethan zugetropft. Man lässt 30 Minuten bei 20 bis 30° rühren und tropft dann innerhalb von 15 Minuten 20,95 g 2,6-Dichlorbenzoylchlorid hinzu, lässt 4 Stunden bei Raumtemperatur rühren und giesst dann auf ein Gemisch von 500 g Eiswasser und 10 ml konzentrierter Salzsäure. Man lässt 30 Minuten nachrühren, fügt 200 ml Trichlormethan hinzu, schüttelt kräftig durch, trennt die organische Phase ab, wäscht mehrmals mit halbkonzentrierter Salzsäure, trocknet über Magnesiumsulfat, dampft zur Trockne ein und kristallisiert aus Dichlormethan/Hexan um. Man erhält das 4-(2,6-Dichlorbenzoyl)-5-hydroxy-benzocyclobuten-1-carbonitril vom Smp. 174-175°.

**Beispiel 14:**

2 g 4-(2,6-Dichlorbenzoyl)-5-hydroxy-benzocyclobuten-1-carbonitril werden mit 20 ml konzentrierter Salzsäure und 10 ml Cellusolve versetzt und 2 Tage bei Raumtemperatur gerührt. Man verrührt mit 100 ml Wasser, saugt ab und kristallisiert aus Aether/Hexan um. Man erhält das 4-(2,6-Dichlorbenzoyl)-4-hydroxy-benzocyclobuten-1-carboxamid vom Smp. 174°.

**Beispiel 15:**

2 g 4-(2,6-Dichlorbenzoyl)-5-hydroxy-benzocyclobuten-1-carboxamid werden in 20 ml Essigsäure und 20 ml konzentrierter Salzsäure 4 Stunden bei 95 - 100° gerührt. Man lässt abkühlen, saugt ab, wäscht mit Wasser nach und kristallisiert aus Trichlormethan/Hexan um. Man erhält die 4-(2,6-Dichlorbenzoyl)-5-hydroxy-benzocyclobuten-1-carbonsäure vom Smp. 202-203°.

**Beispiel 16:**

28 g 4-(2,6-Dichlorbenzoyl)-5-hydroxy-benzocyclobuten-1-carbonitril werden in 50 ml Aethanol gelöst auf 0° gekühlt und unter Rühren bei 0° tropfenweise mit 10 g 30 %igem Wasserstoffperoxid versetzt. Dann wird langsam 1,0 ml 30 %ige Natronlauge zugetropft und 6 Stunden bei Raumtemperatur nachgerührt. Man neutralisiert mit 6n-Salzsäure und schüttelt mit 250 ml Dichlormethan gründlich aus. Die organische Phase wird über Magnesiumsulfat getrocknet und zur Trockne eingedampft. Nach Umkristallisieren aus Aether/Hexan erhält man das 4-(2,6-Dichlorbenzoyl)-5-hydroxy-benzocyclobuten-1-carboxamid vom Smp. 174-175°.

**Beispiel 17:**

Zu 15 ml einer 2,5 %igen Lösung von Ammoniak in Dichlormethan werden unter Rühren innerhalb von 15 Minuten 4,86 g 4-(2,6-Dichlorbenzoyl)-5-hydroxy-benzocyclobuten-1-carbonsäurechlorid zugetropft. Man lässt 7 Stunden bei Raumtemperatur rühren, filtriert von geringen Mengen eines feinkristallinen Niederschlages ab, dampft zur Trockne ein, chromatographiert an Kieselgel mit Dichlormethan/Methanol (15:1) als Laufmittel und kristallisiert aus Aether/Hexan um. Man erhält das 4-(2,6-Dichlorbenzoyl)-5-hydroxy-benzocyclobuten-1-carboxamid vom Smp. 174°.

Das Ausgangsmaterial kann z. B. folgendermassen hergestellt werden:

3,37 g 4-(2,6-Dichlorbenzoyl)-5-hydroxy-benzocyclobuten-1-carbonsäure werden in 100 ml Toluol suspendiert, mit 0,1 ml Dimethylfonmamid und 1,64 g Thionylchlorid versetzt, auf 80° erwärmt, 2 Stunden bei 80 - 90° gerührt und unter vermindertem Druck zur Trockne eingedampft. Man erhält das 4-(2,6-Dichlorbenzoyl)-5-hydroxy-benzocyclobuten-1-carbonsäurechlorid, welches ohne weitere Behandlung verwendet werden kann.

**Beispiel 18:**

5,0 g 4-(o-Fluorbenzoyl)-5-methoxy-benzocyclobuten-1-carbonsäuremethylester werden in 100 ml Dichlormethan gelöst, mit 6,4 g Aluminiumtrichlorid versetzt und 4 Stunden unter Rühren zum Sieden erhitzt. Man giesst auf Eis, fügt 20 ml konzentrierte Salzsäxure hinzu und schüttelt zweimal mit je 100 ml Dichlormethan aus. Die organischen Phasen werden vereinigt, mit Wasser gewaschen, über Magnesiumsulfat getrocknet und zur Trockne eingedampft. Der Rückstand wird über Kieselgel chromatographiert und aus Aether/Hexan umkristallisiert. Man erhält den 4-(o-Fluorbenzoyl)-5-hydroxy-benzocyclobuten-1-carbonsäuremethylester vom Smp. 72-73°.

Das Ausgangsmaterial kann z. B. folgendermassen hergestellt werden:

Zu einer auf 0° gekühlten Suspension von 26,6 g Aluminiumtrichlorid in 60 ml Dichlormethan werden unter Rühren bei 0 bis 3° 9,61 g 5-Methoxybenzocyclobuten-1-carbonsäuremethylester zugetropft. Man lässt 30 Minuten bei 0° rühren, tropft 15,9 g o-Fluorbenzoylchlorid hinzu und lässt 82 Stunden bei 0 bis 3° rühren. Dann giesst man in ein Gemisch von 100 g Eiswasser und 5 ml konzentrierter Salzsäure, schüttelt zweimal mit je 100 ml Dichlormethan aus, wäscht mit Wasser, trocknet über Magnesiumsulfat und dampft zur Trockne ein. Der Rückstand wird in wenig Dichlormethan aufgenommen, bis zur beginnenden Niederschlagsbildung mit Petroläther versetzt und kaltgestellt. Man saugt vom gebildeten Niederschlag ab, dampft ein und chromatographiert an Kieselgel mit Dichlormethan als Laufmittel. Nach einem geringen Vorlauf erhält man den 4-(o-Fluorbenzoyl)-5-methoxy-benzocyclobuten-1-carbonsäuremethylester vom Smp. 73-74°.

**Beispiel 19:**

1,0 g 4-Benzoyl-5-hydroxy-benzocyclobuten-1-methanol werden in 50 ml Essigsäure gelöst und portionsweise mit 4,0 g Chromtrioxid versetzt. Man lässt 16 Stunden bei 40° rühren, dampft unter vermindertem Druck zur Trockne ein, nimmt in 20 ml Wasser auf und schüttelt mit 80 ml Dichlozmethan aus. Die organische Phase wird über Magnesiumsulfat getrocknet und eingedampft. Nach Umkristallisieren des Rückstandes aus Aether/Hexan erhält man die 4-Benzoyl-5-hydroxy-benzocyclobuten-1-carbonsäure vom Smp. 167-169°.

Das Ausgangsmaterial kann z. B. folgendermassen hergestellt werden:

Zu einer Suspension von 0,21 g Lithiumaluminiumhydrid in 30 ml Aether wird unter Rühren innerhalb von 30 Minuten eine Lösung von 1,92 g 5-Methoxybenzocyclobuten-1-carbonsäuremethylester in 30 ml Aether zugetropft. Man lässt 1 Stunde rühren, gibt nochmals 1,0 g Lithiumaluminiumhydrid hinzu und lässt weitere 2 Stunden rühren. Dann tropft man 5 ml Wasser und nach einiger Zeit 12,5 ml 2n-Schwefelsäure hinzu, lässt 30 Minuten rühren, trennt die Aetherphase ab, schüttelt zweimal mit Aether nach, trocknet über Magnesiumsulfat und dampft zur Trockne ein. Man erhält das 5-Methoxybenzocyclobuten-1-methanol in Form eines farblosen Oeles.

1,64 g derselben werden in 30 ml Dichlormethan gelöst und mit 1,21 g Triäthylamin versetzt. Dann werden bei 10 bis 20° innerhalb von 5 Minuten 0,94 g Acetylchlorid in 10 ml Dichlormethan zugetropft. Man lässt 5 Stunden bei Raumtemperatur nachrühren, versetzt mit Wasser und trennt die organische Phase ab. Diese wird mit Wasser gewaschen, über Magnesiumsulfat getrocknet und eingedampft. Man erhält das 1-Acetoxymethyl-5-methoxy-benzocyclobuten als farbloses Oel.

1,78 g derselben werden in 20 ml Dichlormethan gelöst und unter Rühren in eine Suspension von 4,6 g Aluminiumtrichlorid in 40 ml Dichlormethan eingetropft. Man lässt 30 Minuten rühren und tropft dann bei 20 bis 30° 2,4 g Benzoylchlorid, gelöst in 10 ml Dichlormethan, hinzu. Man lässt 4 Stunden bei Raumtemperatur nachrühren, giesst auf Eis, gibt 5 ml konzentrierte Salzsäure hinzu, lässt 30 Minuten rühren, trennt die organische Phase ab, wäscht dreimal mit Wasser, trocknet über Magnesiumsulfat und dampft zur Trockne ein. Man erhält das 1-Acetoxymethyl-4-benzoyl-5-hydroxy-benzocyclobuten als gelbliches Oel.

2,2 g derselben werden in 80 ml Methanol gelöst, mit einer Lösung von 1,5 g Kaliumcarbonat in 50 ml Wasser versetzt und 6 Stunden bei Raumtemperatur gerührt. Man dampft unter venmindertem Druck zur Trockne ein, nimmt in 50 ml Wasser auf, schüttelt zweimal mit je 100 ml Dichlormethan aus, trocknet über Magnesiumsulfat, dampft zur Trockne ein und reinigt durch Chromatographie an einer Kieselgelsäule mit Dichlormethan/Essigester (9:1) als Laufmittel. Man erhält das 4-Benzoyl-5-hydroxy-benzocyclobuten-1-methanol als gelbes, v8iskoses Oel.

**Beispiel 20:**

5 g 5-Benzoyloxybenzocyclobuten-1-carbonsäuremethylester werden in 50 ml 1,2-Dichloräthan gelöst, mit 11,8 g Aluminiumtrichlorid versetzt und 35 Stunden zum Sieden erhitzt. Das Reaktionsgemisch wird auf Eis

gegossen und mit 20 ml konzentrierter Salzsäure versetzt. Die organische Phase wird abgetrennt und die wässrige Phase zweimal mit Dichlormethan ausgeschüttelt. Die organischen Phasen werden vereinigt, über Magnesiumsulfat getrocknet und eingedampft. Der Eindampfrückstand wird aus Aether/Hexan umkristallisiert. Man erhält den 4-Benzoyl-5-hydroxy-benzocyclobuten-1-carbonsäuremethylester, gelbliche Kristalle vom Smp. 75-76°.

Das Ausgangsmaterial kann z. B. folgendermassen hergestellt werden:

19,5 g 5-Methoxybenzocyclobuten-1-carbonsäuremethylester werden mit 1000 ml einer 1-molaren Lösung von Bortribromid in Dichlormethan übergossen und 6 Stunden zum Rückfluss erhitzt. Man dampft zur Trockne ein, schüttelt mit einem Gemisch aus 2000 ml Dichlormethan und 1000 ml Wasser aus, trennt die organische Phase ab, wäscht mit Wasser, trocknet über Magnesiumsulfat und dampft zur Trockne ein. Man erhält den 5-Hydroxybenzocyclobuten-1-carbonsäuremethylester in Fozm weisser Kristalle vom Smp. 108-109° (aus Aether/Hexan).

27 g 5-Hydroxybenzocyclobuten-1-carbonsäuremethylester werden in 2000 ml Dichlormethan gelöst und mit 18 g Triäthylamin versetzt. Dann gibt man unter Rühren bei 10 - 20° innerhalb von 5 Minuten portionsweise 25 g Benzoylchlorid hinzu, lässt 5 Stunden bei Raumtemperatur rühren, fügt 200 ml Wasser hinzu, trennt die organische Phase ab, trocknet über Magnesiumsulfat und dampft zur Trockne ein. Man erhält den 5-Benzoyloxybenzocyclobuten-1-carbonsäure-methylester als farbloses, viskoses Oel.

**Beispiel 21:**

In analoger Weise wie in den Beispielen 1, 3, 5, 12, 18 und 20 beschrieben kann man ferner herstellen:

4-(o-Chlorbenzoyl)-5-hydroxy-benzocyclobuten-1-carbonsäuremethylester als gelbes Oel,
4-(m-Chlorbenzol)-5-hydroxy-benzocyclobuten-1-carbonsäuremethylester als gelbes Oel,
4-(m-Methoxycarbonylbenzoyl)-5-hydroxy-benzocyclobuten-1-carbonsäuremethylester, Smp. 109-110°,
4-(o-Fluorbenzoyl)-5-hydroxy-benzocyclobuten-1-carboxamid,
4-(m-Methylbenzoyl)-5-hydroxy-benzocyclobuten-1-carbonsäuremethylester als gelbes Oel,
4-(p-Chlorbenzoyl)-5-hydroxy-benzocyclobuten-1-carbonsäuremethylester als gelbe Kristalle vom Smp. 82-84°,
4-(p-Methylbenzoyl)-5-hydroxy-benzocyclobuten-1-carbonsäuremethylester als gelbe Kristalle vom Smp. 100-102°,
4-(2,6-Dichlorbenzoyl)-5-hydroxy-benzocyclobuten-1-carbonsäuremethylester,
4-(o-Brombenzoyl)-5-hydroxy-benzocyclobuten-1-carbonsäuremethylester, Oel,
4-(o-Acetoxybenzoyl)-5-hydroxy-benzocyclobuten-1-carbonsäuremethylester,
4-(o-Trifluormethylbenzoyl)-5-hydroxy-benzocyclobuten-1-carbonsäuremethylester,
4-(m-Trifluormethylbenzoyl)-5-hydroxy-benzocyclobuten-1-carbonsäuremethylester,
4-(p-Methoxybenzoyl)-5-hydroxy-benzocyclobuten-1-carbonsäuremethylester,
4-(o-Methoxybenzoyl)-5-hydroxy-benzocyclobuten-1-carbonsäuremethylester,
4-(2,6-Dimethylbenzoyl)-5-hydroxy-benzocyclobuten-1-carbonsäuremethylester,
4-(2,6-Dimethoxybenzoyl)-5-hydroxy-benzocyclobuten-1-carbonsäuremethylester,
4-(o-Methylbenzoyl)-5-hydroxy-benzocyclobuten-1-carbonsäuremethylester.

**Beispiel 22:**

In analoger Weise wie in den Beispielen 2, 4, 10, 12, 13, 15 und 19 beschrieben kann man ferner herstellen:

4-(o-Chlorbenzoyl)-5-hydroxy-benzocyclobuten-1-carbonsäure in Form blassgelber Kristalle vom Smp. 151-152°,
4-(m-Methylbenzoyl)-5-hydroxy-benzocyclobuten-1-carbonsäure in Form gelber Kristalle vom Smp. 142-144°,
4-(p-Chlorbenzoyl)-5-hydroxy-benzocyclobuten-1-carbonsäure in Form gelber Kristalle vom Smp. 181-183°,
4-(p-Methylbenzoyl)-5-hydroxy-benzocyclobuten-1-carbonsäure in Form gelber Kristalle vom Smp. 199-201°,
4-(o-Methylbenzoyl)-5-hydroxy-benzocyclobuten-1-carbonsäure vom Smp. 120,5-121°,
4-(o-Fluorbenzoyl)-5-hydroxy-benzocyclobuten-1-carbonsäure vom Smp. 138-139°,
4-(2,4-Dichlorbenzoyl)-5-hydroxy-benzocyclobuten-1-carbonsäure, Smp. 180-181°,
4-(3,4-Dichlorbenzoyl)-5-hydroxy-benzocyclobuten-1-carbonsäure, Smp. 189-190°,
4(o-Brombenzoyl)-5-hydroxy-benzocyclobuten-1-carbonsäure, Smp 157-158°,
4-(o-Acetoxybenzoyl)-5-hydroxy-benzocyclobuten-1-carbonsäure,
4-(o-Trifluormethylbenzoyl)-5-hydroxy-benzocyclobuten-1-carbonsäure,
4-(m-Trifluormethylbenzoyl)-5-hydroxy-benzocyclobuten-1-carbonsäure,
4-(o-Methoxybenzoyl)-5-hydroxy-benzocyclobuten-1-carbonsäure,
4-(p-Methoxybenzoyl)-5-hydroxy-benzocyclobuten-1-carbonsäure,
4-(p-Cyanobenzoyl)-5-hydroxy-benzocyclobuten-1-carbonsäure, Smp. 164-165°,
4-(2,6-Dimethylbenzoyl)-5-hydroxy-benzocyclobuten-1-carbonsäure und

4-(2,6-Dimethoxybenzoyl)-5-hydroxy-benzocyclobuten-1-carbonsäure.

**Beispiel 23:**

Tabletten, enthaltend 25 mg Wirkstoff, z. B. 4-Benzoyl-5-hydroxy-benzocyclobuten-1-carbonsäure oder ein Salz, z. B. das Natriumsalz, davon, können folgendermassen hergestellt werden:

**Bestandteile** (für 1000 Tabletten):

| | |
|---|---|
| Wirkstoff | 25,0 g |
| Lactose | 100,7 g |
| Weizstärke | 7,5 g |
| Polyäthylenglykol 6000 | 5,0 g |
| Talkum | 5,0 g |
| Magnesiumstearat | 1,8 g |
| entmineralisiertes Wasser | q.s. |

**Herstellung:**

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung das Polyäthylenglykols in 100 ml Wasser hinzugegeben und das Gemisch, wenn nötig unter Hinzufügen von Wasser, granuliert. Das Grunulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 22 genannten Verbindungen der Formel 1 hergestellt werden, wobei Verbindungen, worin R Carboxy ist, auch in Form von Salzen mit Basen, z. B. als Natriumsalz, vorliegen können.

**Beispiel 24:**

Kautabletten, enthaltend 30 mg Wirkstoff, z. B. 4-Benzoyl-5-hydroxy-benzocyclobuten-1-carbonsäure oder ein Salz, z. B. das Natriumsalz davon, können z. B. folgendermassen hergestellt werden:

**Zusammensetzung** (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5 %-ige Gelatinelösung | q.s. |

**Herstellung:**

Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

In analoger Weise können auch Tabletten enthaltend jeweils 30 mg einer anderen der in den Beispielen 1 bis 22 genannten Verbindungen der Formel 1 hergestellt werden, wobei Verbindungen, worin R Carboxy ist, auch in Form von Salzen mit Basen, z. B. als Natriumsalz, vorliegen können.

**Beispiel 25**:

Tabletten enthaltend 100 mg Wirkstoff, z. B. 4-Benzoyl-5-hydroxy-benzocyclobuten-1-carbonsäure oder ein Salz, z. B. das Natriumsalz davon können folgendermassen hergestellt werden:.

**Zusammensetzung** (für 1000 Tabletten):

| | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

**Herstellung**:

Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhalten Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltand 100 mg einer anderen Verbindung der Formel I gemäss den Beispielen 1 bis 22 hergestellt werden, wobei Verbindungen, worin R Carboxy ist, auch in Form von Salzen mit Basen, z. B. als Natriumsalz, vorliegen können.

**Patentansprüche**

für die Vertragsstaaten: BE, CH, LI, DE, FR, IT, LU, NL, SE, GB.

1. Ein neues substituiertes Benzophenon der Formel

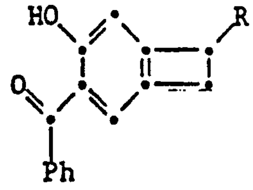

(I),

worin Ph unsubstituiertes oder substituiertes Phenyl bedeutet und R freies, verestertes oder amidiertes Carboxy bedeutet, oder ein Salz davon.

2. Eine Verbindung gemäss Patentanspruch 1, worin Ph unsubstituiertes oder durch Niederalkyl, Niederalkoxy, Niederalkylthio, Niederalkanoyloxy, Halogen, Trifluormethyl, Carboxy, Niederalkoxycarbonyl, Cyano und/oder Nitro substituiertes Phenyl bedeutet und R Carboxy mit einem niederen aliphatischen Alkohol verestertes Carboxy oder als Aminogruppe unsubstituiertes oder durch niedere aliphatische Reste mono- oder disubstituiertes Amino aufweisendes Carbamyl darstellt, oder ein Salz davon mit einer Base.

3. Eine Verbindung gemäss Patentanspruch 1, worin Ph unsubstituiertes oder durch Niederalkyl, Niederalkoxy, Niederalkanoyloxy, Halogen, Trifluormethyl, Cyano und/oder Nitro mono- oder disubstituiertes Phenyl bedeutet und R Carboxy, mit einem niederen aliphatischen Alkohol verestertes Carboxy oder als

Aminogruppe unsubstituiertes oder durch niedere aliphatische Reste mono- oder disubstituiertes Amino aufweisendes Carbamyl darstellt, oder ein Salz davon mit einer Base.

4. Eine Verbindung gemäss Patentanspruch 1, worin Ph unsubstituiertes oder durch Niederalkyl mit bis und mit 4 C-Atomen, Niederalkoxy mit bis und mit 4 C-Atomen, Niederalkanoyloxy mit bis und mit 4 C-Atomen, Halogen der Atomnummer bis und mit 35 und/oder Trifluormethyl mono- oder disubstituiertes Phenyl bedeutet und R Carboxy, Niederalkoxycarbonyl mit bis und mit 5 C-Atomen oder Carbamyl bzw. N-Niederalkylcarbamyl mit bis und mit 5 C-Atomen bedeutet, oder ein Salz davon mit einer Base.

5. Eine Verbindung gemäss Patentanspruch 1, worin Ph unsubstituiertes oder durch Niederalkyl mit bis und mit 4 C-Atomen, Niederalkylthio mit bis und mit 4 C-Atomen, Niederalcoxycarbonyl mit bis und mit 4 C-Atomen im Niederalkoxyteil, Halogen der Atomnummer bis und mit 35, und/oder Cyano, mono- oder disubstituiertes Phenyl bedeütet und R Carboxy oder Carbamyl, ferner Niederalkoxycarbonyl mit bis und mit 4 C-Atomen im Niederalkoxyteil bedeutet, oder ein Salze davon mit einer Base.

6. Eine Verbindung gemäss Patentanspruch 1, worin Ph durch Niederalkyl mit bis und mit 4 C-Atomen oder Halogen der Atomnummer bis und mit 35, monosubstituiertes oder disubstituiertes Phenyl bedeutet und R Carboxy oder Carbamyl, ferner Niederalkoxycarbonyl mit bis und mit 4 C-Atomen im Niederalkoxyteil bedeutet, oder ein Salz davon mit einer Base.

7. Eine Verbindung gemäss Patentanspruch 1, worin Ph durch Halogen der Atomnummer bis und mit 35 in 2-Stellung monosubstituiertes Phenyl bedeutet und R Carboxy oder Carbamyl darstellt, oder ein Salz davon mit einer Base.

8. 4-(Benzoyl)-5-hydroxy-benzocyclobuten-1-carbonsäuremethylester, 4-(o-Chlorbenzoyl)-5-hydroxy-benzocyclobuten-1-carbonsäuresethylester, 4-(m-Chlorbenzoyl)-5-hydroxy-benzocyclobuten-1-carbonsäuremethylester, 4-(m-Methylbenzoyl)-5-hydroxy-benzocyclobuten-1-carbonsäuremethylester, 4-(p-Chlorbenzoyl)-5-hydroxy-benzocyclobuten-1-carbonsäuremethylester, 4-(p-Methylbenzoyl)-5-hydroxy-benzocyclobuten-1-carbonsäuremethylester, 4-(2,6-Dichlorbenzoyl)-5-hydroxy-benzocyclobuten-1-carbonsäuremethylester, 4-(2,4-Dichlorbenzoyl)-5-hydroxy-benzocyclobuten-1-carbonsäuremethylester, 4-(3,4-Dichlorbenzoyl)-5-hydroxy-benzoylcyclobuten-1-carbonsäuremethylester, 4-(o-Brombenzoyl)-5-hydroxy-benzocyclobuten-1-carbonsäuremethylester, 4-(o-Acetoxybenzoyl)-5-hydroxy-benzocyclobuten-1-carbonsäuremethylester, 4-(o-Methylthiobenzoyl)-5-hydroxy-benzocyclobuten-1-carbonsäuremethylester, 4-(o-Trifluormethyl-benzoyl)-5-hydroxy-benzocyclobuten-1-carbonsäuremethylester, 44-(m-Trifluormethylbenzoyl)-5-hydroxy-benzocyclobuten-1-carbonsäuremethylester, 4-(p-Methoxybenzoyl)-5-hydroxy-benzocyclobuten-1-carbonsäuremethylester, 4-(o-Methoxybenzoyl)-5-hydroxy-benzocyclobuten-1-carbonsäuremethylester, 4-x(p-Cyanobenzoyl)-5-hydroxy-benzocyclobuten-1-carbonsäuremethylester, 4-(2,6-Dimethylbenzoyl)-5-hydroxy-benzoylcyclobuten-1-carbonsäuremethylester, 4-(2,6-Dimethoxybenzoyl)-5-hydroxy-benzocyclobuten-1-carbonsäuremethylester, 4-(o-Methylbenzoyl)-5-hydroxy-benzocyclobuten-1-carbonsäuremethylester oder 4-(o-Fluorbenzoyl)-5-hydroxy-benzocyclobuten-1-carbonsäuremethylester gemäss Anspruch 1.

9. 4-(Benzoyl)-5-hydroxy-benzocyclobuten-1-carbonsäure, 4-(m-Chlorbenzoyl)-5-hydroxy-benzocyclobuten-1-carbonsäure, 4-(m-Methylbenzoyl)-5-hydroxy-benzocyclobuten-1-carbonsäure, 4-(p-Chlorbenzoyl)-5-hydroxy-benzocyclobuten-1-carbonsäure, 4-(2,6-Dichlorbenzoyl)-5-hydroxy-benzocyclobuten-1-carbonsäure, 4-(3,4-Dichlorbenzoyl)-5-hydroxy-benzocyclobuten-1-carbonsäure, 4-(o-Acetoxybenzoyl)-5-hydroxy-benzocyclobuten-1-carbonsäure, 4-(m-Methylthiobenzoyl)-5-hydroxy-benzocyclobuten-1-carbonsäuremethylester, 4-(o-Methylthiobenzoyl)-5-hydroxy-benzocyclobuten-1-carbonsäure, 4-(o-Trifluormethylbenzoyl)-5-hydroxy-benzocyclobuten-1-carbonsäure, 4-(m-Trifluormethylbenzoyl)-5-hydroxy-benzocyclobuten-1-carbonsäure, 4-(o-Methoxybenzoyl)-5-hydroxy-benzocyclobuten-1-carbonsäure, 4-(p-Methoxybenzoyl)-5-hydroxy-benzocyclobuten-1-carbonsäure, 4-(p-Cyanobenzoyl)-5-hydroxy-benzocyclobuten-1-carbonsäure, 4-(2,6-Dimethylbenzoyl)-5-hydroxy-benzocyclobuten-1-carbonsäure, 4-(m-Methylthiobenzoyl)-5-hydroxy-benzocyclobuten-1-carbonsäure, 4-(2,6-Dimethoxybenzoyl)-5-hydroxy-benzocyclobuten-1-carbonsäure, 4-(p-Methylbenzoyl)-5-hydroxy-benzocyclobuten-1-carbonsäure, 4-(o-Chlorbenzoyl)-5-hydroxy-benzocyclobuten-1-carbonsäure, 4-(o-Methylbenzoyl)-5-hydroxy-benzocyclobuten-1-carbonsäure oder 4-(2,4-Dichlorbenzoyl)-5-hydroxy-benzocyclobuten-1-carbonsäure oder jeweils ein Salz davon mit einer Base gemäss Anspruch 1.

10. 4-(o-Fluorbenzoyl)-5-hydroxy-benzocyclobuten-1-carbonsäure oder ein Salz davon mit einer Base gemäss Anspruch 1.

11. 4-(o-Brombenzoyl)-5-hydroxy-benzocyclobuten-1-carbonsäure oder ein Salz davon mit einer Base gemäss Anspruch 1.

12. 4-(o-Fluorbenzoyl)-5-hydroxy-benzocyclobuten-1-carboxamid, 4-(m-Methoxycarbonylbenzoyl)-5-hydroxy-benzocyclobuten-1-carbonsäure-methylester oder 4-(2,6-Dichlorbenoyl)-5-hydroxy-benzocyclobuten-1-carboxamid gemäss Anspruch 1.

13. Eine Verbindung gemäss einem der Patentansprüche 1, 3, 4 und 8 bis 11 zur Anwendung in einem Varfahren zur therapeutischen Behandlung des menschlichen oder tierischen Körpers.

14. Eine Verbindung gemäss einem der Patentansprüche 2, 5, 6, 7 und 12 zur Anwendung in einem Verfahren zur therapeutischen Behandlung des menschlichen oder tierischen Körpers.

15. Eine Verbindung gemäss einem der Patentansprüche 1, 3, 4 und 8 bis 11 zur Anwendung gemäss Patentanspruch 13 als Antiinflammatorikum und/oder Antinociceptivum.

16. Eine Verbindung gemäss einem der Patentansprüche 2, 5, 6, 7 und 12 zur Anwendung gemäss Patentanspruch 14 als Antiinflammatorikum und/oder Antinociceptivum.

17. Verfahren zur Herstellung neuer substituierter Benzophenone der Formel I gemäss Anspruch 1, und ihrer

Salze, dadurch gekennzeichnet, dass man eine Verbindung bzw. ein Verbindungsgemisch der Formel

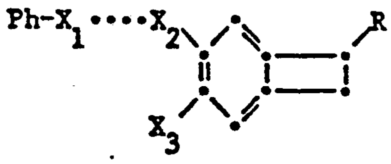

(II),

worin $X_1$ und $X_2$ gemeinsam eine über die Carbonylgruppe mit dem Rest Ph verbundene Gruppe der Formel -C(=O)-O bedeutet und $X_3$ Wasserstoff ist oder einer der Reste $X_1$ und $X_3$ eine Gruppe der Formel -C(=O)-Z, der andere Wasserstoff und $X_2$ eine Gruppe R'O- bedeutet, in der R' Niederalkyl oder eine Hydroxyschutzgruppe R'' darstellt oder, sofern $X_3$ Wasserstoff ist, ebenfalls Wasserstoff bedeuten kann, der Säurebehandlung unterwirft und das Primärprodukt solvolytisch zersetzt oder in einer Verbindung der Formel

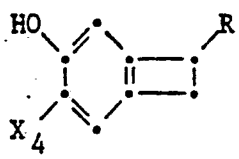

(V)

worin $X_4$ einen oxidativ oder solvolytisch in eine Gruppe der Formel -C(=O)-Ph überführbaren Rest bedeutet, $X_4$ zu der gewünschten Gruppe der Formel -C(=O)-Ph oxidiert oder solvolysiert oder Verbindung der Formel

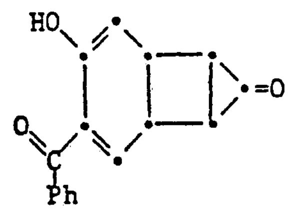

(XI)

oder ein Phenolatsalz davon mit Wasser, einem Alkohol oder mit Ammoniak oder einem mindestens ein Wasserstoffatom aufweisendes Amin behandelt und aus einem verfahrensgemäss erhältlichen Isomerengemisch erforderlichenfalls das Isomere der Formel 1 isoliert oder eine Verbindung der Formel

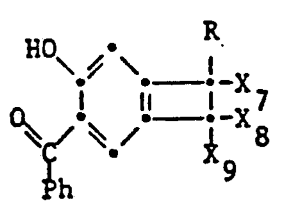

(XIII),

worin mindestens einer der Reste $X_7$ und $X_8$ gegebenenfalls veräthertes oder verestertes Hydroxy und ein davon verschiedener Rest $X_7$ oder $X_8$ sowie $X_9$ für Wasserstoff steht, oder $X_7$ Wasserstoff bedeutet und $X_8$ und $X_9$ gegebenenfalls funktionell abgewandeltes Oxo darstellen, oder ein Phenolat- und/oder Carboxylatsalz davon reduziert oder in einem von einem Ester oder Amid der Formel 1 verschiedenen funktionellen Carboxyderivat derselben oder einem Salz davon die funktionall abgewandelte Carboxygruppe solvolytisch in eine gegebenenfalls veresterte oder amidierte Carboxygruppe überführt oder in einer Verbindung der Formel

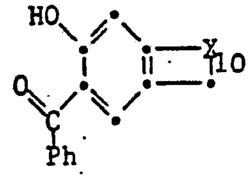

(XXI)

worin $X_{10}$ eine Gruppe der Formel 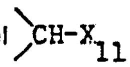

und $X_{11}$ einen oxidativ in eine Gruppe R überführbaren Rest bedeutet, den Rest $X_{11}$ zu einer Gruppe R oxidiert oder in einer Verbindung der Formel

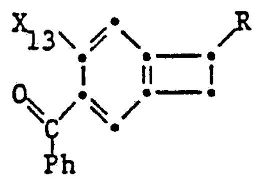

(XXXVII)

worin $X_{13}$ eine in Hydroxy überführbare Gruppe darstellt, oder in einem Salz davon $X_{13}$ in Hydroxy überführt, wobei man die oxidative Überführung eines Restes $X_4$ in eine Gruppe Ph-C(=O)- in Verbindung der Formel V mit der oxidativen Überführung eines Restes $X_{10}$ in eine Gruppe 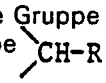

in Verbindungen der Formel XXI kombinieren kann, indem man von einer entsprechenden Verbindung der Formel

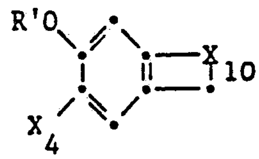

(XXXIX)

ausgeht, bzw. die solvolytische Überführung eines Restes $X_4$ in eine Gruppe Ph-C(=O)- in Verbindungen der Formel V mit der solvolytischen Überführung eines Restes $X_{10}$ in eine Gruppe 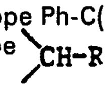

in Verbindung der Formel XXI und/oder die solvolytische Überführung von $X_{13}$ in Hydroxy in Verbindungen der Formel XXXVII zusammenfassen kann, indem man von einer entsprechenden Verbindung der Formel XXXIX oder einer Verbindung der Formel

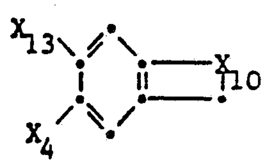

(XL)

ausgeht, gegebenenfalls die Hydroxyschutzgruppe nach Durchführung der Umsetzung abspaltet, und gewünschtenfalls die verfahrensgemäss erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

18. Ein pharmazeutisches Präparat enthaltend eine Verbindung gemäss einem der Patentansprüche 1, 3, 4 und 8 bis 11 neben üblichen pharmazeutischen Hilfs- und/oder Trägerstoffen.

19. Ein pharmazeutisches Präparat enthaltend eine Verbindung gemäss einem der Patentansprüche 2, 5, 6, 7 und 12 neben üblichen pharmazeutischen Hilfs- und/oder Trägerstoffen.

**Patentansprüche**

für den Vertragsstaat AT

1. Verfahren zur Herstellung neuer substituierter Benzophenone der Formel

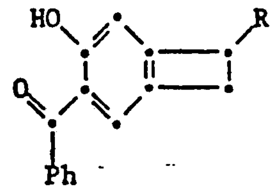

(I),

worin Ph unsubstituierter oder substituierte Phenyl bedeutet und R freies, verestertes oder amidiertes Carboxy bedeutet, und ihrer Salze, dadurch gekennzeichnet, dass man eine Verbindung bzw. ein Verbindungsgemisch der Formel

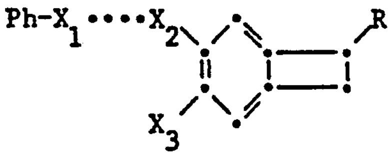

(II),

worin $X_1$ und $X_2$ gemeinsam eine über die Carbonylgruppe mit dem Rest Ph verbundene Gruppe der Formel -C(=O)-O bedeutet und $X_3$ Wasserstoff ist oder einer der Reste $X_1$ und $X_3$ eine Gruppe der Formel -C(=O)-Z, der andere Wasserstoff und $X_2$ eine Gruppe R'O- bedeutet, in der R' Niederalkyl oder eine Hydroxyschutzgruppe R'' darstellt oder, sofern $X_3$ Wasserstoff ist, ebenfalls Wasserstoff bedeuten kann, der Säurebehandlung unterwirft und das Primärprodukt solvolytisch zersetzt oder in einer Verbindung der Formel

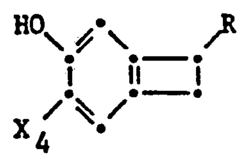

(V)

worin $X_4$ einen oxidativ oder solvolytisch in eine Gruppe der Formel -C(=O)-Ph überführbaren Rest bedeutet, $X_4$ zu der gewünschten Gruppe der Formel -C(=O)-Ph oxidiert oder solvolysiert oder Verbindung der Formel

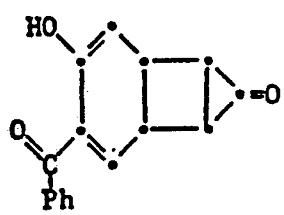

(XI)

oder ein Phenolatsalz davon mit Wasser, einem Alkohol oder mit Ammoniak oder einem mindestens ein Wasserstoffatom aufweisendes Amin behandelt und aus einem verfahrensgemäss erhältlichen Isomerengemisch erforderlichenfalls das Isomere der Formel 1 isoliert oder eine Verbindung der Formel

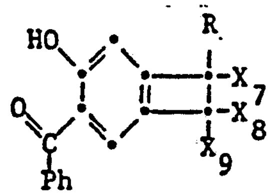

(XIII),

worin mindestens einer der Reste $X_7$ und $X_8$ gegebenenfalls veräthertes oder verestertes Hydroxy und ein davon verschiedener Rest $X_7$ oder $X_8$ sowie $X_9$ für Wasserstoff steht, oder $X_7$ Wasserstoff bedeutet und $X_8$ und $X_9$ gegebenenfalls funktionell abgewandeltes Oxo darstellen, oder ein Phenolat- und/oder Carboxylatsalz davon reduziert oder in einem von einem Ester oder Amid der Formel 1 verschiedenen funktionellen Carboxyderivat derselben oder einem Salz davon die funktionell abgewandelte Carboxygruppe solvolytisch in eine gegebenenfalls veresterte oder amidierte Carboxygruppe überführt oder in einer Verbindung der Formel

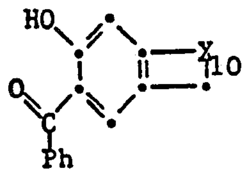

(XXI)

worin $X_{10}$ eine Gruppe der Formel 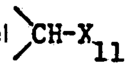

und $X_{11}$ einen oxidativ in eine Gruppe R überführbaren Rest bedeutet, den Rest $X_{11}$ zu einer Gruppe R oxidiert oder in einer Verbindung der Formel

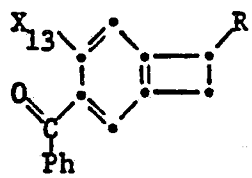

(XXXVII)

worin $X_{13}$ eine in Hydroxy überführbare Gruppe darstellt, oder in einem Salz davon $X_{13}$ in Hydroxy überführt, wobei man die oxidative Überführung eines Restes $X_4$ in eine Gruppe Ph-C(=O)- in Verbindung der Formel V mit der oxidativen Überführung eines Restes $X_{10}$ in eine Gruppe 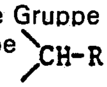

in Verbindungen der Formel XXI kombinieren kann, indem man von einer entsprechenden Verbindung der Formel

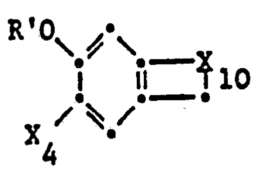

(XXXIX)

ausgeht, bzw. die solvolytische Überführung eines Restes $X_4$ in eine Gruppe Ph-C(=O)- in Verbindungen der Formel V mit der solvolytischen Überführung eines Restes $X_{10}$ in eine Gruppe 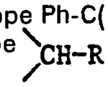

in Verbindung der Formel XXI und/oder die solvolytische Überführung von $X_{13}$ in Hydroxy in Verbindungen der Formel XXXVII zusammenfassen kann, indem man von einer entsprechenden Verbindung der Formel XXXIX oder einer Verbindung der Formel

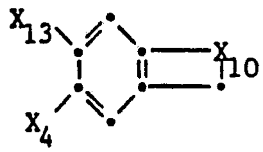

(XL)

Umsetzung abspaltet, und gewünschtenfalls die verfahrensgemäss erhältliche Verbindung in eine andere Verbindung der Formel I umwandelt und/oder eine verfahrensgemäss erhältliche Verbindung in ein Salz oder ein verfahrensgemäss erhältliches Salz in die freie Verbindung oder in ein anderes Salz überführt.

2. Verfahren gemäss Patentanspruch 1, dadurch gekennzeichnet, dass man Verbindungen der Formel I, worin Ph unsubstituiertes oder durch Niederalkyl, Niederalkoxy, Niederalkylthio, Niederalkanoyloxy, Halogen, Trifluormethyl, Carboxy, Niederalkoxycarbonyl, Cyano und/oder Nitro substituiertes Phenyl bedeutet und R Carboxy, mit einem niederen aliphatischen Alkohol verestertes Carboxy oder als Aminogruppe unsubstituiertes oder durch niedere aliphatische Reste mono- oder disubstituiertes Amino aufweisendes Carbamyl darstellt, oder ein Salz davon mit einer Base herstellt.

3. Verfahren gemäss Patentanspruch 1, dadurch gekennzeichnet, dass man Verbindungen der Formel I, worin Ph unsubstituiertes oder durch Niederalkyl mit bis und mit 4 C-Atomen, Niederalkoxy mit bis und mit 4 C-Atomen, Niederalkanoyloxy mit bis und mit 4 C-Atomen, Halogen der Atomnummer bis und mit 35 und/oder Trifluormethyl mono- oder disubstituiertes Phenyl bedeutet und R Carboxy, Niederalkoxycarbonyl mit bis und mit 5 C-Atomen oder Carbamyl bzw. N-Niederalkylcarbamyl mit bis und mit 5 C-Atomen bedeutet, oder ein Salz davon mit einer Base herstellt.

4. Verfahren gemäss Patentanspruch 1, dadurch gekennzeichnet, dass man Verbindungen der Formel I, worin Ph unsubstituiertes oder durch Niederalkyl mit bis und mit 4 C-Atomen, Niederalkylthio mit bis und mit 4 C-Atomen, Niederalkoxycarbonyl mit bis und mit 4 C-Atomen im Niederalkoxyteil, Halogen der Atomnummer bis und mit 35, und/oder Cyano, mono- oder disubstituiertes Phenyl bedeutet und R Carboxy oder Carbamyl, ferner Niederalkoxycarbonyl mit bis und mit 4 C-Atomen im Niederalkoxyteil bedeutet oder ein Salz davon mit einer Base herstellt.

5. Verfahren gemäss Patentanspruch 1, dadurch gekennzeichnet, dass man Verbindungen der Formel I, worin Ph durch Niederalkyl mit bis und mit 4 C-Atomen oder Halogen der Atomnummer bis und mit 35, monosubstituiertes oder disubstituiertes Phenyl bedeutet und R Carboxy oder Carbamyl, ferner Niederalkoxycarbonyl mit bis und mit 4 C-Atomen im Niederalkoxyteil bedeutet, oder ein Salz davon mit einer Base herstellt.

6. Verfahren gemäss Patentanspruch 1, dadurch gekennzeichnet, dass man Verbindungen der Formel I, worin Ph durch Halogen der Atomnummer bis und mit 35 in 2-Stellung monosubstituiertes Phenyl bedeutet und R Carboxy oder Carbamoyl darstellt, oder ein Salz davon mit einer Base herstellt.

7. Verfahren gemäss Patentanspruch 1, dadurch gekennzeichnet, dass man 4-(Benzoyl)-5-hydroxy-benzocyclobuten-1-carbonsäuremethylester, 4-(o-Chlorbenzoyl)-5-hydroxy-benzocyclobuten-1-carbonsäuremethylester, 4-(m-Chlorbenzoyl)-5-hydroxy-benzocyclobuten-1-carbonsäuremethylester, 4-(m-Methylbenzoyl)-5-hydroxy-benzocyclobuten-1-carbonsäuremethylester, 4-(p-Chxlorbenzoyl)-5-hydroxy-benzocyclobuten-1-carbonsäuremethylester, 4-(p-Methylbenzoyl)-5-hydroxy-benzocyclobuten-1-carbonsäuremethylester, 4-(2,6-Dichlorbenzoyl)-5-hydroxy-benzocyclobuten-1-carbonsäuremethylester, 4-(2,4-Dichlorbenzoyl)-5-hydroxy-benzocyclobuten-1-carbonsäuremethylester, 4-(3,4-Dichlorbenzoyl)-5-hydroxy-benzocyclobuten-1-carbonsäuremethylester, 4-(o-Brombenzoyl)-5-hydroxy-benzocyclobuten-1-carbonsäuremethylester, 4-(o-Acetoxybenzoyl)-5-hydroxy-benzocyclobuten-1-carbonsäuremethylester, 4-(o-Methylthiobenzoyl)-5-hydroxy-benzocyclobuten-1-carbonsäuremethylester, 4-(o-Trifluormethylbenzoyl)-5-hydroxy-benzocyclobuten-1-carbonsäuremethylester, 4-(m-Trifluormethylbenzoyl)-5-hydroxy-benzocyclobuten-1-carbonsäuremethylester, 4-(p-Methoxybenzoyl)-5-hydroxy-benzocyclobuten-1-carbonsäuremethylester, 4-(o-Methoxybenzoyl)-5-hydroxy-benzocyclobuten-1-carbonsäuremethylester, 4-(p-Cyanbenzoyl)-5-hydroxy-benzocyclobuten-1-carbonsäuremethylester, 4-(m-Methylthiobenzoyl)-5-hydroxy-benzocyclobuten-1-carbonsäuremethylester, 4-(m-Methoxycarbonylbenzoyl)-5-hydroxy-benzocyclobuten-1-carbonsäuremethylester, 4-(2,6-Dichlorbenzoyl)-5-hydroxy-benzocyclobuten-1-carboxamid, 4-(2,6-Dimethylbenzoyl)-5-hydroxy-benzocyclobuten-1-carbonsäuremethylester, 4-(2,6-Dimethoxybenzoyl)-5-hydroxy-benzocyclobuten-1-carbonsäuremethylester oder 4-(o-Methylbenzoyl)-5-hydroxy-benzocyclobuten-1-carbonsäuremethylester herstellt.

8. Verfahren gemäss Patentanspruch 1, dadurch gekennzeichnet, dass man 4-(Benzoyl)-5-hydroxy-benzocyclobuten-1-carbonsäure, 4-(m-Chlorbenzoyl)-5-hydroxy-benzocyclobuten-1-carbonsäure, 4-(m-Methylbenzoyl)-5-hydroxy-benzocyclobuten-1-carbonsäure, 4-(p-Chlorbenzoyl)-5-hydroxy-benzocyclobuten-1-carbonsäure, 4-(2,6-Dichlorbenzoyl)-5-hydroxy-benzocyclobutan-1-carbonsäure, 4-(3,4-Dichlorbenzoyl)-5-hydroxy-benzocyclobuten-1-carbonsäure, 4-(o-Acetoxybenzoyl)-5-hydroxy-benzocyclobuten-1-carbonsäure, 4-(o-Methylthiobenzoyl)-5-hydroxy-benzocyclobuten-1-carbonsäure, 4-(o-Trifluormethylbenzoyl)-5-hydroxy-benzocyclobuten-1-carbonsäure, 4-(m-Trifluormethylbenzoyl)-5-hydroxy-benzocyclobuten-1-carbonsäure, 4-(o-Methoxybenzoyl)-5-hydroxy-benzocyclobuten-1-carbonsäure, 4-(p-Methoxybenzoyl)-5-hydroxy-benzocyclobuten-1-carbonsäure, 4-(p-Cyanobenzoyl)-5-hydroxy-benzocyclobuten-1-carbonsäure, 4-(2,6-Dimethylbenzoyl)-5-hydroxy-benzocyclobuten-1-carbonsäure, 4-(m-Methylthiobenzoyl)-5-hydroxy-benzocyclobuten-1-carbonsäure, 4-(2,6-Dimethoxybenzoyl)-5-hydroxy-benzocyclobuten-1-carbonsäure, 4-(p-Methylbenzoyl)-5-hydroxy-benzocyclobuten-1-carbonsäure, 4-(o-Chlorbenzoyl)-5-hydroxy-benzocyclobuten-1-carbonsäure oder jeweils ein Salz davon mit einer Base herstellt.

9. Verfahren gemäss Patentanspruch 1, dadurch gekennzeichnet, dass man 4-(o-Fluorbenzoyl)-5-hydroxy-benzocyclobuten-1-carbonsäure oder ain Salz davon mit einer Base herstellt.

10. Verfahren gemäss Patentanspruch 1, dadurch gekennzeichnet, dass man 4-(o-Brombenzoyl)-5-hydroxy-benzocyclobuten-1-carbonsäure oder ein Salz davon mit einer Base herstellt.

11. Verfahren zur Herstellung von pharmazeutischen Präparaten, dadurch gekennzeichnet, dass man eine gemäss einem der Patentansprüche 1-10 erhältliche Verbindung mit üblichen pharmazeutischen Hilfs- und/oder Trägerstoffen vermischt.

**Claims**

for the Contracting States: BE, CH, DE, FR, GB, IT, LI, LU, NL, SE.

1. A novel substituted benzophenone of the formula

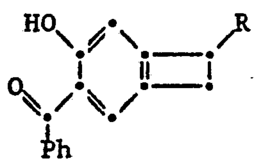

(I)

in which Ph represents unsubstituted or substituted phenyl and R represents free, esterified or amidated carboxy, or a salt thereof.

2. A compound according to claim 1, in which Ph represents phenyl that is unsubstituted or substituted by lower alkyl, lower alkoxy, lower alkylthio, lower alkanoyloxy, halogen, trifluoromethyl, carboxy, lower alkoxycarbonyl, cyano and/or nitro, and R represents carboxy, carboxy esterified by a lower aliphatic alcohol, or carbamoyl having as amino group amino that is unsubstituted or mono- or di-substituted by lower aliphatic radicals, or a salt thereof with a base.

3. A compound according to claim 1, in which Ph represents phenyl that is unsubstituted or mono- or di-substituted by lower alkyl, lower alkoxy, lower alkanoyloxy, halogen, trifluoromethyl, cyano and/or nitro, and R represents carboxy, carboxy esterified by a lower aliphatic alcohol, or carbamoyl having as amino group amino that is unsubstituted or mono- or di-substituted by lower aliphatic radicals, or a salt thereof with a base.

4. A compound according to claim 1, in which Ph represents phenyl that is unsubstituted or mono- or di-substituted by lower alkyl having up to and including 4 C-atoms, lower alkoxy having up to and including 4 C-atoms, lower alkanoyloxy having up to and including 4 C-atoms, halogen having an atomic number of up to and including 35, and/or trifluoromethyl and R represents carboxy, lower alkoxycarbonyl having up to and including 5 C-atoms, or carbamoyl or N-lower alkylcarbamoyl having up to and including 5 C-atoms, or a salt thereof with a base.

5. A compound according to claim 1, in which Ph represents phenyl that is unsubstituted or mono- or di-substituted by lower alkyl having up to and including 4 C-atoms, lower alkylthio having up to and including 4 C-atoms, lower alkoxycarbonyl having up to and including 4 C-atoms in the lower alkoxy moiety, halogen having an atomic number of up to and including 35, and/or cyano, and R represents carboxy or carbamoyl, and also lower alkoxycarbonyl having up to and including 4 C-atoms in the lower alkoxy moiety, or a salt thereof with a base.

6. A compound according to claim 1, in which Ph represents phenyl that is mono- or di-substituted by lower alkyl having up to and including 4 C-atoms or by halogen having an atomic number of up to and including 35, and R represents carboxy or carbamoyl, and also lower alkoxycarbonyl having up to and including 4 C-atoms in the lower alkoxy moiety, or a salt thereof with a base.

7. A compound according to claim 1, in which Ph represents phenyl that is monosubstituted in the 2-position by halogen having an atomic number of up to and including 35, and R represents carboxy or carbamoyl, or a salt thereof with a base.

8. 4-(Benzoyl)-5-hydroxybenzocyclobutene-1-carboxylic acid methyl ester, 4-(o-chlorobenzoyl)-5-hydroxybenzocyclobutene-1-carboxylic acid methyl ester, 4-(m-chlorobenzoyl)-5-hydroxybenzocyclobutene-1-carboxylic acid methyl ester, 4-(m-methylbenzoyl)-5-hydroxybenzocyclobutene-1-carboxylic acid methyl ester, 4-(p-chlorobenzoyl)-5-hydroxybenzocyclobutene-1-carboxylic acid methyl ester, 4-(p-methylbenzoyl)-5-hydroxybenzocyclobutene-1-carboxylic acid methyl ester, 4-(2,6-dichlorobenzoyl)-5-hydroxybenzocyclobutene-1-carboxylic acid methyl ester, 4-(2,4-dichlorobenzoyl)-5-hydroxybenzocyclobutene-1-carboxylic acid methyl ester, 4-(3,4-dichlorobenzoyl)-5-hydroxybenzocyclobutene-1-carboxylic acid methyl ester, 4-(o-bromobenzoyl)-5-hydroxybenzocyclobutene-1-carboxylic acid methyl ester, 4-(o-acetoxybenzoyl)-5-hydroxybenzocyclobutene-1-carboxylic acid methyl ester, 4-(o-methylthiobenzoyl)-5-hydroxybenzocyclobutene-1-carboxylic acid methyl ester, 4-(o-trifluoromethylbenzoyl)-5-hydroxybenzocyclobutene-1-carboxylic acid methyl ester, 4-(m-trifluoromethylbenzoyl)-5-hydroxybenzocyclobutene-1-carboxylic acid methyl ester, 4-(p-methoxybenzoyl)-5-hydroxybenzocyclobutene-1-carboxylic acid methyl ester, 4-(o-methoxybenzoyl)-5-hydroxybenzocyclobutene-1-carboxylic acid methyl ester, 4-(p-cyanobenzoyl)-5-hydroxybenzocyclobutene-1-carboxylic acid methyl ester, 4-(2,6-dimethylbenzoyl)-5-hydroxybenzoylcyclobutene-1-carboxylic acid methyl ester, 4-(2,6-dimethoxybenzoyl)-5-hydroxybenzocyclobutene-1-carboxylic acid methyl ester, 4-(o-methylbenzoyl)-5-hydroxybenzocyclobutene-1-carboxylic acid methyl ester or 4-(o-fluorobenzoyl)-5-hydroxybenzocyclobutene-1-carboxylic acid methyl ester according to claim 1.

9. 4-(Benzoyl)-5-hydroxybenzocyclobutene-1-carboxylic acid, 4-(m-chlorobenzoyl)-5-hydroxybenzocyclobutene-1-carboxylic acid, 4-(m-methylbenzoyl)-5-hydroxybenzocyclobutene-1-carboxylic acid, 4-(p-chlorobenzoyl)-5-hydroxybenzocyclobutene-1-carboxylic acid, 4-(2,6-dichlorobenzoyl)-5-hydroxybenzocyclobutene-1-carboxylic acid, 4-(3,4-dichlorobenzoyl)-5-hydroxybenzocyclobutene-1-carboxylic acid, 4-(o-acetoxybenzoyl)-5-hydroxybenzocyclobutene-1-carboxylic acid, 4-(m-methylthiobenzoyl)-5-hydroxybenzocyclobutene-1-carboxylic acid methyl ester, 4-(o-methylthiobenzoyl)-5-hydroxybenzocyclobutene-1-carboxylic acid, 4-(o-trifluoromethylbenzoyl)-5-hydroxybenzocyclobutene-1-carboxylic acid, 4-(m-trifluoromethylbenzoyl)-5-hydroxybenzocyclobutene-1-carboxylic acid, 4-(o-methoxybenzoyl)-5-hydroxybenzocyclobutene-1-carboxylic acid, 4-(p-methoxybenzoyl)-5-hydroxybenzocyclobutene-1-carboxylic acid, 4-(p-cyanobenzoyl)-5-hydroxybenzocyclobutene-1-carboxylic acid, 4-(2,6-dimethylbenzoyl)-5-hydroxybenzocyclobutene-1-carboxylic acid, 4-(m-methylthiobenzoyl)-5-hydroxybenzocyclobutene-1-carboxylic acid, 4-(2,6-dimethoxybenzoyl)-5-hydroxybenzocyclobutene-1-carboxylic acid, 4-(p-methylbenzoyl)-5-hydroxybenzocyclobutene-1-carboxylic acid, 4-(o-chlorobenzoyl)-5-hydroxybenzocyclobutene-1-carboxylic acid, 4-(o-methylbenzoyl)-5-hydroxybenzocyclobutene-1-carboxylic acid or 4-(2,4-dichlorobenzoyl)-5-hydroxybenzocyclobutene-1-carboxylic acid or a salt of one said compounds with a base according to claim 1.

10. 4-(o-Fluorobenzoyl)-5-hydroxybenzocyclobutene-1-carboxylic acid or a salt thereof with a base according to claim 1.

11. 4-(o-Bromobenzoyl)-5-hydroxybenzocyclobutene-1-carboxylic acid or a salt thereof with a base according to claim 1.

12. 4-(o-Fluorobenzoyl)-5-hydroxybenzocyclobutene-1-carboxamide, 4-(m-methoxycarbonylbenzoyl)-5-hydroxybenzocyclobutene-1-carboxylic acid methyl ester or 4-(2,6-dichlorobenzoyl)-5-hydroxybenzocyclobutene-1-carboxamide according to claim 1.

13. A compound according to any one of claims 1, 3, 4 and 8 to 11 for use in a therapeutic method of treating the human or animal body.

14. A compound according to any one of claims 2, 5, 6, 7 and 12 for use in a therapeutic method of treating the human or animal body.

15. A compound according to any one of claims 1, 3, 4 and 8 to 11 for use according to claim 13 as antiinflammatory agent and/or antinociceptive agent.

16. A compound according to any one of claims 2, 5, 6, 7 and 12 for use according to claim 14 as antiinflammatory agent and/or antinociceptive agent.

17. A process for the preparation of a novel substituted benzophenone of formula I according to claim 1, or of a salt thereof, characterised in that a compound or a mixture of compounds of the formula

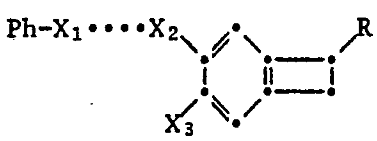

(II)

in which $X_1$ and $X_2$ together represent a group of the formula -C(=O)-O- that is bonded via the carbonyl group to the radical Ph and $X_3$ represents hydrogen, or one of the radicals $X_1$ and $X_3$ represents a group of the formula -C(=O)-Z, the other represents hydrogen and $X_2$ represents a group R'O- in which R' represents lower alkyl or a hydroxy-protecting group R'', or, if $X_3$ represents hydrogen, R' may also represent hydrogen, is subjected to acid treatment and the primary product is decomposed solvolytically, or, in a compound of the formula

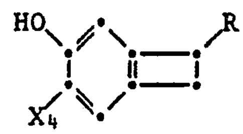

(V)

in which $X_4$ represents a radical that can be converted by oxidation or solvolysis into a group of the formula -C(=O)-Ph, $X_4$ is oxidised or solvolysed to the desired group of the formula -C(=O)-Ph, or a compound of the formula

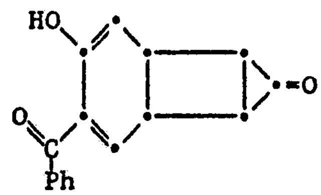

(XI)

or a phenolate salt thereof, is treated with water, an alcohol or ammonia or an amine having at least one hydrogen atom and, if necessary, the isomer of the formula I is isolated from a mixture of isomers obtainable according to the process, or a compound of the formula

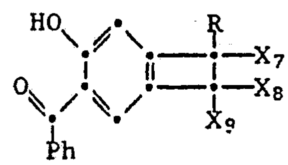

(XIII)

or a phenolate and/or carboxylate salt thereof, in which formula at least one of the radicals $X_7$ and $X_8$ represents free, etherified or esterified hydroxy and a radical $X_7$ or $X_8$ that does not represent free, etherified or esterified hydroxy represents hydrogen, as does $X_9$, or $X_7$ represents hydrogen and $X_8$ and $X_9$ represent free or functionally modified oxo, is reduced, or, in a functional carboxy derivative of the same that is other than an ester or amide of the formula I, or in a salt thereof, the functionally modified carboxy group is converted by solvolysis into a free, esterified or amidated carboxy group, or, in a compound of the formula

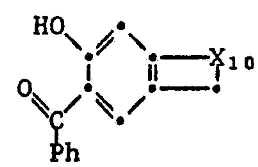

(XXI)

in which $X_{10}$ represents a group of the formula >CH-$X_{11}$

and $X_{11}$ represents a radical that can be converted by oxidation into a group R, the radical $X_{11}$ is oxidised to a group R, or, in a compound of the formula

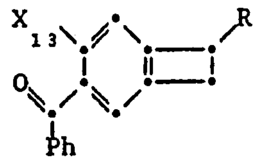

(XXXVII)

in which $X_{13}$ represents a group that can be converted into hydroxy, or in a salt thereof, $X_{13}$ is converted into hydroxy, it being possible to combine the oxidative conversion of a radical $X_4$ into a a group Ph-C(=O)- in compounds of the formula V with the oxidative conversion of a radical $X_{10}$ into a group 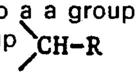

in compounds of the formula XXI by starting from a corresponding compound of the formula

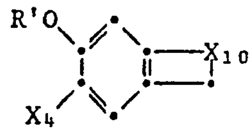

(XXXIX)

or to combine the solvolytic conversion of a radical $X_4$ into a group Ph-C(=O)- in compounds of the formula V with the solvolytic conversion of a radical $X_{10}$ into a group 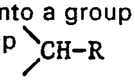

in compounds of the formula XXI and/or with the solvolytic conversion of $X_{13}$ into hydroxy in compounds of the formula XXXVII by starting from a corresponding compound of the formula XXXIX or a compound of the formula

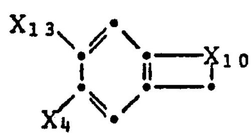

(XL)

the hydroxy-protecting group is, if appropriate, removed after carrying out the reaction and, if desired, the compound obtainable according to the process is converted into a different compound of the formula I and/or a compound obtainable according to the process is converted into a salt, or a salt obtainable according to the process is converted into the free compound or into a different salt.

18. A pharmaceutical composition containing a compound according to any one of claims 1, 3, 4 and 8 to 11, together with customary pharmaceutical adjuvants and/or carriers.

19. A pharmaceutical composition containing a compound according to any one of claims 2, 5, 6, 7 and 12, together with customary pharmaceutical adjuvants and/or carriers.

## Claims

for the Contracting State: AT

1. A process for the preparation of a novel substituted benzophenone of the formula

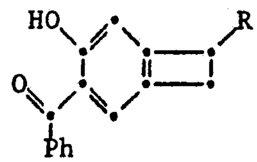

(I)

in which Ph represents unsubstituted or substituted phenyl and R represents free, esterified or amidated carboxy, or of a salt thereof, characterised in that a compound or a mixture of compounds of the formula

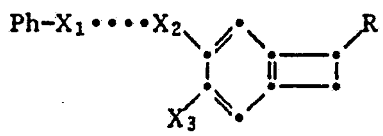

(II)

in which $X_1$ and $X_2$ together represent a group of the formula -C(=O)-O- that is bonded via the carbonyl group to the radical Ph and $X_3$ represents hydrogen, or one of the radicals $X_1$ and $X_3$ represents a group of the formula -C(=O)-Z, the other represents hydrogen and $X_2$ represents a group R'O- in which R' represents lower alkyl or a hydroxy-protecting group R'', or, if $X_3$ represents hydrogen, R' may also represent hydrogen, is subjected to acid treatment and the primary product is decomposed solvolytically, or, in a compound of the formula

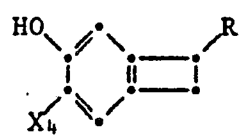

(V)

in which $X_4$ represents a radical that can be converted by oxidation or solvolysis into a group of the formula -C(=O)-Ph, $X_4$ is oxidised or solvolysed to the desired group of the formula -C(=O)-Ph, or a compound of the formula

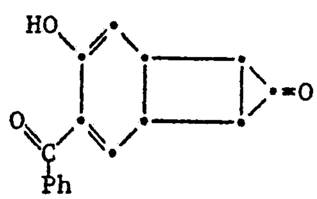

(XI)

or a phenolate salt thereof, is treated with water, an alcohol or ammonia or an amine having at least one hydrogen atom and, if necessary, the isomer of the formula I is isolated from a mixture of isomers obtainable according to the process, or a compound of the formula

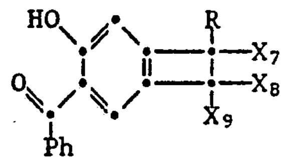

(XIII)

or a phenolate and/or carboxylate salt thereof, in which formula at least one of the radicals $X_7$ and $X_8$ represents free, etherified or esterified hydroxy and a radical $X_7$ or $X_8$ that does not represent free, etherified or esterified hydroxy represents hydrogen, as does $X_9$, or $X_7$ represents hydrogen and $X_8$ and $X_9$ represent free or functionally modified oxo, is reduced, or, in a functional carboxy derivative of the same that is other than an ester or amide of the formula I, or in a salt thereof, the functionally modified carboxy group is converted by solvolysis into a free, esterified or amidated carboxy group, or, in a compound of the formula

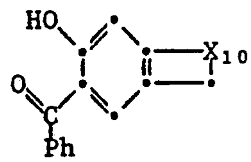

(XXI)

in which $X_{10}$ represents a group of the formula 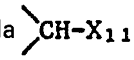

and $X_{11}$ represents a radical that can be converted by oxidation into a group R, the radical $X_{11}$ is oxidised to a group R, or, in a compound of the formula

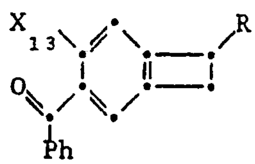

(XXXVII)

in which $X_{13}$ represents a group that can be converted into hydroxy, or in a salt thereof, $X_{13}$ is converted into hydroxy, it being possible to combine the oxidative conversion of a radical $X_4$ into a a group Ph-C(=O)- in compounds of the formula V with the oxidative conversion of a radical $X_{10}$ into a group 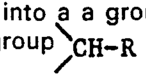

in compounds of the formula XXI by starting from a corresponding compound of the formula

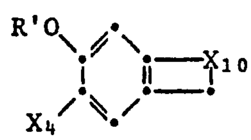

(XXXIX)

or to combine the solvolytic conversion of a radical $X_4$ into a group Ph-C(=O)- in compounds of the formula V with the solvolytic conversion of a radical $X_{10}$ into a group 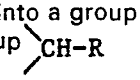

in compounds of the formula XXI and/or with the solvolytic conversion of $X_{13}$ into hydroxy in compounds of the formula XXXVII by starting from a corresponding compound of the formula XXXIX or a compound of the formula

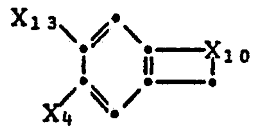

(XL)

the hydroxy-protecting group is, if appropriate, removed after carrying out the reaction and, if desired, the compound obtainable according to the process is converted into a different compound of the formula I and/or a compound obtainable according to the process is converted into a salt, or a salt obtainable according to the process is converted into the free compound or into a different salt.

2. A process according to claim 1, characterised by preparing a compound of the formula I, in which Ph represents phenyl that is unsubstituted or substituted by lower alkyl, lower alkoxy, lower alkylthio, lower alkanoyloxy, halogen, trifluoromethyl, carboxy, lower alkoxycarbonyl, cyano and/or nitro, and R represents carboxy, carboxy esterified by a lower aliphatic alcohol, or carbamoyl having as amino group amino that is unsubstituted or mono- or di-substituted by lower aliphatic radicals, or a salt thereof with a base.

3. A process according to claim 1, characterised by preparing a compound of the formula I, in which Ph represents phenyl that is unsubstituted or mono- or di-substituted by lower alkyl having up to and including 4 C-atoms, lower alkoxy having up to and including 4 C-atoms, lower alkanoyloxy having up to and including 4 C-atoms, halogen having an atomic number of up to and including 35, and/or trifluoromethyl, and R represents carboxy, lower alkoxycarbonyl having up to and including 5 C-atoms, or carbamoyl or N-lower alkylcarbamoyl having up to and including 5 C-atoms, or a salt thereof with a base.

4. A process according to claim 1, characterised by preparing a compound of the formula I, in which Ph represents phenyl that is unsubstituted or mono- or di-substituted by lower alkyl having up to and including 4 C-atoms, lower alkylthio having up to and including 4 C-atoms, lower alkoxycarbonyl having up to and including 4 C-atoms in the lower alkoxy moiety, halogen having an atomic number of up to and including 35, and/or cyano, and R represents carboxy or carbamoyl, and also lower alkoxycarbonyl having up to and including 4 C-atoms in the lower alkoxy moiety, or a salt thereof with a base.

5. A process according to claim 1, characterised by preparing a compound of the formula I, in which Ph represents phenyl that is mono- or di-substituted by lower alkyl having up to and including 4 C-atoms or by halogen having an atomic number of up to and including 35, and R represents carboxy or carbamoyl, and also lower alkoxycarbonyl having up to and including 4 C-atoms in the lower alkoxy moiety, or a salt thereof with a base.

6. A process according to claim 1, characterised by preparing a compound of the formula I, in which Ph represents phenyl that is monosubstituted in the 2-position by halogen having an atomic number of up to and including 35, and R represents carboxy or carbamoyl, or a salt thereof with a base.

7. A process according to claim 1, characterised by preparing 4-(benzoyl)-5-hydroxybenzocyclobutene-1-carboxylic acid methyl ester, 4-(o-chlorobenzoyl)-5-hydroxybenzocyclobutene-1-carboxylic acid methyl ester, 4-(m-chlorobenzoyl)-5-hydroxybenzocyclobutene-1-carboxylic acid methyl ester, 4-(m-methylbenzoyl)-5-hydroxy-benzocyclobutene-1-carboxylic acid methyl ester, 4-(p-chlorobenzoyl)-5-hydroxybenzocyclobutene-1-carboxylic acid methyl ester, 4-(p-methylbenzoyl)-5-hydroxybenzocyclobutene-1-carboxylic acid methyl ester, 4-(2,6-dichlorobenzoyl)-5-hydroxybenzocyclobutene-1-carboxylic acid methyl ester, 4-(2,4-dichlorobenzoyl)-5-hydroxy-benzocyclobutene-1-carboxylic acid methyl ester, 4-(3,4-dichlorobenzoyl)-5-hydroxybenzocyclobutene-1-carboxylic axcid methyl ester, 4-(o-bromobenzoyl)-5-hydroxybenzocyclobutene-1-carboxylic acid methyl ester, 4-(o-acetoxybenzoyl)-5-hydroxybenzocyclobutene-1-carboxylic acid methyl ester, 4-(o-methylthiobenzoyl)-5-hydroxybenzocyclobutene-1-carboxylic acid methyl ester, 4-(o-trifluoromethylbenzoyl)-5-hydroxybenzocyclobutene-1-carboxylic acid methyl ester, 4-(m-trifluoromethylbenzoyl)-5-hydroxybenzocyclobutene-1-carboxylic acid methyl ester, 4-(p-methoxybenzoyl)-5-hydroxybenzocyclobutene-1-carboxylic acid methyl ester, 4-(o-methoxybenzoyl)-5-hydroxybenzocyclobutene-1-carboxylic acid methyl ester, 4-(p-cyanobenzoyl)-5-hydroxybenzocyclobutene-1-carboxylic acid methyl ester, 4-(m-methylthiobenzoyl)-5-hydroxybenzocyclobutene-1-carboxylic aacid methyl ester, 4-(m-methoxycarbonylbenzoyl)-5-hydroxybenzocyclobutene-1-carboxylic acid methyl ester, 4-(2,6-dichlorobenzoyl)-5-hydroxybenzocyclobutene-1-carboxamide, 4-(2,6-dimethylbenzoyl)-5-hydroxybenzocyclobutene-1-carboxylic acid methyl ester, 4-(2,6-dimethoxybenzoyl)-5-hydroxybenzocyclobutene-1-carboxylic acid methyl ester or 4-(o-methylbenzoyl)-5-hydroxybenzocyclobutene-1-carboxylic acid methyl ester.

8. A process according to claim 1, characterised by preparing 4-(benzoyl)-5-hydroxybenzocyclobutene-1-carboxylic acid, 4-(m-chlorobenzoyl)-5-hydroxybenzocyclobutene-1-carboxylic acid, 4-(m-methylbenzoyl)-5-hydroxybenzocyclobutene-1-carboxylic acid, 4-(p-chlorobenzoyl)-5-hydroxybenzocyclobutene-1-carboxylic acid, 4-(2,6-dichlorobenzoyl)-5-hydroxybenzocyclobutene-1-carboxylic acid, 4-(3,4-dichlorobenzoyl)-5-hydroxybenzocyclobutene-1-carboxylic acid, 4-(o-acetoxybenzoyl)-5-hydroxybenzocyclobutene-1-carboxylic acid, 4-(o-methylthiobenzoyl)-5-hydroxybenzocyclobutene-1-carboxylic acid, 4-(o-trifluoromethylbenzoyl)-5-hydroxybenzocyclobutene-1-carboxylic acid, 4-(m-trifluoromethylbenzoyl)-5-hydroxybenzocyclobutene-1-carboxylic acid, 4-(o-methoxybenzoyl)-5-hydroxybenzocyclobutene-1-carboxylic acid, 4-(p-methoxybenzoyl)-5-hydroxybenzocyclobutene-1-carboxylic acid, 4-(p-cyanobenzoyl)-5-hydroxybenzocyclobutene-1-carboxylic acid, 4-(2,6-dimethylbenzoyl)-5-hydroxybenzocyclobutene-1-carboxylic acid, 4-(m-methylthiobenzoyl)-5-hydroxybenzocyclobutene-1-carboxylic acid, 4-(2,6-dimethoxybenzoyl)-5-hydroxybenzocyclobutene-1-carboxylic acid, 4-(p-methylbenzoyl)-5-hydroxybenzocyclobutene-1-carboxylic acid, 4-(o-chlorobenzoyl)-5-hydroxybenzocyclobutene-1-carboxylic acid or a salt of one of said compounds with a base.

9. A process according to claim 1, characterised by preparing 4-(o-fluorobenzoyl)-5-hydroxybenzocyclobutene-1-carboxylic acid or a salt thereof with a base.

10. A process according to claim 1, characterised by preparing 4-(o-bromobenzoyl)-5-hydroxybenzocyclobutene-1-carboxylic acid or a salt thereof with a base.

11. A process for the preparation of a pharmaceutical composition, characterised in that a compound obtainable by a process according to any one of claims 1 to 10 is combined with customary pharmaceutical adjuvants and/or carriers.

**Revendications**

pour les Etats contractants: BE, CH, DE, FR, GB, IT, LI, LU, NL, SE.

1. Une nouvelle benzophénone substituée de formule

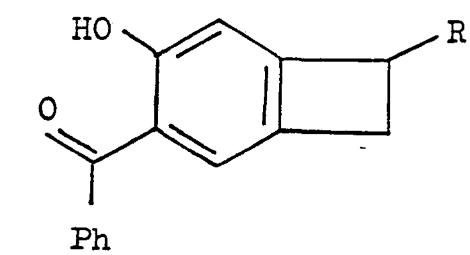

(I).

dans laquelle Ph représente un groupe phényle non substitué ou substitué et R représente un groupe carboxy libre, estérifié ou amidé, ou un sel de ce composé.

2. Un composé selon la revendication 1, dans lequel Ph représente un groupe phényle non substitué ou substitué par des groupes alkyle inférieurs, alcoxy inférieurs, alkylthio inférieurs, alcanoyloxy inférieurs, des halogènes, des groupes trifluorométhyle, carboxy, (alcoxy inférieur)-carbonyle, cyano et/ou nitro, et R représente un groupe carboxy, un groupe carboxy estérifié par un alcool aliphatique inférieur ou un groupe carbamyle portant en tant que groupe amino un groupe amino non substitué ou mono- ou di-substitué par des groupes aliphatiques inférieurs, ou un sel d'un tel composé et d'une base.

3. Un composé selon la revendication 1, dans lequel Ph représente un groupe phényle non substitué ou mono- ou di-substitué par des groupes alkyle inférieurs, alcoxy inférieurs, alcanoyloxy inférieurs, des halogènes, des groupes trifluorométhyle, cyano et/ou nitro, et R représente un groupe carboxy, un groupe carboxy estérifié par un alcool aliphatique inférieur ou un groupe carbamyle portant en tant que groupe amino un groupe amino non substitué ou mono- ou di-substitué par des groupes aliphatiques inférieurs, ou un sel d'un tel composé et d'une base.

4. Un composé selon la revendication 1, dans lequel Ph représente un groupe phényle non substitué ou mono- ou di-substitué par des groupes alkyle inférieurs contenant jusqu'à 4 atomes de carbone inclus, alcoxy inférieurs contenant jusqu'à 4 atomes de carbone inclus, alcanoyloxy inférieurs contenant jusqu'à 4 atomes de carbone inclus, des halogènes de numéro atomique allant jusqu'à 35 inclus et/ou des groupes trifluorométhyle, et R représente un groupe carboxy, (alcoxy inférieur)-carbonyle contenant jusqu'à 5 atomes de carbone inclus, ou carbamyle ou N-(alkyle inférieur)carbamyle contenant jusqu'à 5 atomes de carbone inclus, ou un sel d'un tel composé et d'une base.

5. Un composé selon la revendication 1, dans lequel Ph représente un groupe phényle non substitué ou mono- ou di-substitué par des groupes alkyle inférieurs contenant jusqu'à 4 atomes de carbone inclus, alkylthio inférieurs contenant jusqu'à 4 atomes de carbone inclus, (alcoxy inférieur)-carbonyle contenant jusqu'à 4 atomes de carbone inclus dans la partie alcoxy inférieur, des halogènes de numéro atomique allant jusqu'à 35 inclus et/ou des groupes cyano, et R représente un groupe carboxy ou carbamyle, ou encore (alcoxy inférieur)-carbonyle contenant jusqu'à 4 atomes de carbone inclus dans la partie alcoxy inférieur, ou un sel d'un tel composé et d'une base.

6. Un composé selon la revendication 1, dans lequel Ph représente un groupe phényle mono- ou di- substitué par des groupes alkyle inférieurs contenant jusqu'à 4 atomes de carbone inclus ou des halogènes de numéro

atomique allant jusqu'à 35 inclus et R représente un groupe carboxy ou carbamyle, ou encore (alcoxy inférieur)-carbonyle contenant jusqu'à 4 atomes de carbone inclus dans la partie alcoxy inférieur, ou un sel d'un tel composé et d'une base.

7. Un composé selon la revendication 1, dans lequel Ph représente un groupe phényle monosubstitué par un halogène de numéro atomique allant jusqu'à 35 inclus en position 2 et R représente un groupe carboxy ou carbamyle, ou un sel d'un tel composé et d'une base.

8. Le 4-(benzoyl)-5-hydroxy-benzocyclobutène-1-carboxylate de méthyle, le 4-(o-chlorobenzoyl)-5-hydroxy-benzocyclobutène-1-carboxylate de méthyle, le 4-(m-chlorobenzoyl)-5-hydroxy-benzocyclobutène-1-carboxylate de méthyle, le 4-(m-méthylbenzoyl)-5-hydroxy-benzocyclobutène-1-carboxylate de méthyle, le 4-(p-chlorobenzoyl)-5-hydroxy-benzocyclobutène-1-carboxylate de méthyle, le 4-(p-méthylbenzoyl)-5-hydroxy-benzocyclobutène-1-carboxylate de méthyle, le 4-(2,6-dichloro-benzoyl)-5-hydroxy-benzocyclobutène-1-carboxylate de méthyle, le 4-(2,4-dichlorobenzoyl)-5-hydroxy-benzocyclobutène-1-carboxylate de méthyle, le 4-(3,4-dichloro-benzoyl)-5-hydroxy-benzoylcyclobutène-1-carboxylate de métyle, le 4-(o-bromobenzoyl)-5-hydroxy-benzocyclobutène-1-carboxylate de méthyle, le 4-(o-acétoxybenzoyl)-5-hydroxy-benzocyclobutène-1-carboxylate de méthyle, le 4-(o-méthylthiobenzoyl)-5-hydroxy-benzocyclo-butène-1-carboxylate de méthyle, le 4-(o-trifluorométhyl-benzoyl)-5-hydroxy-benzocyclobutène-1-carboxylate de méthyle, le 4-(m-trifluorométhylbenzoyl)-5-hydroxy-benzo-cyclobutène-1-carboxylate de méthyle, le 4-(p-méthoxy-benzoyl)-5-hydroxy-benzocyclobutène-1-carboxylate de méthyle, le 4-(o-méthoxybenzoyl)-5-hydroxy-benzocyclobutène-1-carboxylate de méthyle, le 4-(p-cyanobenzoyl)-5-hydroxy-benzocyclobutène-1-carboxylate de méthyle, le 4-(2,6-diméthylbenzoyl)-5-hydroxy-benzocyclobutène-1-carboxylate de méthyle, le 4-(2,6-diméthoxybenzoyl)-5-hydroxy-benzocyclobutène-1-carboxylate de méthyle, le 4-(o-méthylbenzoyl)-5-hydroxy-benzocyclobutène-1-carboxylate de méthyle, ou le 4-(o-fluorobenzoyl)-5-hydroxy-benzocyclobutène-1-carboxylate de méthyle, selon la revendication 1.

9. L'acide 4-(benzoyl)-5-hydroxy-benzocyclobutène-1-carboxylique,
l'acide 4-(m-chlorobenzoyl)-5-hydroxy-benzocyclobutène-1-carboxylique,
l'acide 4-(m-méthylbenzoyl)-5-hydroxy-benzocyclobutène-1-carboxylique,
l'acide 4-(p-chlorobenzoyl)-5-hydroxy-benzocyclobutène-1-carboxylique,
l'acide 4-(2,6-dichlorobenzoyl)-5-hydroxy-benzocyclobutène-1-carboxylique,
l'acide 4-(3,4-dichlorobenzoyl)-5-hydroxy-benzocyclobutène-1-carboxylique,
l'acide 4-(o-acétoxybenzoyl)-5-hydroxy-benzocyclobutène-1-carboxylique,
le 4-(m-méthylthiobenzoyl)-5-hydroxy-benzocyclobutène-1-carboxylate de méthyle,
l'acide 4-(o-méthylthiobenzoyl)-5-hydroxy-benzocyclobutène-1-carboxylique,
l'acide 4-(o-trifluorométhylbenzoyl)-5-hydroxy-benzocyclobutène-1-carboxylique,
l'acide 4-(m-trifluorométhylbenzoyl)-5-hydroxy-benzocyclobutène-1-carboxylique,
l'acide 4-(o-méthoxybenzoyl)-5-hydroxy-benzocyclobutène-1-carboxylique,
l'acide 4-(p-méthoxybenzoyl)-5-hydroxy-benzocyclobutène-1-carboxylique,
l'acide 4-(p-cyanobenzoyl)-5-hydroxy-benzocyclobutène-1-carboxylique,
l'acide 4-(2,6-diméthylbenzoyl)-5-hydroxy-benzocyclobutène-1-carboxylique,
l'acide 4-(m-méthylthiobenzoyl)-5-hydroxy-benzocyclobutène-1-carboxylique,
l'acide 4-(2,6-diméthoxybenzoyl)-5-hydroxy-benzocyclobutène-1-carboxylique,
l'acide 4-(p-méthylbenzoyl)-5-hydroxy-benzocyclobutène-1-carboxylique,
l'acide 4-(o-chlorobenzoyl)-5-hydroxy-benzocyclobutène-1-carboxylique,
l'acide 4-(o-méthylbenzoyl)-5-hydroxy-benzocyclobutène-1-carboxylique, ou
l'acide 4-(2,4-dichlorobenzoyl)-5-hydroxy-benzocyclobutène-1-carboxylique,
ou un sel de l'un de ces composés et d'une base selon la revendication 1.

10. L'acide 4-(o-fluorobenzoyl)-5-hydroxy-benzocyclobutène-1-carboxylique ou un sel de cet acide et d'une base selon la revendication 1.

11. L'acide 4-(o-bromobenzoyl)-5-hydroxy-benzocyclobutène-1-carboxylique ou un sel de ce composé et d'une base selon la revendication 1.

12. Le 4-(o-fluorobenzoyl)-5-hydroxy-benzocyclobutène-1-carboxamide, le 4-(m-méthoxycarbonyl-benzoyl)-5-hydroxy-benzocyclobutène-1-carboxylate de méthyle ou le 4-(2,6-dichlorobenzoyl)-5-hydroxy-benzo-cyclobutène-1-carboxamide selon la revendication 1.

13. Un composé selon l'une des revendications 1, 3, 4 et 8 à 11, pour l'utilisation dans un procédé pour le traitement thérapeutique de l'organisme humain ou animal.

14. Un composé selon l'une des revendications 2, 5, 6, 7 et 12, pour l'utilisation dans un procédé pour le traitement thérapeutique de l'organisme humain ou animal.

15. Un composé selon l'une des revendications 1, 3, 4 et 8 à 11, pour l'utilisation selon la revendication 13 en tant qu'agent anti-inflammatoire et/ou antinociceptif.

16. Un composé selon l'une des revendications 2, 5, 6, 7 et 12, pour l'utilisation selon la revendication 14 en tant qu'agent anti-inflammatoire et/ou antinociceptif.

17. Procédé de préparation des nouvelles benzophénones substituées de formule I selon la revendication 1, et de leurs sels, caractérisé en ce que l'on soumet à traitement par un acide un composé ou mélange de composés de formule

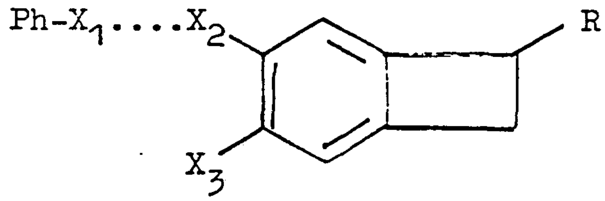

(II),

dans laquelle $X_1$ et $X_2$ représentent ensemble un groupe de formule -C(=O)-O relié au cycle Ph par l'intermédiaire du groupe carbonyle et $X_3$ représente l'hydrogène ou bien l'un des symboles $X_1$ et $X_3$ représente un groupe de formule -C(=O)-Z, l'autre l'hydrogène et $X_2$ un groupe R'O- dans lequel R' représente un groupe alkyle inférieur ou un groupe R'' protecteur du groupe hydroxy ou bien encore, lorsque $X_3$ représente l'hydrogène, également l'hydrogène, et on soumet le produit obtenu en premier à décomposition par solvolyse, ou bien,

dans un composé de formule

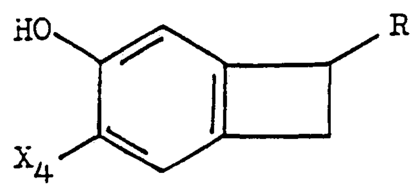

(V)

dans laquelle $X_4$ représente un groupe convertible par oxydation ou solvolyse en un groupe de formule -C(=O)-Ph, on oxyde ou on solvolyse $X_4$ en le groupe recherché de formule -C(=O)-Ph, ou bien

on traite un composé de formule

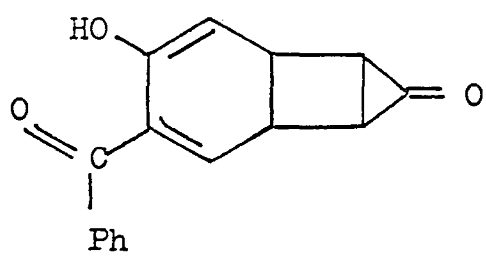

(XI)

ou un phénate de ce composé par l'eau, un alcool ou l'ammoniac ou une amine portant au moins un atome d'hydrogène et, à partir d'un mélange d'isomères éventuellement obtenu par ce procédé, on isole si c'est nécessaire l'isomère de formule I, ou bien on réduit un composé de formule

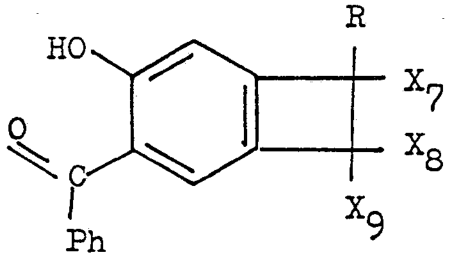

(XIII),

dans laquelle l'un au moins des symboles $X_7$ et $X_8$ représente un groupe hydroxy éventuellement éthérifié ou estérifié et l'autre, ainsi que $X_9$, représentent l'hydrogène, ou bien $X_7$ représente l'hydrogène et $X_8$ et $X_9$ un groupe oxo qui a éventuellement subi une modification fonctionnelle, ou un phénate et/ou carboxylate d'un tel composé, ou bien,

dans un dérivé carboxylé fonctionnel de formule I autre qu'un ester ou un amide de formule I, ou dans un sel d'un tel composé, on convertit le groupe carboxy à fonction modifiée par solvolyse en un groupe carboxy éventuellement estérifié ou amidé, ou bien

dans un composé de formule

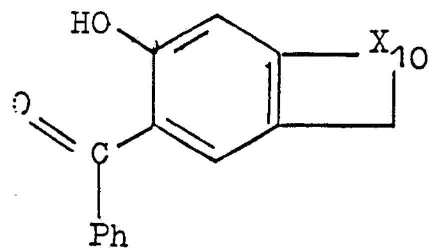

(XXI)

dans laquelle $X_{10}$ représente un groupe de formule $>CH-X_{11}$

et $X_{11}$ représente un groupe convertible par oxydation en un groupe R, on oxyde le groupe $X_{11}$ en un groupe R, ou bien,

dans un composé de formule

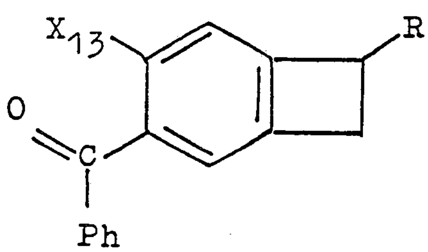

(XXXVII)

dans laquelle $X_{13}$ représente un groupe convertible en un groupe hydroxy, ou dans un sel d'un tel composé, on convertit $X_{13}$ en un groupe hydroxy, étant précisé que la conversion par oxydation d'un groupe $X_4$ en un groupe Ph-C(=O)- dans un composé de formule V peut être combinée avec la conversion par oxydation d'un groupe $X_{10}$ en un groupe $>CH-R$

dans les composés de formule XXI en partant d'un composé correspondant de formule

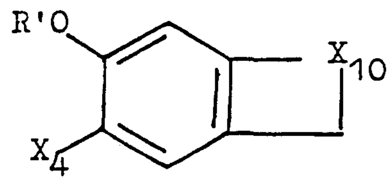

(XXXIX)

et que la conversion par solvolyse d'une groupe $X_4$ et un groupe Ph-C(=O)- dans les composés de formule V peut être associée à la conversion par solvolyse d'un groupe $X_{10}$ en un groupe $>CH-R$

dans le composé de formule XXI et/ou avec la conversion par solvolyse de $X_{13}$ en un groupe hydroxy dans les composés de formule XXXVII, en partant d'un composé correspondant de formule XXXIX ou d'un composé de formule

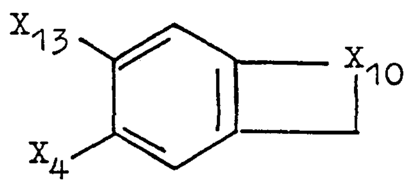

(XL)

le cas échéant, après la conversion, on élimine le groupe protecteur du groupe hydroxy et, si on le désire, on convertit le composé obtenu par le procédé en un autre composé de formule I et/ou un composé obtenu par le procédé en un sel ou un sel obtenu par le procédé en le composé libre ou en un autre sel.

18. Une composition pharmaceutique contenant un composé selon l'une des revendications 1, 3, 4 et 8 à 11, avec des produits auxiliaires et/ou véhicules pharmaceutiques usuels.

19. Une composition pharmaceutique contenant un composé selon l'une des revendications 2, 5, 6, 7 et 12, avec des produits auxiliaires et/ou véhicules pharmaceutiques usuels.

**Revendications**

pour l'Etat contractant: AT

1. Procédé de préparation de nouvelles benzophénones substituées de formule

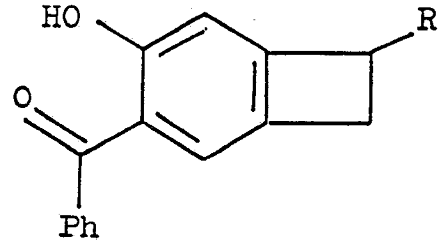

(I)

dans laquelle Ph représente un groupe phényle non substitué ou substitué et R représente un groupe carboxy libre, estérifié ou amidé, et de leurs sels, caractérisé en ce que l'on soumet à un traitement par un acide un composé ou mélange de composés de formule

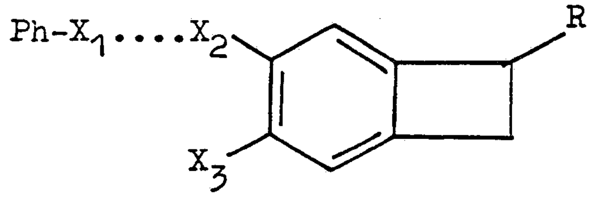

(II)

dans laquelle $X_1$ et $X_2$ représentent ensemble un groupe de formule -C(=O)-O relié au cycle Ph par l'intermédiaire du groupe carbonyle et $X_3$ représente l'hydrogène, ou bien l'un des symboles $X_1$ et $X_3$ représente un groupe de formule -C(=O)-Z, l'autre l'hydrogène et $X_2$ un groupe R'O- dans lequel R' représente un groupe alkyle inférieur ou un groupe protecteur du groupe hydroxy R'' ou bien encore, lorsque $X_3$ représente l'hydrogène, également l'hydrogène, et on décompose le produit obtenu en premier par solvolyse, ou bien

dans un composé de formule

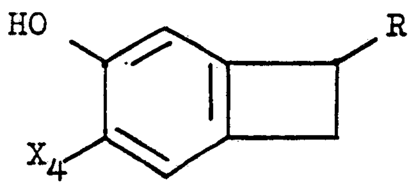

(V)

dans laquelle $X_4$ représente un groupe convertible par oxydation ou par solvolyse en un groupe de formule -C(=O)-Ph, on convertit $X_4$ en le groupe recherché de formule -C(=O)-Ph par oxydation ou solvolyse, ou bien on traite un composé de formule

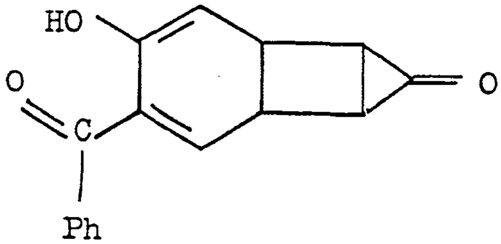

(XI)

ou un phénate de ce composé par l'eau, un alcool ou l'ammoniac ou une amine portant au moins un atome d'hydrogène et à partir d'un mélange d'isomères obtenu par ce procédé, on isole, lorsque c'est nécessaire, l'isomère de formule I, ou bien

on réduit un composé de formule

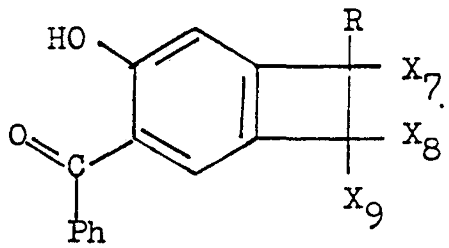

(XIII),

dans laquelle l'un au moins des symboles $X_7$ et $X_8$ représente un groupe hydroxy éventuellement éthérifié ou estérifié et l'autre, ainsi que $X_9$, représentent l'hydrogène, ou bien $X_7$ représente l'hydrogène et $X_8$ et $X_9$ un groupe oxo qui a éventuellement subi une modification fonctionnelle, ou un phénate et/ou un carboxylate d'un tel composé, ou bien

dans un dérivé carboxylé fonctionnel de formule I autre qu'un ester ou un amide de formule I, ou dans un sel d'un tel composé, on convertit le groupe carboxy qui a subi une modification fonctionnelle par solvolyse en un groupe carboxy éventuellement estérifié ou amidé, ou bien

dans un composé de formule

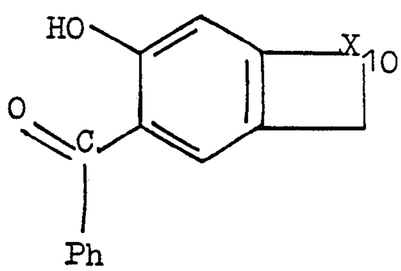

(XXI)

dans laquelle $X_{10}$ représente un groupe de formule $>CH-X_{11}$

et $X_{11}$ un groupe convertible par oxydation en un groupe R, on convertit le groupe $X_{11}$ en un groupe R par oxydation, ou bien,

dans un composé de formule

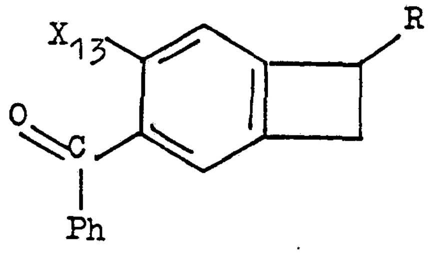

(XXXVII)

dans laquelle $X_{13}$ represente un groupe convertible en un groupe hydroxy, ou dans un sel d'un tel composé, on convertit $X_{13}$ en un groupe hydroxy, la conversion par oxydation d'un groupe $X_4$ dans un groupe Ph-C(=O)- d'un composé de formule V pouvant être combinée avec la conversion par oxydation d'un groupe $X_{10}$ d'un groupe >CH-R

de composés de formule XXI, en partant d'un composé correspondant de formule

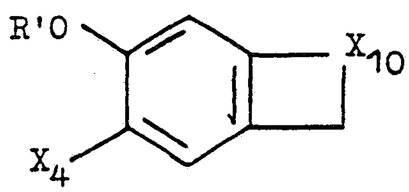

(XXXIX)

et la conversion par solvolyse d'un groupe $X_4$ dans un groupe Ph-C(=O)- de composés de formule V pouvant être associée avec la conversion par solvolyse d'un groupe $X_{10}$ dans un groupe >CH-R

d'un composé de formule XXI et/ou la conversion par solvolyse de $X_{13}$ en un groupe hydroxy dans les composés de formule XXXVII, en partant d'un composé correspondant de formule XXXIX ou d'un compose de formule

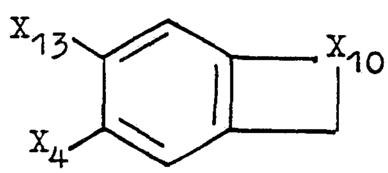

(XL)

le cas échéant, après avoir réalisé la réaction, on élimine le groupe protecteur du groupe hydroxy et, si on le désire, on convertit le composé obtenu par le procédé en un autre composé de formule I et/ou un composé obtenu par le procédé en un sel ou un sel obtenu par le procédé en le composé libre ou en un autre sel.

2. Procédé selon la revendication 1, caractérisé en ce que l'on prépare des composés de formule I dans laquelle Ph représente un groupe phényle non substitué ou substitué par des groupes alkyle inférieurs, alcoxy inférieurs, alkylthio inférieurs, alcanoyloxy inférieurs, des halogènes, des groupes trifluorométhyle, carboxy, (alcoxy inférieur)-carbonyle, cyano et/ou nitro et R représente un groupe carboxy, un groupe carboxy estérifié par un alcool aliphatique inférieur ou un groupe carbamyle portant en tant que groupe amino un groupe amino non substitué ou mono- ou di-substitué par des groupes alkyle inférieurs, ou un sel d'un tel composé et d'une

base.

3. Procédé selon la revendication 1, caractérisé en ce que l'on prépare des composés de formule I dans laquelle Ph représente un groupe phényle non substitué ou mono- ou di-substitué par des groupes alkyle inférieurs contenant jusqu'à 4 atomes de carbone inclus, alcoxy inférieurs contenant jusqu'à 4 atomes de carbone inclus, alcanoyloxy inférieurs contenant jusqu'à 4 atomes de carbone inclus, des halogènes de numéro atomique allant jusqu'à 35 inclus et/ou des groupes trifluorométhyle, et R représente un groupe carboxy, (alcoxy inférieur)-carbonyle contenant jusqu'à 5 atomes de carbone inclus ou carbamyle ou N(alkyle inférieur)-carbamyle contenant jusqu'à 5 atomes de carbone inclus, ou un sel d'un tel composé et d'une base.

4. Procédé selon la revendication 1, caractérisé en ce que l'on prépare des composés de formule I dans laquelle Ph représente un groupe phényle non substitué ou mono- ou di-substitué par des groupes alkyle inférieurs contenant jusqu'à 4 atomes de carbone inclus, alkylthio inférieurs contenant jusqu'à 4 atomes de carbone inclus, (alcoxy inférieur)-carbonyle contenant jusqu'à 4 atomes de carbone inclus dans la partie alcoxy inférieur, des halogènes de numéro atomique allant jusqu'à 35 inclus et/ou des groupes cyano, et R représente un groupe carboxy ou carbamyle, ou encore (alcoxy inférieur)-carbonyle contenant jusqu'à 4 atomes de carbone inclus dans la partie alcoxy inférieur, ou un sel d'un tel composé et d'une base.

5. Procédé selon la revendication 1, caractérisé en ce que l'on prépare des composés de formule I dans laquelle Ph représente un groupe phényle mono- ou di-substitué par des groupes alkyle inférieurs contenant jusqu'à 4 atomes de carbone inclus ou des halogènes de numéro atomique allant jusqu'à 35 inclus et R représente un groupe carboxy ou carbamyle, ou encore (alcoxy inférieur)-carbonyle contenant jusqu'à 4 atomes de carbone inclus dans la partie alcoxy inférieur, ou un sel d'un tel composé et d'une base.

6. Procédé selon la revendication 1, caractérisé en ce que l'on prépare des composés de formule I dans laquelle Ph représente un groupe phényle monosubstitué en position 2 par un halogène de numéro atomique allant jusqu'à 35 inclus et R représente un groupe carboxy ou carbamyle, ou un sel d'un tel composé et d'une base.

7. Procédé selon la revendication 1, caractérisé en ce que l'on prépare le 4-(benzoyl)-5-hydroxy-benzocyclobutène-1-carboxylate de méthyle,
le 4-(o-chlorobenzoyl)-5-hydroxy-benzocyclobutène-1-carboxylate de méthyle,
le 4-(m-chlorobenzoyl)-5-hydroxy-benzocyclobutène-1-carboxylate de méthyle,
le 4-(m-méthylbenzoyl)-5-hydroxy-benzocyclobutène-1-carboxylate de méthyle,
le 4-(p-chlorobenzoyl)-5-hydroxy-benzocyclobutène-1-carboxylate de méthyle,
le 4-(p-méthylbenzoyl)-5-hydroxy-benzocyclobutène-1-car-boxylate de méthyle,
le 4-(2,6-dichlorobenzoyl)-5-hydroxy-benzocyclobutène-1-carboxylate de méthyle,
le 4-(2,4-dichlorobenzoyl)-5-hydroxy-benzocyclobutène-1-carboxylate de méthyle,
le 4-(3,4-dichlorobenzoyl)-5-hydroxy-benzocyclobutène-1-carboxylate de méthyle,
le 4-(o-bromobenzoyl)-5-hydroxy-benzocyclobutène-1-carboxylate de méthyle,
le 4-(o-acétoxybenzoyl)-5-hydroxy-benzocyclobutène-1-carboxylate de méthyle,
le 4-(o-méthylthiobenzoyl)-5-hydroxy-benzocyclobutène-1-carboxylate de méthyle,
le 4-(o-trifluorométhylbenzoyl)-5-hydroxy-benzocyclobutène-1-carboxylate de méthyle,
le 4-(m-trifluorométhylbenzoyl)-5-hydroxy-benzocyclo-butène-1-carboxylate de méthyle,
le 4-(p-méthoxybenzoyl)-5-hydroxy-benzocyclobutène-1-carboxylate de méthyle,
le 4-(o-méthoxybenzoyl)-5-hydroxy-benzocyclobutène-1-carboxylate de méthyle,
le 4-(p-cyanobenzoyl)-5-hydroxy-benzocyclobutène-1-carboxylate de méthyle,
le 4-(m-méthylthiobenzoyl)-5-hydroxy-benzocyclobutène-1-carboxylate de méthyle,
le 4-(m-méthoxycarbonylbenzoyl)-5-hydroxy-benzocyclobutène-1-carboxylate de méthyle,
le 4-(2,6-dichlorobenzoyl)-5-hydroxy-benzocyclobutène-1-carboxamide,
le 4-(2,6-diméthylbenzoyl)-5-hydroxy-benzocyclobutène-1-carboxylate de méthyle,
le 4-(2,6-diméthoxybenzoyl)-5-hydroxy-benzocyclobutène-1-carboxylate de méthyle, ou
le 4-(o-méthylbenzoyl)-5-hydroxy-benzocyclobutène-1-carboxylate de méthyle.

8. Procédé selon la revendication 1, caractérisé en ce que l'on prépare l'acide 4-(benzoyl)-5-hydroxy-benzocyclobutène-1-carboxylique,
l'acide 4-(m-chlorobenzoyl)-5-hydroxy-benzocyclobutène-1-carboxylique,
l'acide 4-(m-méthylbenzoyl)-5-hydroxy-benzocyclobutène-1-carboxylique,
l'acide 4-(p-chlorobenzoyl)-5-hydroxy-benzocyclobutène-1-carboxylique,
l'acide 4-(2,6-dichlorobenzoyl)-5-hydroxy-benzocyclobutène-1-carboxylique,
l'acide 4-(3,4-dichlorobenzoyl)-5-hydroxy-benzocyclobutène-1-carboxylique,
l'acide 4-(o-acétoxybenzoyl)-5-hydroxy-benzocyclobutène-1-carboxylique,
l'acide 4-(o-méthylthiobenzoyl)-5-hydroxy-benzocyclo-butène-1-carboxylique,
l'acide 4-(o-trifluorométhylbenzoyl)-5-hydroxy-benzo-cyclobutène-1-carboxylique,
l'acide 4-(m-trifluorométhylbenzoyl)-5-hydroxy-benzo-cyclobutène-1-carboxylique,
l'acide 4-(o-méthoxybenzoyl)-5-hydroxy-benzocyclobutène-1-carboxylique,
l'acide 4-(p-méthoxybenzoyl)-5-hydroxy-benzocyclobutène-1-carboxylique,
l'acide 4-(p-cyanobenzoyl)-5-hydroxy-benzocyclobutène 1-carboxylique,
l'acide 4-(2,6-diméthylbenzoyl)-5-hydroxy-benzocyclobutène-1-carboxylique,
l'acide 4-(m-méthylthiobenzoyl)-5-hydroxy-benzocyclobutène-1-carboxylique,
l'acide 4-(2,6-diméthoxybenzoyl)-5-hydroxy-benzocyclobutène-1-carboxylique,

l'acide 4-(p-méthylbenzoyl)-5-hydroxy-benzocyclobutène-1-carboxylique,
l'acide 4-(o-chlorobenzoyl)-5-hydroxy-benzocyclobutène-1-carboxylique,
ou un sel de l'un de ces acides et d'une base.

9. Procédé selon la revendication 1, caractérisé en ce que l'on prépare l'acide 4-(o-fluorobenzoyl)-5-hydroxy-benzocyclobutène-1-carboxylique ou un sel de cet acide et d'une base.

10. Procédé selon la revendication 1, caractérisé en ce que l'on prépare l'acide 4-(o-bromobenzoyl)-5-hydroxy-benzocyclobutène-1-carboxylique ou un sel de cet acide et d'une base.

11. Procédé de préparation de compositions pharmaceutiques, caractérisé en ce que l'on mélange un composé obtenu selon l'une des revendications 1 à 10 des produits auxiliaires et/ou véhicules pharmaceutiques usuels.